# EUROPEAN PATENT APPLICATION

(11) **EP 4 669 086 A1**
(43) Date of publication of application: **24.12.2025**
(21) Application number: 25181206.1
(22) Date of filing: 05.06.2025
(51) Int. Cl.: H10K 85/60

(54) **ORGANIC COMPOUND, LIGHT-EMITTING DEVICE INCLUDING THE SAME, ELECTRONIC APPARATUS INCLUDING THE LIGHT-EMITTING DEVICE, AND ELECTRONIC EQUIPMENT INCLUDING THE ELECTRONIC APPARATUS**

(30) Priority: 20.06.2024 KR 20240080206
(71) Applicant: Samsung Display Co., Ltd., Yongin-si, Gyeonggi-do 17113 (KR)
(72) Inventor: KIM, Dojin, 17113 Yongin-si (KR); AHN, Heechoon, 17113 Yongin-si (KR); UM, Hyunah, 17113 Yongin-si (KR); RYOO, Chihyun, 17113 Yongin-si (KR); LEE, Yeseul, 17113 Yongin-si (KR); HAN, Junghoon, 17113 Yongin-si (KR)
(74) Representative: Marks & Clerk LLP

(57) **Abstract**

Embodiments provide an organic compound, a light-emitting device including the organic compound, an electronic apparatus including the light-emitting device, and an electronic equipment including the electronic apparatus. The light-emitting device includes a first electrode, a second electrode facing the first electrode, an interlayer between the first electrode and the second electrode and including an emission layer, and an organic compound represented by Formula 1, which is explained in the specification:

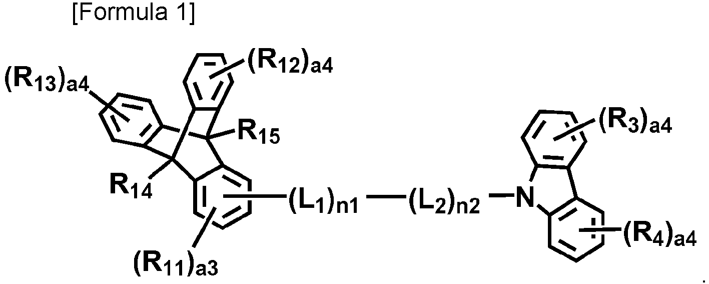

## Description

### BACKGROUND

### 1. Technical Field

Embodiments relate to an organic compound, a light-emitting device including the same, an electronic apparatus including the light-emitting device, and electronic equipment including the electronic apparatus.

### 2. Description of the Related Art

Light-emitting devices (for example, organic light-emitting devices, etc.) are self-emissive devices that have wide viewing angles, high contrast ratios, short response times, and excellent characteristics in terms of luminance, driving voltage, and response speed.

A light-emitting device may include a first electrode, a hole transport region, an emission layer, an electron transport region, and a second electrode, arranged sequentially. Holes injected from the first electrode may move toward the emission layer through the hole transport region. Electrons injected from the second electrode may move toward the emission layer through the electron transport region. Carriers, such as holes and electrons, may recombine in the emission layer to produce excitons. When the excitons drop from an excited state to a ground state, light may be generated.

It is to be understood that this background of the technology section is, in part, intended to provide useful background for understanding the technology. However, this background of the technology section may also include ideas, concepts, or recognitions that were not part of what was known or appreciated by those skilled in the pertinent art prior to a corresponding effective filing date of the subject matter disclosed herein.

### SUMMARY

Embodiments include an organic compound, which has improved thermal stability due to a high glass transition temperature and has improved charge transport capability while maintaining a high triplet (T₁) energy level, a light-emitting device having a long lifespan by including the organic compound, an electronic apparatus having excellent display quality by including the light-emitting device, and high-quality electronic equipment including the electronic apparatus.

Additional aspects will be set forth in part in the description which follows and, in part, will be apparent from the description, or may be learned by practice of the embodiments of the disclosure.

According to embodiments, a light-emitting device includes a first electrode, a second electrode facing the first electrode, an interlayer between the first electrode and the second electrode and including an emission layer, and an organic compound represented by Formula 1:

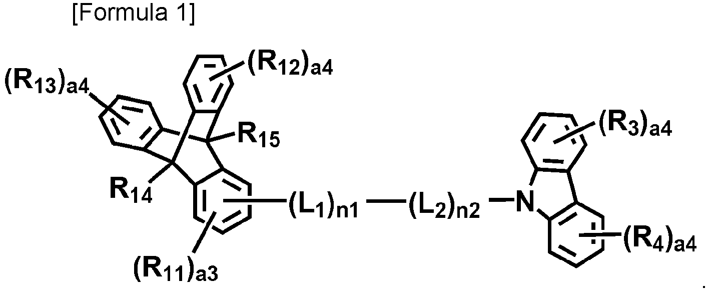

In Formula 1,
L₁ is a C₃-C₆₀ carbocyclic group unsubstituted or substituted with at least one R₁ or a C₁-C₆₀ heterocyclic group unsubstituted or substituted with at least one R₁,
n1 is an integer from 0 to 3,
L₂ is a carbazole group unsubstituted or substituted with at least one R₂,
n2 is 1 or 2,
R₁ to R₄ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₁)(Q₂)(Q₃), -N(Q₁)(Q₂), -B(Q₁)(Q₂), -C(=O)(Q₁), -S(=O)₂(Q₁), or -P(=O)(Q₁)(Q₂),
R₁₁ to R₁₅ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -N(Q₁)(Q₂), - B(Q₁)(Q₂), -C(=O)(Q₁), -S(=O)₂(Q₁), or -P(=O)(Q₁)(Q₂),
a3 is an integer from 0 to 3,
a4 is an integer from 0 to 4,
R₁₀ₐ is:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ are each independently:
hydrogen, deuterium, -F, -Cl, -Br, -I, a hydroxyl group, a cyano group, or a nitro group; or
a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

In an embodiment, the interlayer may include the organic compound.

In an embodiment, the emission layer may include the organic compound.

In an embodiment, the emission layer may emit blue light.

In an embodiment, the emission layer may include an electron-transporting host including at least one π electron-deficient nitrogen-containing C₁-C₆₀ heterocyclic group.

In an embodiment, the emission layer may include at least one of a fluorescent dopant, a phosphorescent dopant, and a delayed fluorescence dopant.

According to embodiments, an electronic apparatus includes the light-emitting device, and a thin-film transistor electrically connected to the light-emitting device.

In an embodiment, the electronic apparatus may include: a color filter, a color conversion layer, a touch screen layer, a polarizing layer, or any combination thereof.

In an embodiment, the color conversion layer may include quantum dots.

According to embodiments, an electronic equipment includes the electronic apparatus, wherein the electronic equipment is a flat panel display, a curved display, a computer monitor, a medical monitor, a television, a billboard, an indoor light, an outdoor light, a signal light, a head-up display, a fully transparent display, a partially transparent display, a flexible display, a rollable display, a foldable display, a stretchable display, a laser printer, a telephone, a mobile phone, a tablet computer, a phablet, a personal digital assistant (PDA), a wearable device, a laptop computer, a digital camera, a camcorder, a viewfinder, a micro display, a three-dimensional (3D) display, a virtual reality display, an augmented reality display, a vehicle, a video wall with multiple displays tiled together, a theater screen, a stadium screen, a phototherapy device, or a signboard.

According to embodiments, an organic compound is represented by Formula 1, which is explained herein.

In an embodiment, L₁ may be a C₆-C₆₀ arylene group unsubstituted or substituted with at least one R₁.

In an embodiment, L₁ may be a group represented by one of Formulae B1 to B3, which are explained below.

In an embodiment, in Formula 1, L₂ may be a group represented by Formula C1, which is explained below.

In an embodiment, the organic compound may include two carbazole groups linked to each other via a single bond.

In an embodiment, R₁ to R₄ and R₁₁ to R₁₅ may each not include a carbazole group.

In an embodiment, in Formula 1, a moiety represented by 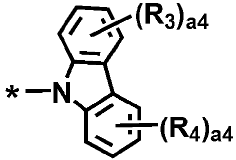 may be a moiety represented by one of Formulae Cz1 to Cz5, which are explained below.

In an embodiment, R₁₄ and R₁₅ may each independently be hydrogen or deuterium.

In an embodiment, in Formula 1, a moiety represented by 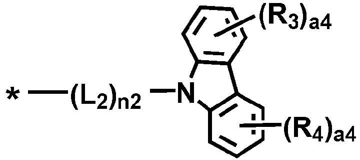 may be a moiety represented by one of Formulae A1 to A8, which are explained below.

In an embodiment, the organic compound may be represented by Formula 1' or Formula 1", which are explained below.

In an embodiment, the organic compound may be represented by Formula 1-1, which is explained below.

In an embodiment, the organic compound may be one of Compounds 1 to 100, which are explained below, or the organic compound may be a compound in which at least one hydrogen atom in one of Compounds 1 to 100 is substituted with deuterium.

At least some of the above and other features of the invention are set out in the claims.

It is to be understood that the embodiments above are described in a generic and explanatory sense only and not for the purposes of limitation, and the disclosure is not limited to the embodiments described above.

### BRIEF DESCRIPTION OF THE DRAWINGS

The accompanying drawings are included to provide a further understanding of the embodiments, and are incorporated in and constitute a part of this specification. The drawings illustrate embodiments of the disclosure and principles thereof. The above and other aspects and features of the disclosure will become more apparent by describing in detail embodiments thereof with reference to the accompanying drawings, in which:
FIG. 1 is a schematic cross-sectional view of a light-emitting device according to an embodiment;
FIG. 2 is a schematic cross-sectional view of an electronic apparatus according to an embodiment;
FIG. 3 is a schematic cross-sectional view of an electronic apparatus according to another embodiment;
FIG. 4 is a schematic perspective view of an electronic equipment including a light-emitting device according to an embodiment;
FIG. 5 is a schematic perspective view of an exterior of a vehicle as an electronic equipment including a light-emitting device according to an embodiment; and
FIGS. 6A to 6C are each a schematic diagram of an interior of a vehicle according to embodiments.

### DETAILED DESCRIPTION OF THE EMBODIMENTS

The disclosure will now be described more fully hereinafter with reference to the accompanying drawings, in which embodiments are shown. This disclosure may, however, be embodied in different forms and should not be construed as limited to the embodiments set forth herein. Rather, these embodiments are provided so that this disclosure will be thorough and complete, and will fully convey the scope of the disclosure to those skilled in the art.

In the drawings, the sizes, thicknesses, ratios, and dimensions of the elements may be exaggerated for ease of description and for clarity. Like reference numbers and/or like reference characters refer to like elements throughout.

In the description, it will be understood that when an element (or region, layer, part, etc.) is referred to as being "on", "connected to", or "coupled to" another element, it can be directly on, connected to, or coupled to the other element, or one or more intervening elements may be present therebetween. In a similar sense, when an element (or region, layer, part, etc.) is described as "covering" another element, it can directly cover the other element, or one or more intervening elements may be present therebetween.

In the description, when an element is "directly on," "directly connected to," or "directly coupled to" another element, there are no intervening elements present. For example, "directly on" may mean that two layers or two elements are disposed without an additional element such as an adhesion element therebetween.

As used herein, the expressions used in the singular such as "a," "an," and "the," are intended to include the plural forms as well, unless the context clearly indicates otherwise.

As used herein, the term "and/or" includes any and all combinations of one or more of the associated listed items. For example, "A and/or B" may be understood to mean "A, B, or A and B." The terms "and" and "or" may be used in the conjunctive or disjunctive sense and may be understood to be equivalent to "and/or".

In the specification and the claims, the term "at least one of" is intended to include the meaning of "at least one selected from the group consisting of" for the purpose of its meaning and interpretation. For example, "at least one of A, B, and C" may be understood to mean A only, B only, C only, or any combination of two or more of A, B, and C, such as ABC, ACC, BC, or CC. When preceding a list of elements, the term, "at least one of," modifies the entire list of elements and does not modify the individual elements of the list.

It will be understood that, although the terms first, second, etc. may be used herein to describe various elements, these elements should not be limited by these terms. These terms are only used to distinguish one element from another element. Thus, a first element could be termed a second element without departing from the teachings of the disclosure. Similarly, a second element could be termed a first element, without departing from the scope of the disclosure.

The spatially relative terms "below", "beneath", "lower", "above", "upper", or the like, may be used herein for ease of description to describe the relations between one element or component and another element or component as illustrated in the drawings. It will be understood that the spatially relative terms are intended to encompass different orientations of the device in use or operation, in addition to the orientation depicted in the drawings. For example, in the case where a device illustrated in the drawing is turned over, the device positioned "below" or "beneath" another device may be placed "above" another device. Accordingly, the illustrative term "below" may include both the lower and upper positions. The device may also be oriented in other directions and thus the spatially relative terms may be interpreted differently depending on the orientations.

The terms "about" or "approximately" as used herein is inclusive of the stated value and means within an acceptable range of deviation for the recited value as determined by one of ordinary skill in the art, considering the measurement in question and the error associated with measurement of the recited quantity (for example, the limitations of the measurement system). For example, "about" may mean within one or more standard deviations, or within ±20%, ±10%, or ±5% of the stated value.

It should be understood that the terms "comprises," "comprising," "includes," "including," "have," "having," "contains," "containing," and the like are intended to specify the presence of stated features, integers, steps, operations, elements, components, or combinations thereof in the disclosure, but do not preclude the presence or addition of one or more other features, integers, steps, operations, elements, components, or combinations thereof.

Unless otherwise defined or implied herein, all terms (including technical and scientific terms) used have the same meaning as commonly understood by those skilled in the art to which this disclosure pertains. It will be further understood that terms, such as those defined in commonly used dictionaries, should be interpreted as having a meaning that is consistent with their meaning in the context of the relevant art and should not be interpreted in an ideal or excessively formal sense unless clearly defined in the specification.

According to embodiments, a light-emitting device includes: a first electrode; a second electrode facing the first electrode; an interlayer between the first electrode and the second electrode and including an emission layer; and an organic compound represented by Formula 1, which will be explained below.

In the specification, the term "interlayer" may be a single layer and/or multiple layers between the first electrode and the second electrode of the light-emitting device. In an embodiment, the interlayer may further include a hole transport region between the first electrode and the emission layer and an electron transport region between the emission layer and the second electrode.

In an embodiment, the interlayer may include the organic compound represented by Formula 1. For example, at least one of the hole transport region, the emission layer, and the electron transport region may include the organic compound. In an embodiment, the emission layer may include the organic compound. The organic compound may be referred to as a hole transport host. In the specification, the expression "the interlayer (or the emission layer) includes the organic compound represented by Formula 1" may be interpreted as "the interlayer (or the emission layer) includes one type of the organic compound represented by Formula 1" or "the interlayer (or the emission layer) includes two or more types of the organic compound, each independently represented by Formula 1."

In an embodiment, the light-emitting device may further include a capping layer outside the first electrode and/or outside the second electrode. In an embodiment, the light-emitting device may further include a first capping layer outside the first electrode. For example, the light-emitting device may include the first capping layer, the first electrode, the interlayer, and the second electrode that are arranged in this order. In embodiments, the light-emitting device may further include a second capping layer outside the second electrode. For example, the light-emitting device may include the first electrode, the interlayer, the second electrode, and the second capping layer that are arranged in this order. In embodiments, the light-emitting device may further include a first capping layer outside the first electrode and a second capping layer outside the second electrode. For example, the light-emitting device may include the first capping layer, the first electrode, the interlayer, the second electrode, and the second capping layer that are arranged in this order.

In an embodiment, the capping layer may include the organic compound. In an embodiment, the first capping layer may include the organic compound. In embodiments, the second capping layer may include the organic compound. In embodiments, the first capping layer and the second capping layer may each include the organic compound.

In an embodiment, the emission layer may further include an electron-transporting host. The electron-transporting host may be different from the organic compound. In an embodiment, the electron-transporting host may include at least one π electron-deficient nitrogen-containing C₁-C₆₀ heterocyclic group. In an embodiment, the electron-transporting host may be represented by Formula 2:

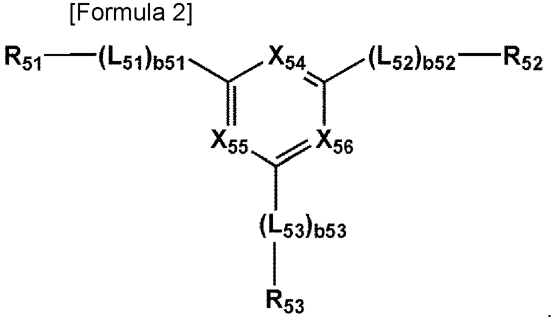

In Formula 2,
L₅₁ to L₅₃ may each independently be a single bond, a C₃-C₆₀ (e.g. C₃-C₃₀) carbocyclic group unsubstituted or substituted with at least one R₁₀ₐ, or a C₁-C₆₀ (e.g. C₁-C₂₀) heterocyclic group unsubstituted or substituted with at least one R₁₀ₐ,
b51 to b53 may each independently be an integer from 1 to 5,
X₅₄ may be N or C(R₅₄), X₅₅ may be N or C(R₅₅), X₅₆ may be N or C(R₅₆), and at least one of X₅₄ to X₅₆ may be N,
R₅₁ to R₅₆ may each independently be the same as described in connection with R₁, and
R₁₀ₐ may be the same as described herein.
In an embodiment, the electron-transporting host may be one of Compounds ETH1 to ETH100:

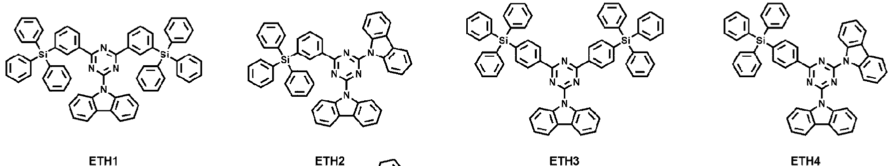

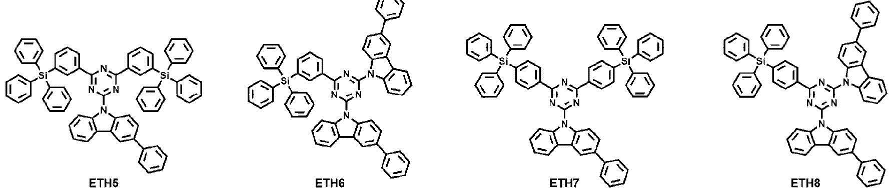

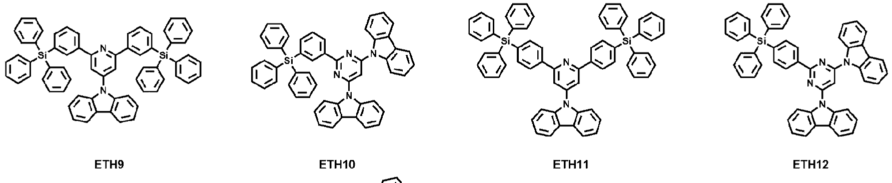

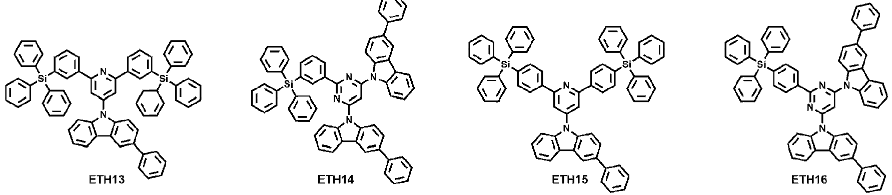

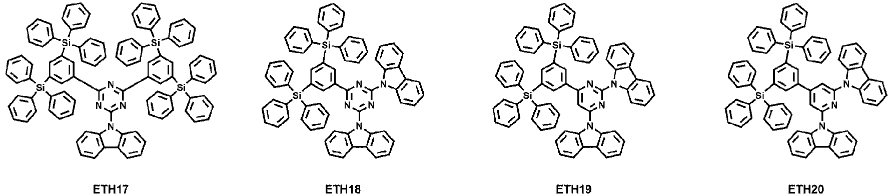

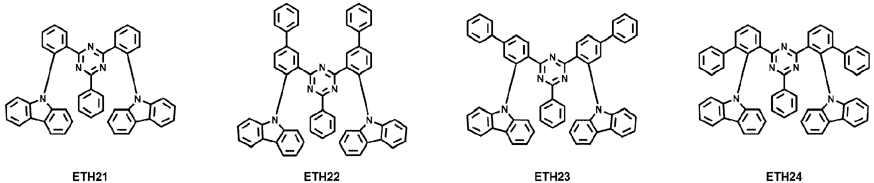

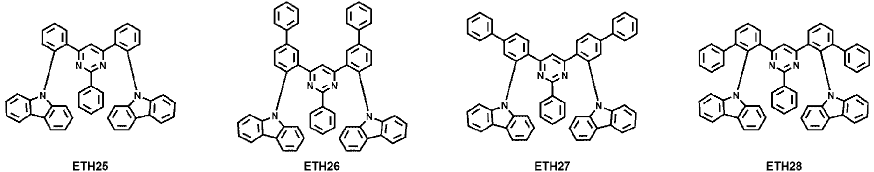

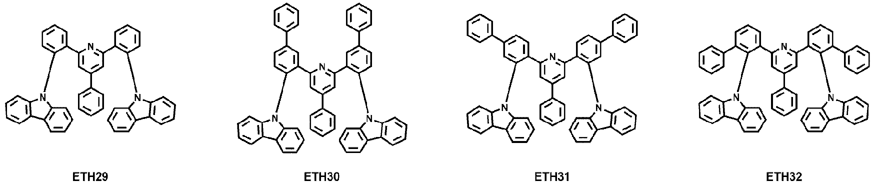

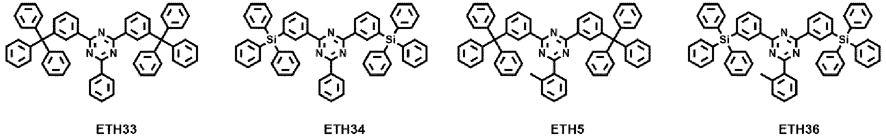

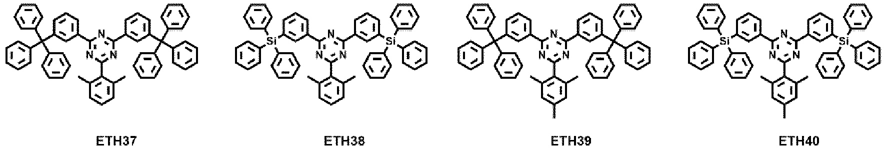

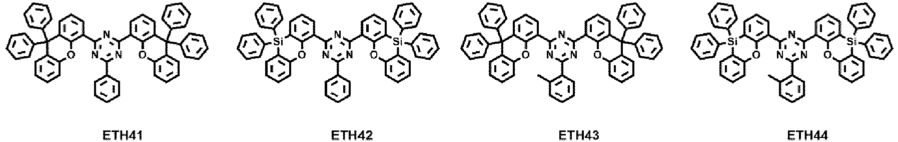

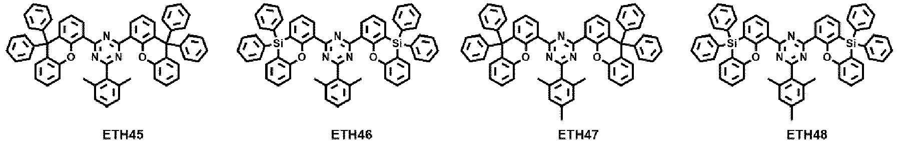

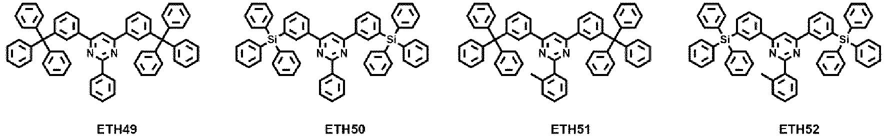

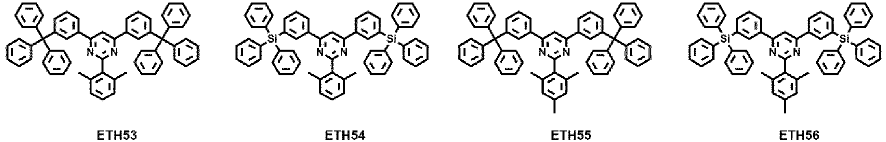

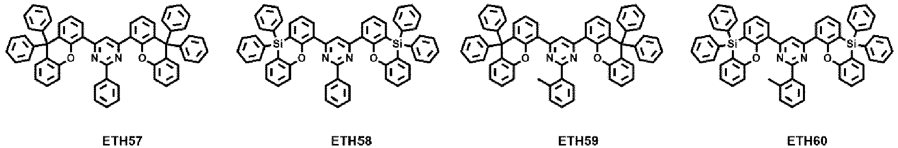

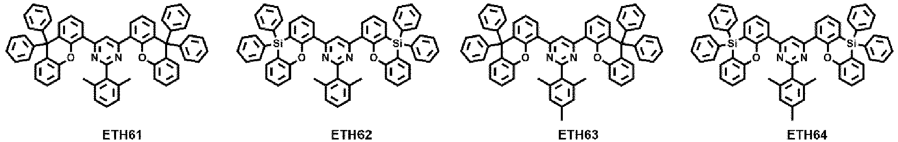

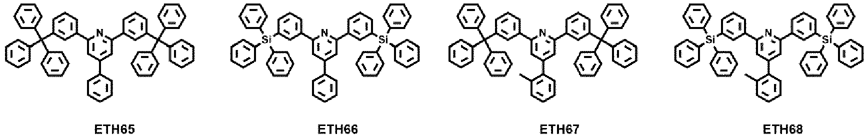

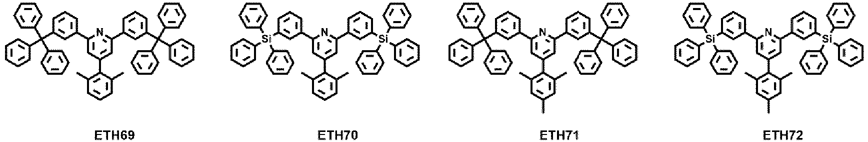

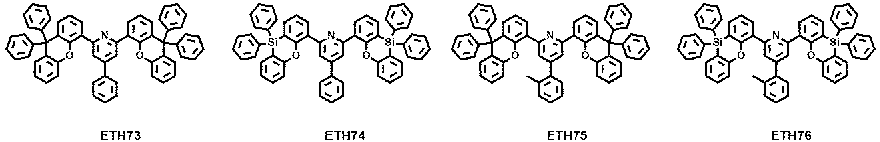

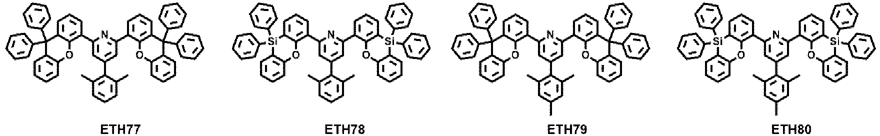

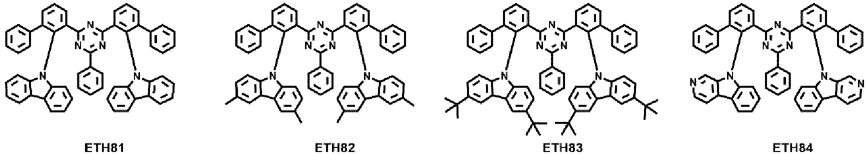

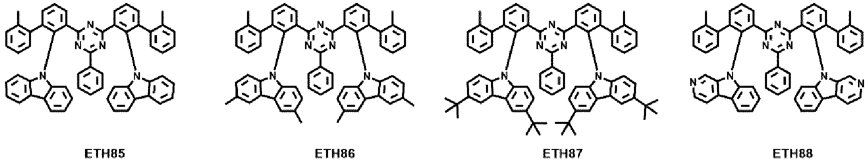

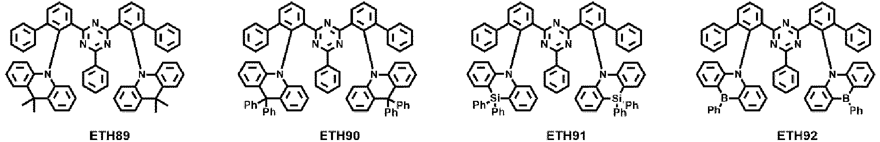

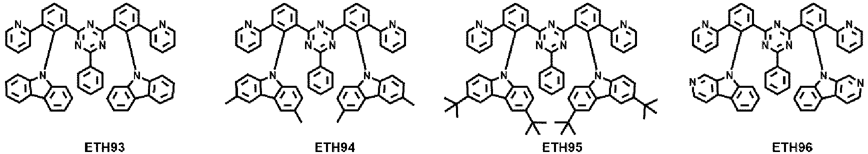

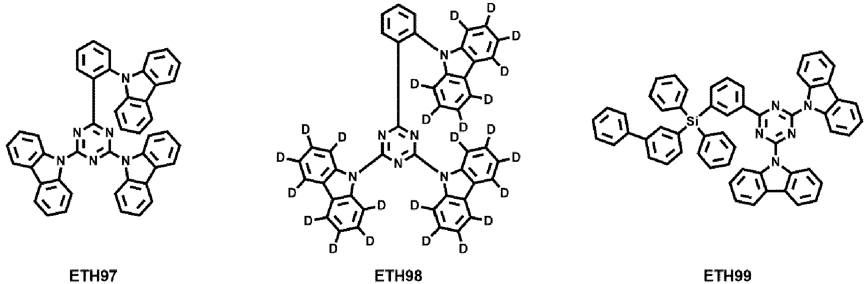

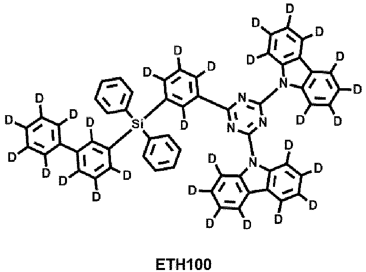

In an embodiment, the emission layer may further include at least one of a fluorescent dopant, a phosphorescent dopant, and a delayed fluorescent dopant.

In an embodiment, the emission layer may emit blue light. The blue light may be deep blue light. For example, the blue light may have a maximum emission wavelength in a range of about 430 nm to about 475 nm. For example, the blue light may have a maximum emission wavelength in a range of about 440 nm to about 475 nm. For example, the blue light may have a maximum emission wavelength in a range of about 450 nm to about 475 nm. For example, the blue light may have a maximum emission wavelength in a range of about 430 nm to about 470 nm. For example, the blue light may have a maximum emission wavelength in a range of about 440 nm to about 470 nm. For example, the blue light may have a maximum emission wavelength in a range of about 450 nm to about 470 nm. For example, the blue light may have a maximum emission wavelength in a range of about 430 nm to about 465 nm. For example, the blue light may have a maximum emission wavelength in a range of about 440 nm to about 465 nm. For example, the blue light may have a maximum emission wavelength in a range of about 450 nm to about 465 nm. For example, the blue light may have a maximum emission wavelength in a range of about 430 nm to about 460 nm. For example, the blue light may have a maximum emission wavelength in a range of about 440 nm to about 460 nm. For example, the blue light may have a maximum emission wavelength in a range of about 450 nm to about 460 nm. The blue light may have a CIEx coordinate in a range of about 0.125 to 0.140. For example, the blue light may have a CIEx coordinate in a range of about 0.130 to 0.140. The blue light may have a CIEy of 0.120 to 0.210.

According to another embodiment, an electronic apparatus includes: the light-emitting device; and a thin-film transistor electrically connected to the light-emitting device.

According to another embodiment, an electronic equipment includes the electronic apparatus, wherein the electronic equipment is a flat panel display, a curved display, a computer monitor, a medical monitor, a television, a billboard, an indoor light, an outdoor light, a signal light, a head-up display, a fully transparent display, a partially transparent display, a flexible display, a rollable display, a foldable display, a stretchable display, a laser printer, a telephone, a mobile phone, a tablet computer, a phablet, a personal digital assistant (PDA), a wearable device, a laptop computer, a digital camera, a camcorder, a viewfinder, a micro display, a three-dimensional (3D) display, a virtual reality display, an augmented reality display, a vehicle, a video wall with multiple displays tiled together, a theater stadium screen, a stadium screen, a phototherapy device, or a signboard.

### [Description of Formula 1]

According to embodiments, the organic compound is represented by Formula 1:

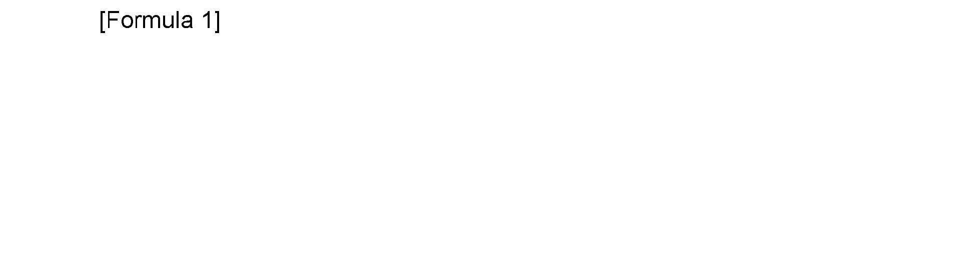

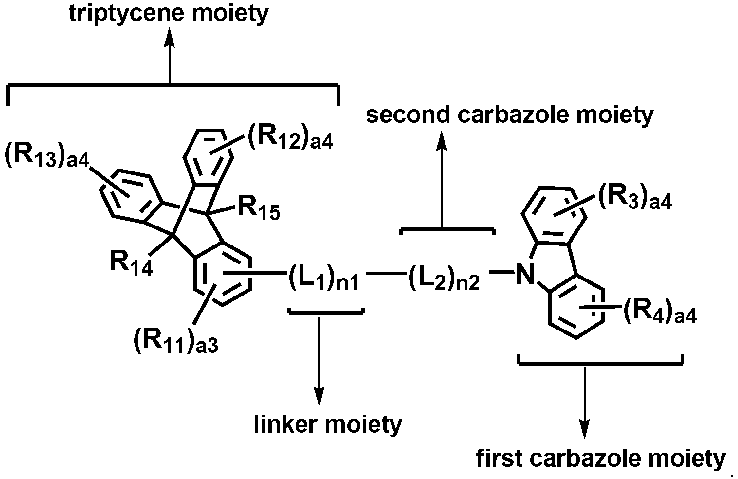

In Formula 1,
L₁ is a C₃-C₆₀ (e.g. C₃-C₃₀) carbocyclic group unsubstituted or substituted with at least one R₁ or a C₁-C₆₀ (e.g. C₁-C₂₀) heterocyclic group unsubstituted or substituted with at least one R₁,
n1 is an integer from 0 to 3,
L₂ is a carbazole group unsubstituted or substituted with at least one R₂,
n2 is 1 or 2,
R₁ to R₄ are each independently hydrogen, deuterium, -F, -Cl, -Br, -I, a hydroxyl group, a cyano group, a nitro group, a C₁-C₆₀ (e.g. C₁-C₂₀) alkyl group unsubstituted or substituted with at least one R₁₀ₐ, a C₂-C₆₀ (e.g. C₂-C₂₀) alkenyl group unsubstituted or substituted with at least one R₁₀ₐ, a C₂-C₆₀ (e.g. C₂-C₂₀) alkynyl group unsubstituted or substituted with at least one R₁₀ₐ, a C₁-C₆₀ (e.g. C₁-C₂₀) alkoxy group unsubstituted or substituted with at least one R₁₀ₐ, a C₃-C₆₀ (e.g. C₃-C₃₀) carbocyclic group unsubstituted or substituted with at least one R₁₀ₐ, a C₁-C₆₀ (e.g. C₁-C₂₀) heterocyclic group unsubstituted or substituted with at least one R₁₀ₐ, -Si(Q₁)(Q₂)(Q₃), -N(Q₁)(Q₂), -B(Q₁)(Q₂), -C(=O)(Q₁), -S(=O)₂(Q₁), or -P(=O)(Q₁)(Q₂),
R₁₁ to R₁₅ are each independently hydrogen, deuterium, -F, -Cl, -Br, -I, a hydroxyl group, a cyano group, a nitro group, a C₁-C₆₀ (e.g. C₁-C₂₀) alkyl group unsubstituted or substituted with at least one R₁₀ₐ, a C₂-C₆₀ (e.g. C₂-C₂₀) alkenyl group unsubstituted or substituted with at least one R₁₀ₐ, a C₂-C₆₀ (e.g. C₂-C₂₀) alkynyl group unsubstituted or substituted with at least one R₁₀ₐ, a C₁-C₆₀ (e.g. C₁-C₂₀) alkoxy group unsubstituted or substituted with at least one R₁₀ₐ, a C₃-C₆₀ (e.g. C₃-C₃₀) carbocyclic group unsubstituted or substituted with at least one R₁₀ₐ, a C₁-C₆₀ (e.g. C₁-C₂₀) heterocyclic group unsubstituted or substituted with at least one R₁₀ₐ, -N(Q₁)(Q₂), - B(Q₁)(Q₂), -C(=O)(Q₁), -S(=O)₂(Q₁), or -P(=O)(Q₁)(Q₂),
a3 is an integer from 0 to 3,
a4 is an integer from 0 to 4,
R₁₀ₐ is:
deuterium, -F, -Cl, -Br, -I, a hydroxyl group, a cyano group, or a nitro group;
a C₁-C₆₀ (e.g. C₁-C₂₀) alkyl group, a C₂-C₆₀ (e.g. C₂-C₂₀) alkenyl group, a C₂-C₆₀ (e.g. C₂-C₂₀) alkynyl group, or a C₁-C₆₀ (e.g. C₁-C₂₀) alkoxy group, each unsubstituted or substituted with deuterium, -F, -Cl, -Br, -I, a hydroxyl group, a cyano group, a nitro group, a C₃-C₆₀ (e.g. C₃-C₃₀) carbocyclic group, a C₁-C₆₀ (e.g. C₁-C₂₀) heterocyclic group, a C₆-C₆₀ (e.g. C₆-C₃₀) aryloxy group, a C₆-C₆₀ (e.g. C₆-C₃₀) arylthio group, a C₇-C₆₀ (e.g. C₇-C₃₀) arylalkyl group, a C₂-C₆₀ (e.g. C₂-C₂₀) heteroarylalkyl group, -Si(Q₁₁)(Q₁₂)(Q₁₃), -N(Q₁₁)(Q₁₂), -B(Q₁₁)(Q₁₂), -C(=O)(Q₁₁), -S(=O)2(Q₁₁), - P(=O)(Q₁₁)(Q₁₂), or any combination thereof;
a C₃-C₆₀ (e.g. C₃-C₃₀) carbocyclic group, a C₁-C₆₀ (e.g. C₁-C₂₀) heterocyclic group, a C₆-C₆₀ (e.g. C₆-C₃₀) aryloxy group, a C₆-C₆₀ (e.g. C₆-C₃₀) arylthio group, a C₇-C₆₀ (e.g. C₇-C₃₀) arylalkyl group, or a C₂-C₆₀ (e.g. C₂-C₂₀) heteroarylalkyl group, each unsubstituted or substituted with deuterium, -F, -Cl, -Br, -I, a hydroxyl group, a cyano group, a nitro group, a C₁-C₆₀ (e.g. C₁-C₂₀) alkyl group, a C₂-C₆₀ (e.g. C₂-C₂₀) alkenyl group, a C₂-C₆₀ (e.g. C₂-C₂₀) alkynyl group, a C₁-C₆₀ (e.g. C₁-C₂₀) alkoxy group, a C₃-C₆₀ (e.g. C₃-C₃₀) carbocyclic group, a C₁-C₆₀ (e.g. C₁-C₂₀) heterocyclic group, a C₆-C₆₀ (e.g. C₆-C₃₀) aryloxy group, a C₆-C₆₀ (e.g. C₆-C₃₀) arylthio group, a C₇-C₆₀ (e.g. C₇-C₃₀) arylalkyl group, a C₂-C₆₀ (e.g. C₂-C₂₀) heteroarylalkyl group, -Si(Q₂₁)(Q₂₂)(Q₂₃), - N(Q₂₁)(Q₂₂), -B(Q₂₁)(Q₂₂), -C(=O)(Q₂₁), -S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ are each independently:
hydrogen, deuterium, -F, -Cl, -Br, -I, a hydroxyl group, a cyano group, or a nitro group; or
a C₁-C₆₀ (e.g. C₁-C₂₀) alkyl group, a C₂-C₆₀ (e.g. C₂-C₂₀) alkenyl group, a C₂-C₆₀ (e.g. C₂-C₂₀) alkynyl group, a C₁-C₆₀ (e.g. C₁-C₂₀) alkoxy group, a C₃-C₆₀ (e.g. C₃-C₃₀) carbocyclic group, or a C₁-C₆₀ (e.g. C₁-C₂₀) heterocyclic group, each unsubstituted or substituted with deuterium, -F, a cyano group, a C₁-C₆₀ (e.g. C₁-C₂₀) alkyl group, a C₁-C₆₀ (e.g. C₁-C₂₀) alkoxy group, a phenyl group, a biphenyl group, or any combination thereof.

In an embodiment, in Formula 1, L₁ may be a C₆-C₆₀ (e.g. C₆-C₃₀) arylene group unsubstituted or substituted with at least one R₁. For example, L₁ may be a benzene group, a naphthalene group, an anthracene group, a phenanthrene group, a perylene group, a triphenylene group, a phenalene group, a pyrene group, or a chrysene group.

In an embodiment, in Formula 1, L₁ may be a group represented by one of Formulae B1 to B3. For example, L₁ may be an ortho-phenylene group, a meta-phenylene group, or a para-phenylene group:

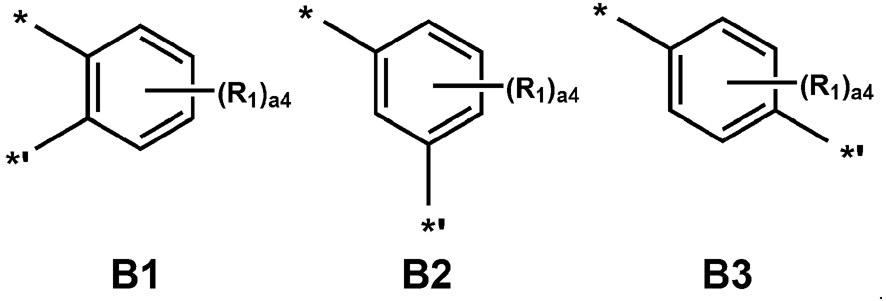

In Formulae B1 to B3,
R₁ and a4 may each be the same as described herein,
   * indicates a binding site to (L₂)ₙ₂ in Formula 1, and
   *' indicates a binding site to a neighboring atom.

In an embodiment, in Formula 1, n1 may be 0, 1, or 2. When n1 is 0, (L₁)ₙ₁ may be a single bond. When n1 is 2 or more, multiple L₁ groups may be identical to or different from each other.

In an embodiment, in Formula 1, L₂ may be a group represented by Formula C1. For example, L₂ may be a carbazole group linked to (L₁)ₙ₁ via nitrogen:

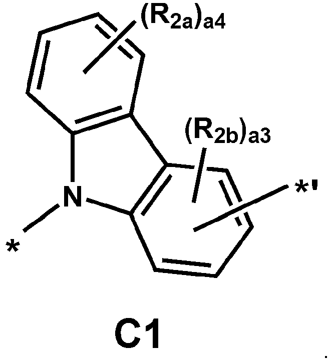

In Formula C1,
a3 and a4 may each be the same as described herein,
R₂ₐ and R_{2b} may each independently be the same as described in connection with R₂,
   * indicates a binding site to (L₁)ₙ₁ in Formula 1, and
   *' indicates a binding site to a neighboring atom.

In an embodiment, in Formula 1, L₂ may not be a carbazole group in which an atom linked to a group represented by (L₁)ₙ₁ is carbon and an atom linked to a group represented by 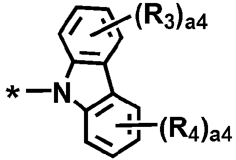 is carbon. In an embodiment, in Formula 1, L₂ may not be a carbazole group in which N is linked to a group represented by

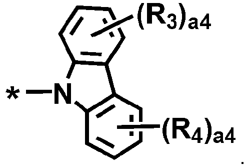

In an embodiment, the organic compound may include two carbazole groups that are linked to each other via a single bond. For example, referring to Formula 1, the organic compound may include a first carbazole moiety and a second carbazole moiety, wherein the first carbazole moiety and the second carbazole moiety may be linked to each other via a single bond. Thus, the organic compound may be distinguished from a compound that includes a linker, such as a benzene group, between two carbazole groups, and as shown in Comparative Compound CE3, may also be distinguished from a compound that includes multiple carbazole groups, each bonded to a triptycene moiety:

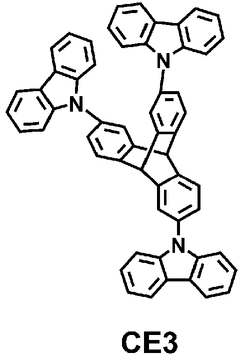

In an embodiment, R₁ to R₄ may each independently be hydrogen, deuterium, -F, a cyano group, a nitro group, a C₁-C₆₀ (e.g. C₁-C₂₀) alkyl group unsubstituted or substituted with at least one R₁₀ₐ, a C₂-C₆₀ (e.g. C₂-C₂₀) alkenyl group unsubstituted or substituted with at least one R₁₀ₐ, a C₂-C₆₀ (e.g. C₂-C₂₀) alkynyl group unsubstituted or substituted with at least one R₁₀ₐ, a C₁-C₆₀ (e.g. C₁-C₂₀) alkoxy group unsubstituted or substituted with at least one R₁₀ₐ, a C₃-C₆₀ (e.g. C₃-C₃₀) carbocyclic group unsubstituted or substituted with at least one R₁₀ₐ, a C₁-C₆₀ (e.g. C₁-C₂₀) heterocyclic group unsubstituted or substituted with at least one R₁₀ₐ, -Si(Q₁)(Q₂)(Q₃), -N(Q₁)(Q₂), -B(Q₁)(Q₂), -C(=O)(Q₁), -S(=O)₂(Q₁), or -P(=O)(Q₁)(Q₂). For example, R₁ to R₄ may each independently be hydrogen, deuterium, a phenyl group unsubstituted or substituted with deuterium, or a triphenylsilyl group (-Si(Ph)₃) unsubstituted or substituted with deuterium.

In an embodiment, R₁₁ to R₁₅ may each independently be hydrogen, deuterium, -F, a cyano group, a nitro group, a C₁-C₆₀ (e.g. C₁-C₂₀) alkyl group unsubstituted or substituted with at least one R₁₀ₐ, a C₂-C₆₀ (e.g. C₂-C₂₀) alkenyl group unsubstituted or substituted with at least one R₁₀ₐ, a C₂-C₆₀ (e.g. C₂-C₂₀) alkynyl group unsubstituted or substituted with at least one R₁₀ₐ, a C₁-C₆₀ (e.g. C₁-C₂₀) alkoxy group unsubstituted or substituted with at least one R₁₀ₐ, a C₃-C₆₀ (e.g. C₃-C₃₀) carbocyclic group unsubstituted or substituted with at least one R₁₀ₐ, a C₁-C₆₀ (e.g. C₁-C₂₀) heterocyclic group unsubstituted or substituted with at least one R₁₀ₐ, -N(Q₁)(Q₂), -B(Q₁)(Q₂), - C(=O)(Q₁), -S(=O)₂(Q₁), or -P(=O)(Q₁)(Q₂). For example, each of R₁₁ to R₁₅ may not include Si that is directly bonded to the triptycene moiety in Formula 1. For example, R₁₁ to R₁₅ may each not be -Si(Ph)₃.

In an embodiment, at least one of R₁ to R₄ and R₁₁ to R₁₅ may be deuterium.

In an embodiment, in Formula 1, R₁ to R₄ and R₁₁ to R₁₅ may each not include a carbazole group.

In an embodiment, in Formula 1, a moiety represented by 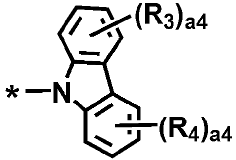 may be a moiety represented by one of Formulae Cz1 to Cz5:

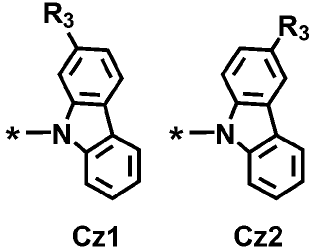

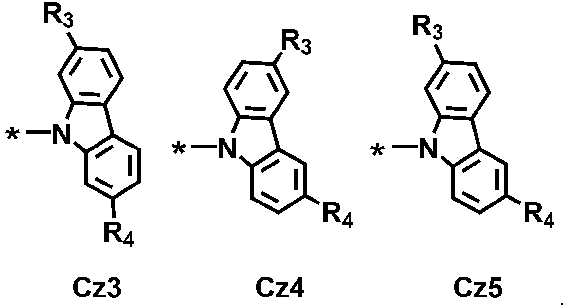

In Formulae Cz1 to Cz5, R₃ and R₄ may each be the same as described herein, and
* indicates a binding site to (L₂)ₙ₂ in Formula 1.

In an embodiment, R₁₄ and R₁₅ may each independently be hydrogen or deuterium.

In an embodiment, in Formula 1, a moiety represented by 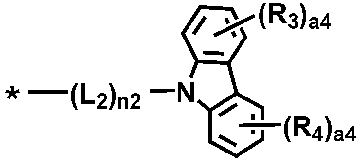 may be a moiety represented by one of Formulae A1 to A8:

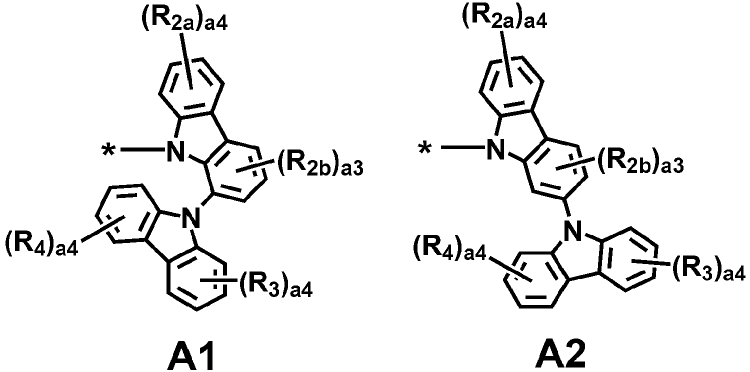

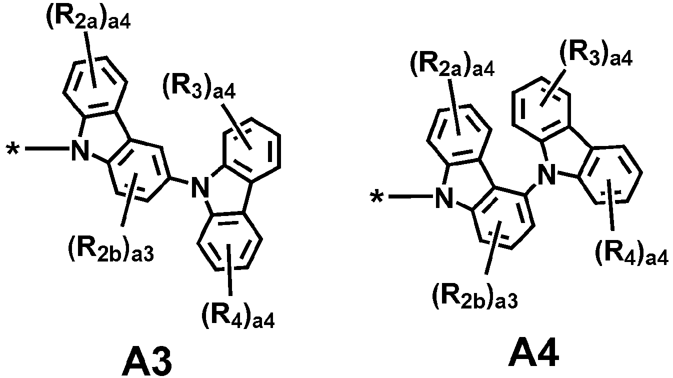

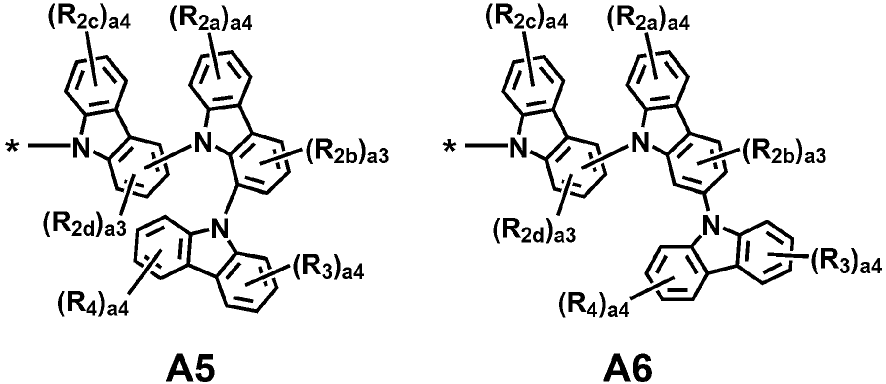

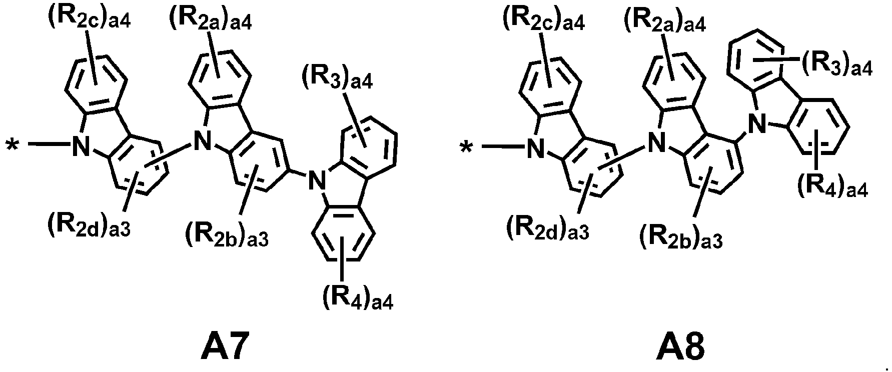

In Formulae A1 to A8,
* indicates a binding site to (L₁)ₙ₁ in Formula 1,
   R₂ₐ to R_{2d} may each independently be the same as described in connection with R₂, and
   R₃, R₄, a3, and a4 may each be the same as described in Formula 1.

In an embodiment, the organic compound may be represented by Formula 1' or Formula 1":

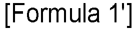

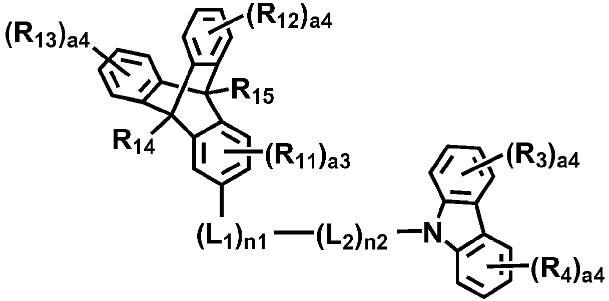

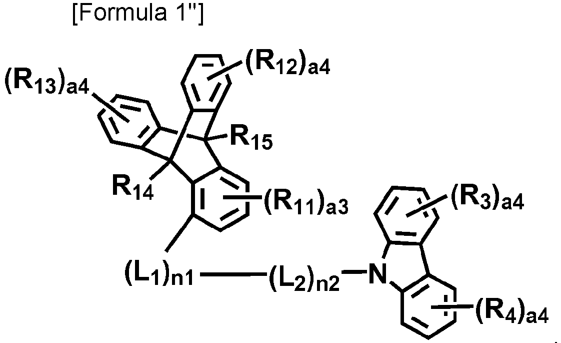

In Formulae 1' and 1", L₁, L₂, n1, n2, R₃, R₄, R₁₁ to R₁₅, a3, and a4 may each be the same as described in Formula 1.

In an embodiment, the organic compound may be represented by Formula 1'-B or Formula 1"-B:

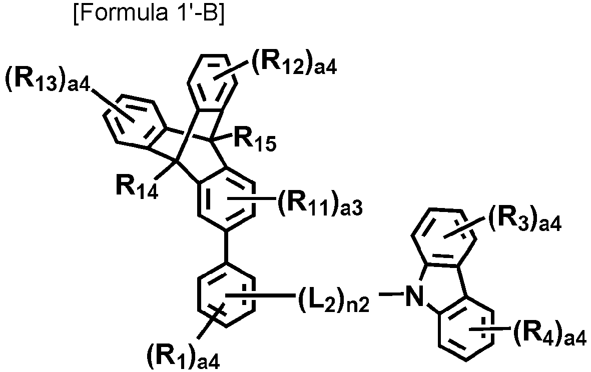

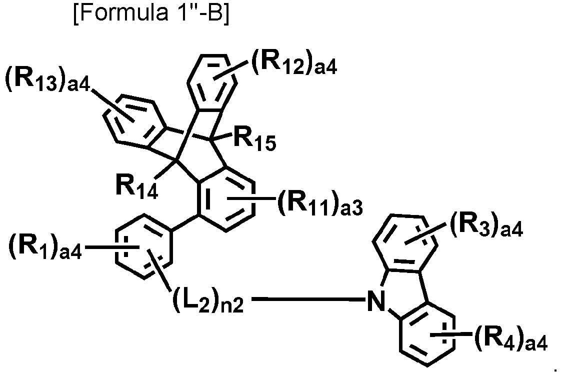

In Formulae 1'-B and 1"-B, L₂, n2, R₁, R₃, R₄, R₁₁ to R₁₅, a3, and a4 may each be the same as described in Formula 1.

In an embodiment, the organic compound may be represented by one of Formulae 1'-B1 to 1'-B3 and 1"-B1 to 1"-B3:

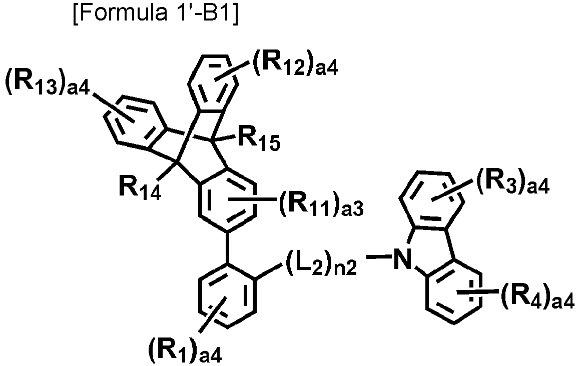

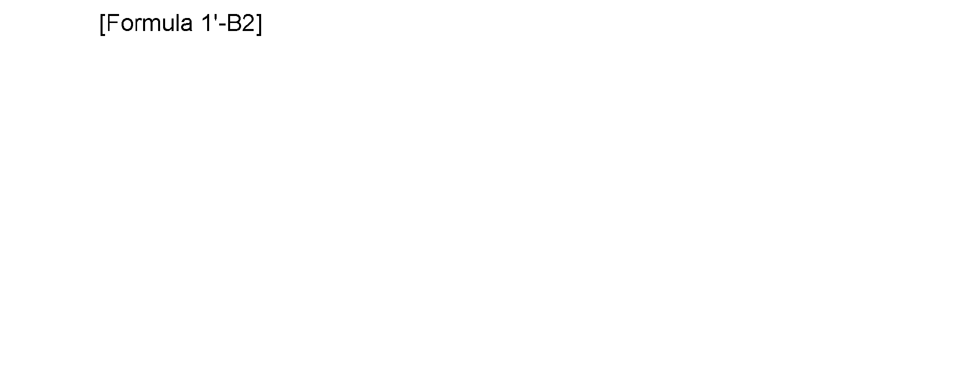

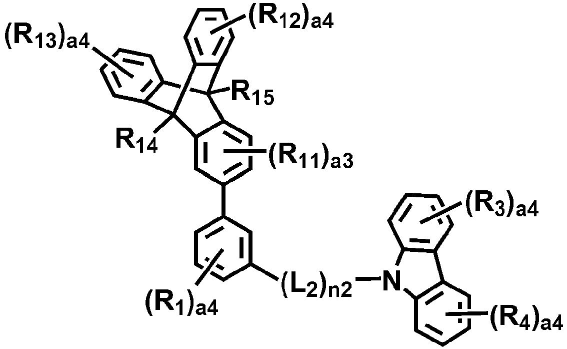

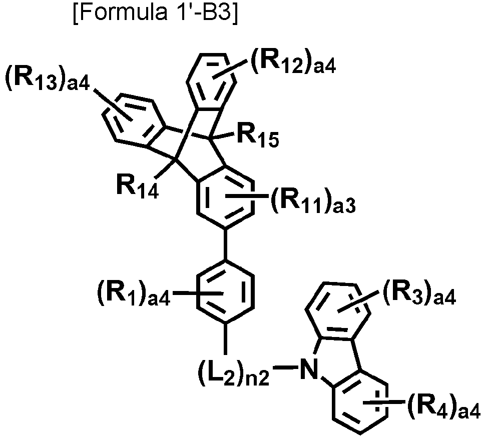

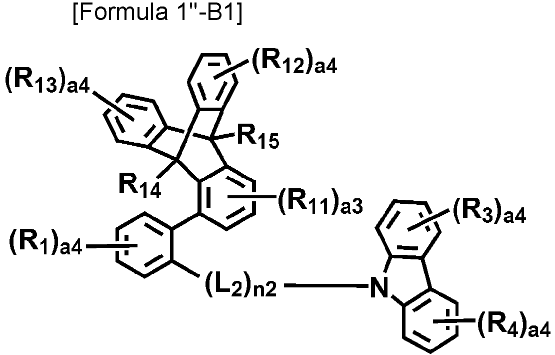

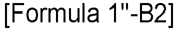

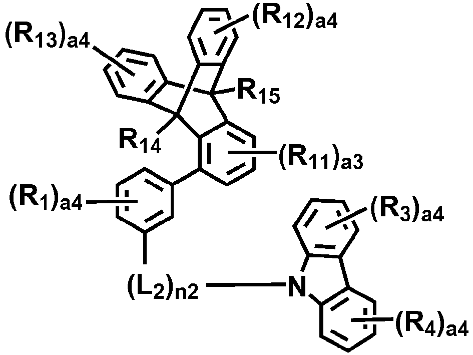

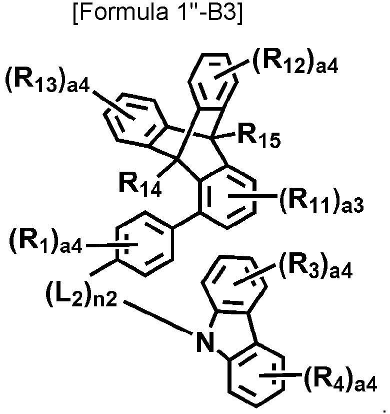

In Formulae 1'-B1 to 1'-B3 and 1"-B1 to 1"-B3, L₂, n2, R₁, R₃, R₄, R₁₁ to R₁₅, a3, and a4 may each be the same as described in Formula 1.

In an embodiment, the organic compound may be represented by Formula 1-1:

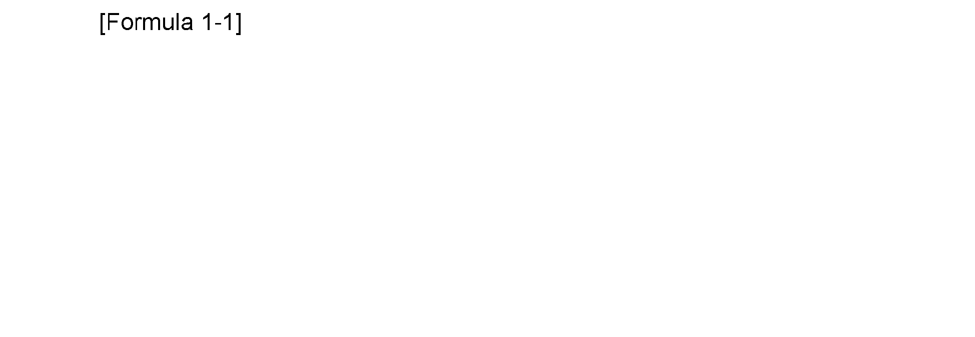

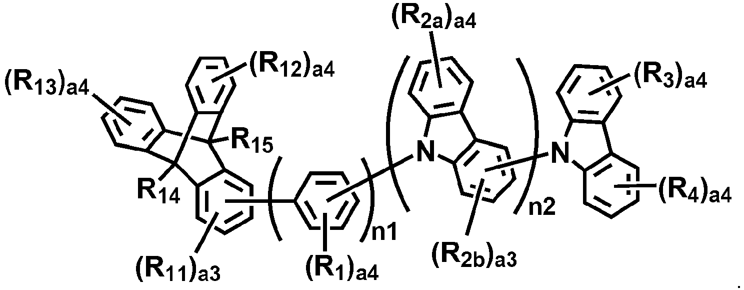

In Formula 1-1,
R₁, R₃, R₄, R₁₁ to R₁₅, n1, n2, a3, and a4 may each be the same as described in Formula 1, and
R₂ₐ and R_{2b} may each independently be the same as described in connection with R₂ in Formula 1.

In an embodiment, the organic compound may include at least one deuterium.

In an embodiment, the organic compound may be one of Compounds 1 to 100, or the organic compound may be a compound in which at least one hydrogen atom in one of Compounds 1 to 100 is substituted with deuterium:

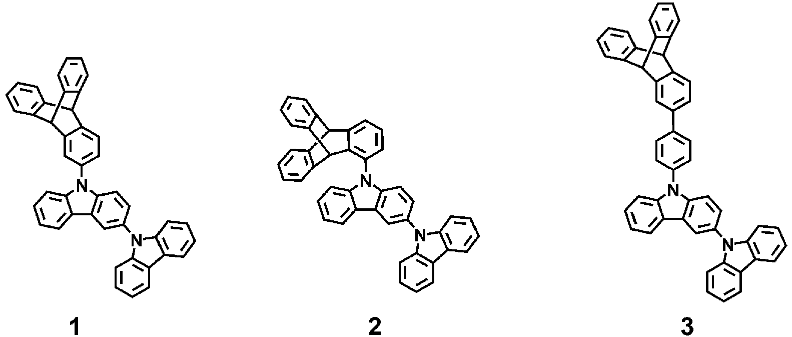

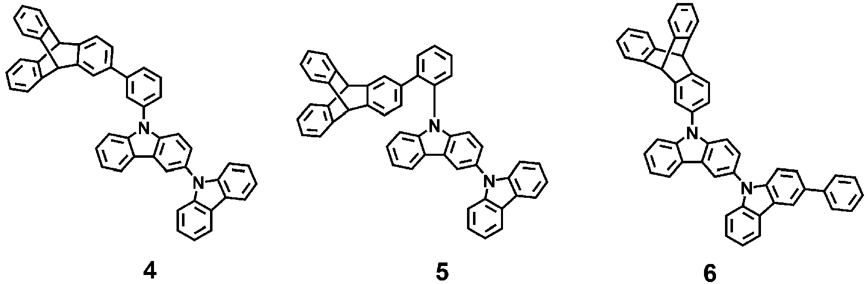

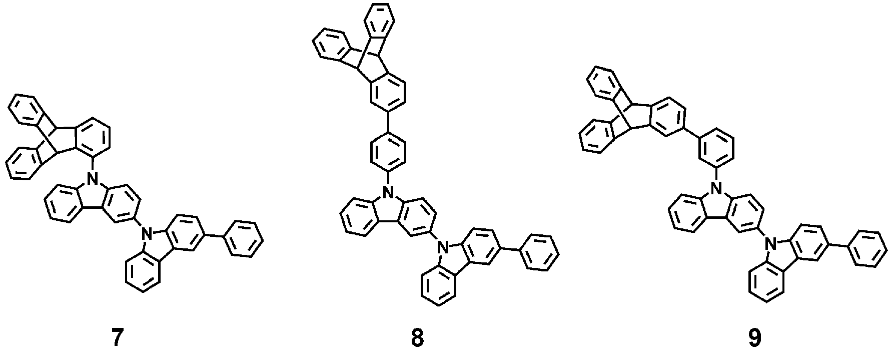

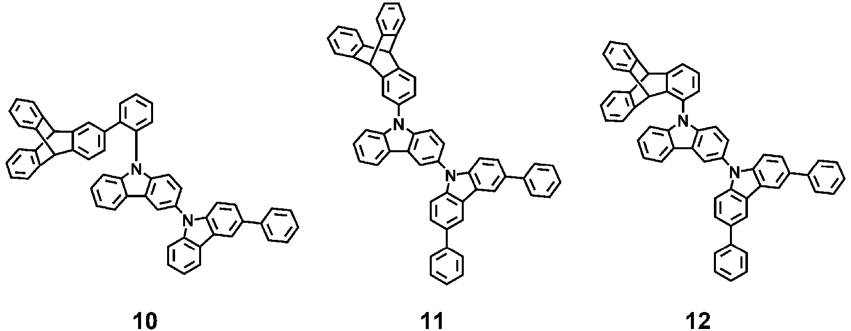

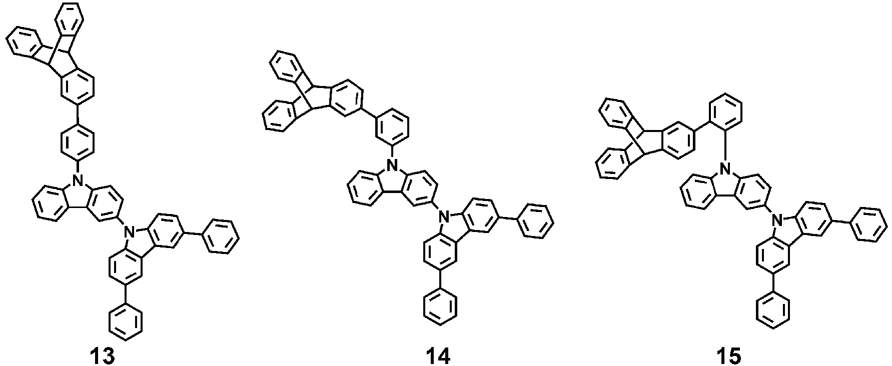

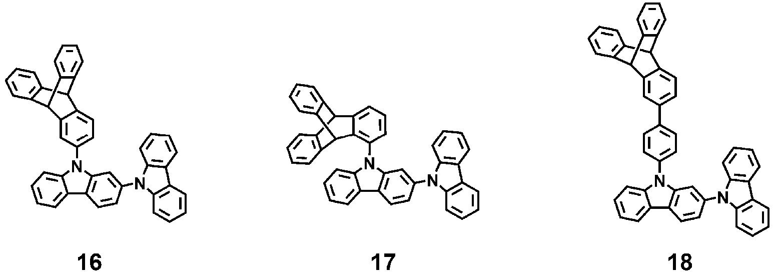

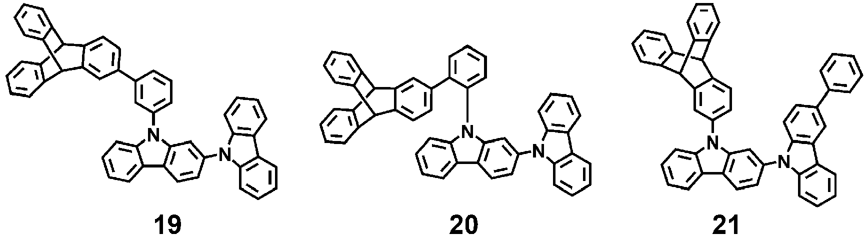

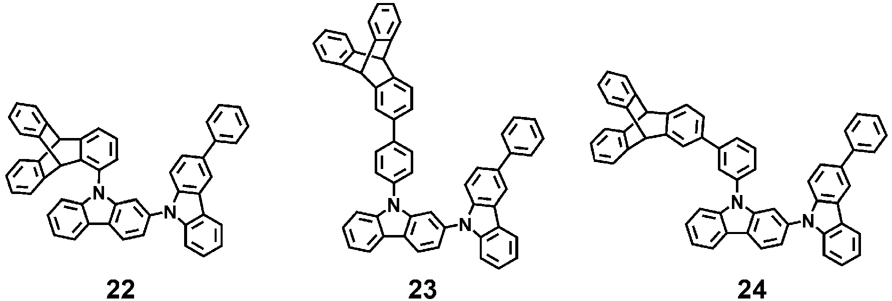

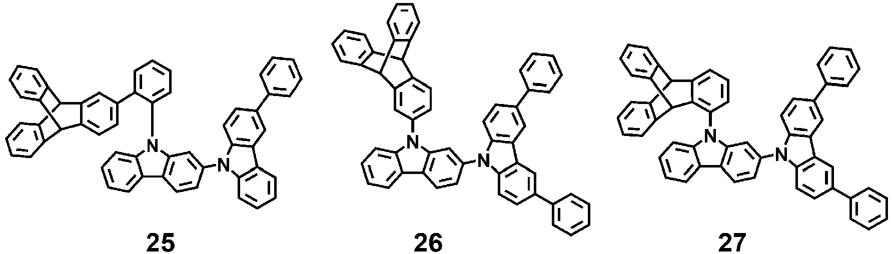

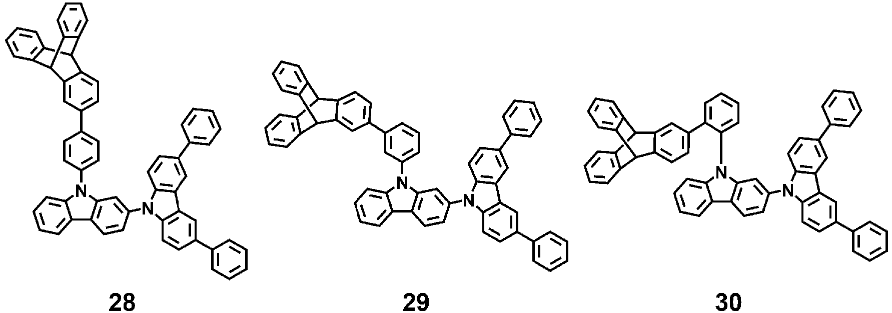

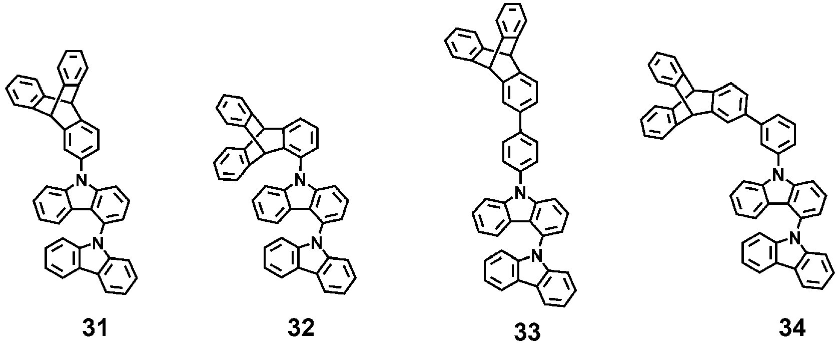

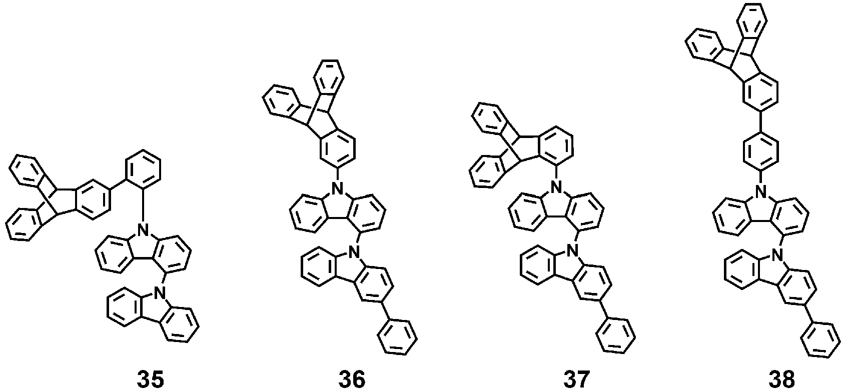

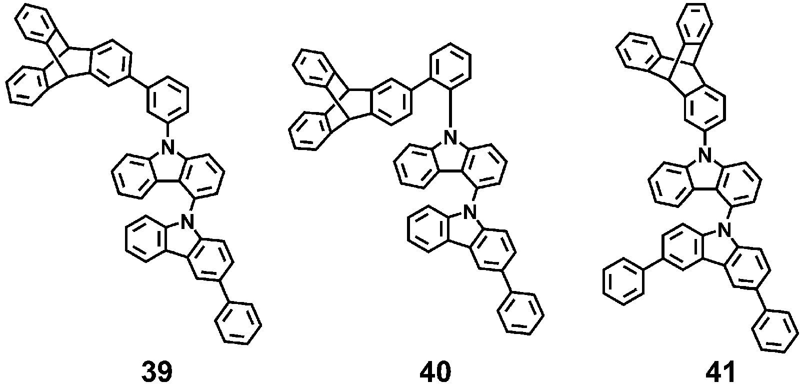

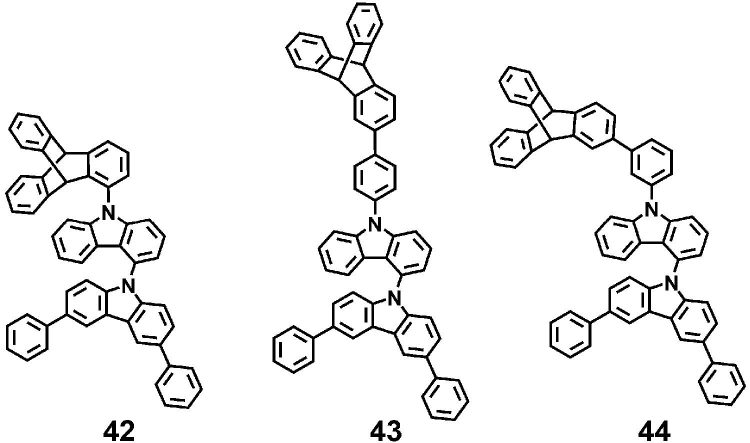

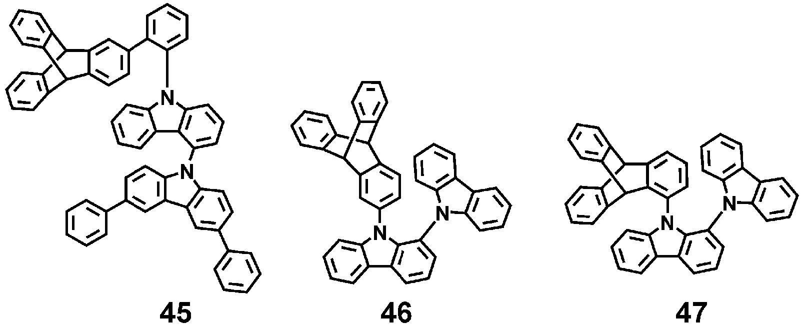

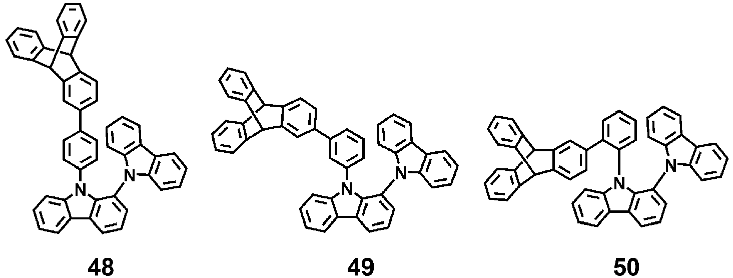

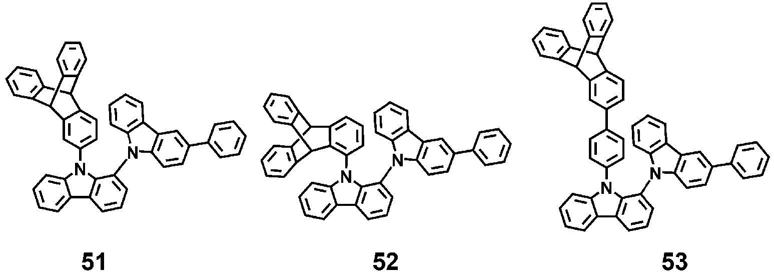

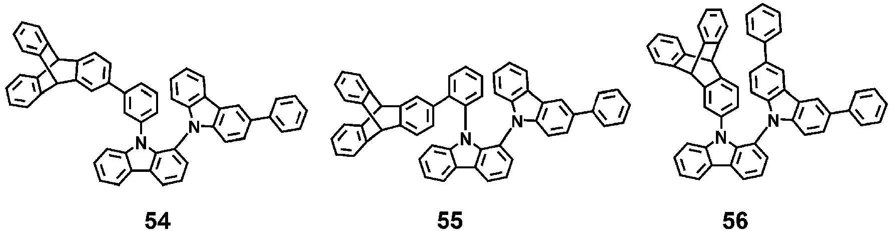

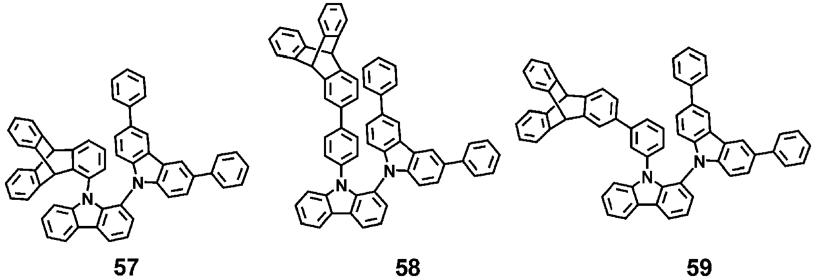

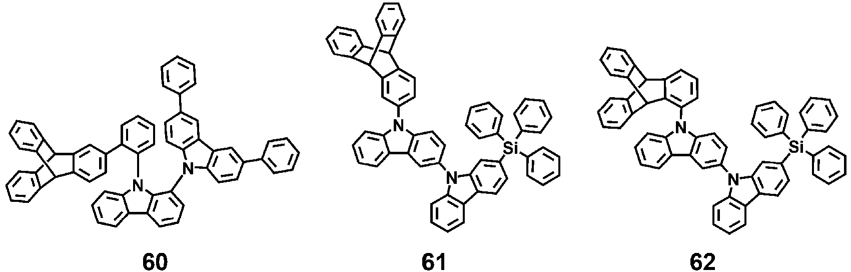

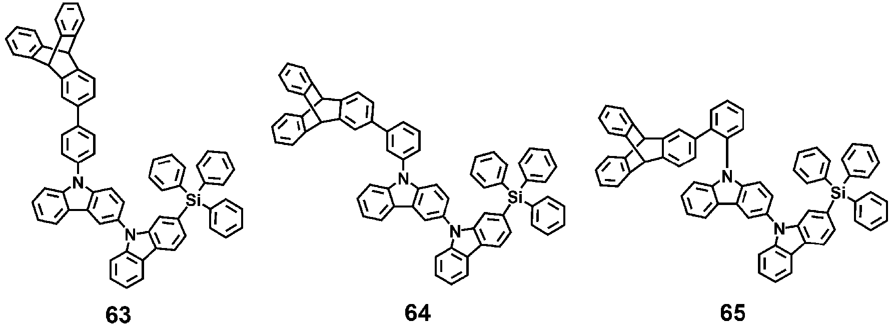

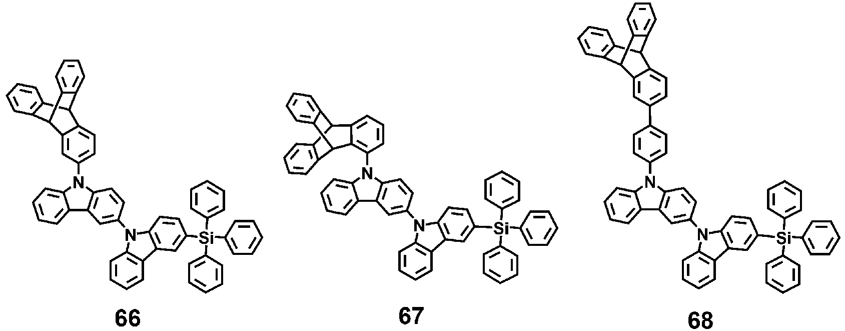

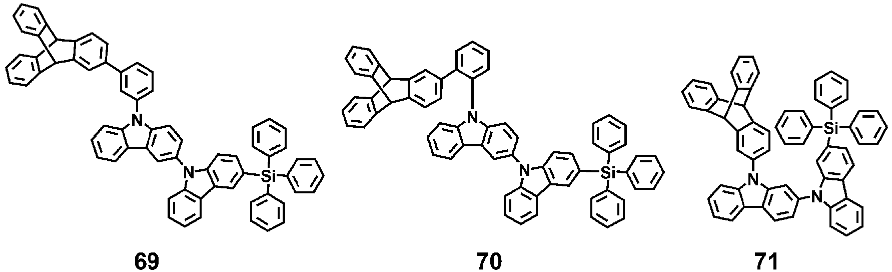

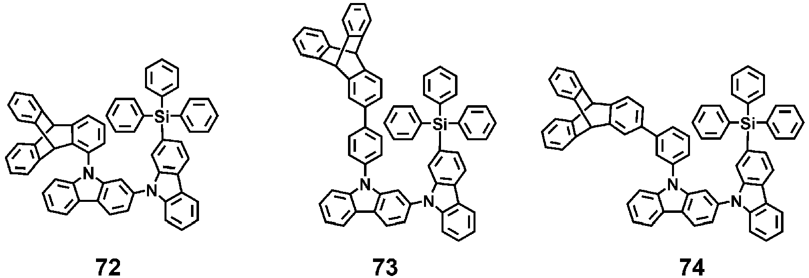

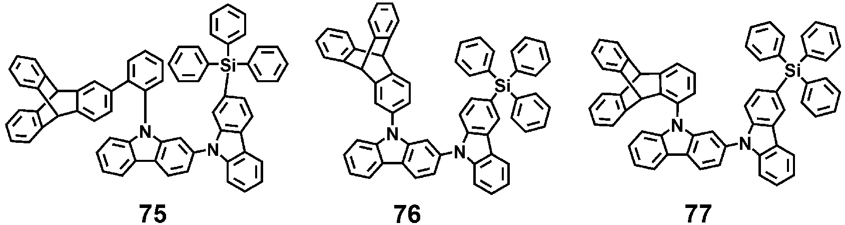

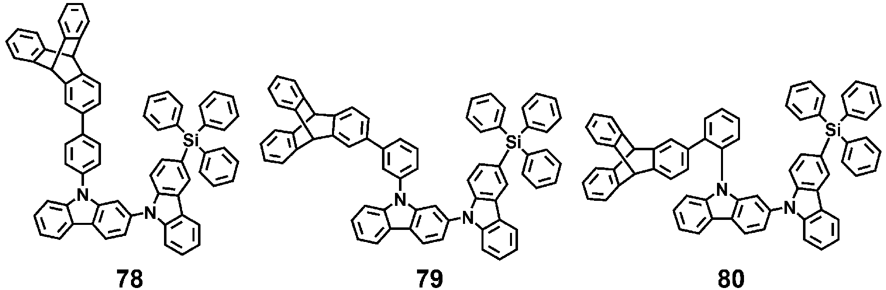

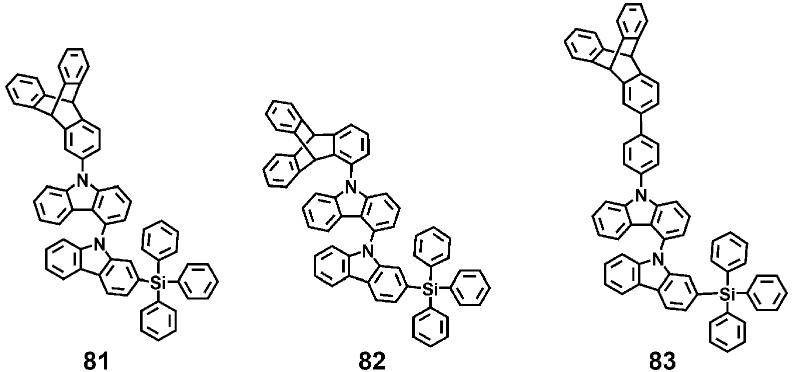

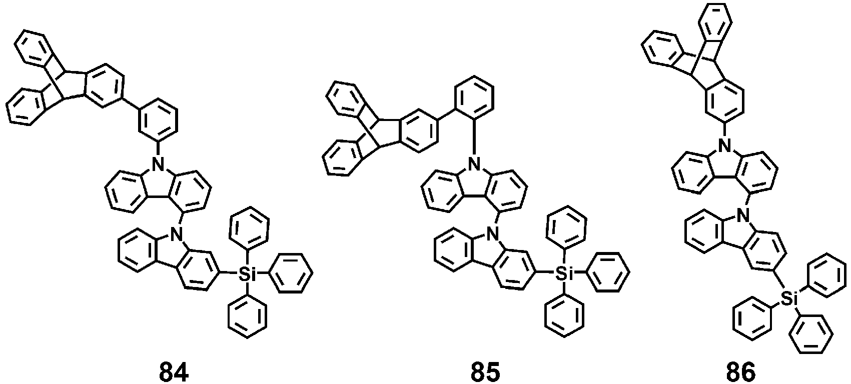

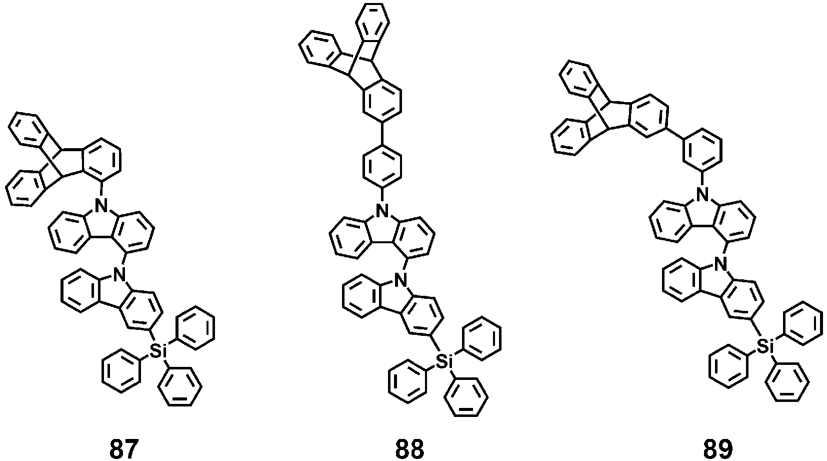

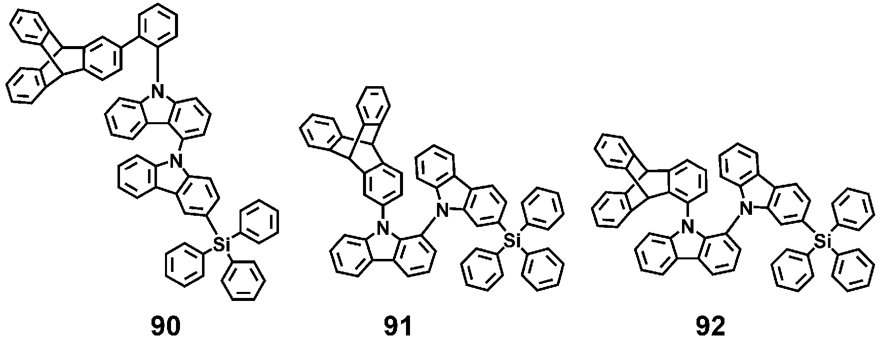

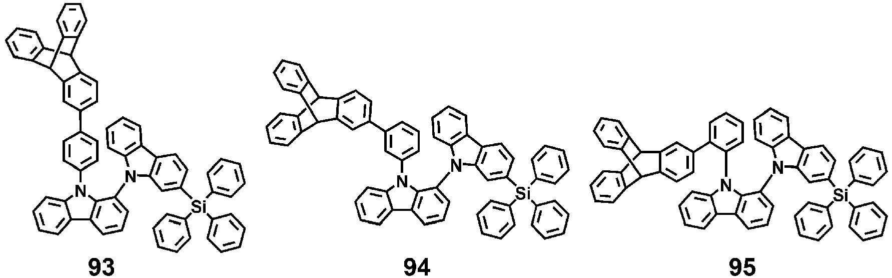

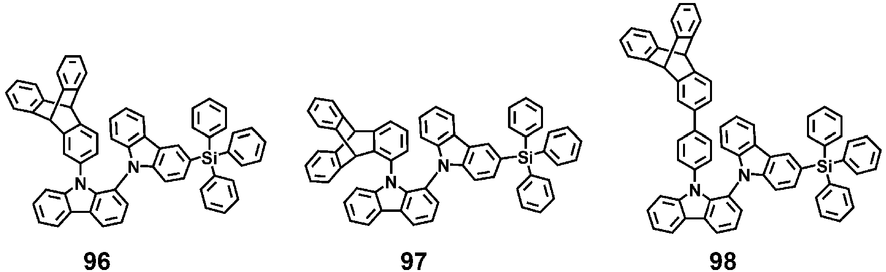

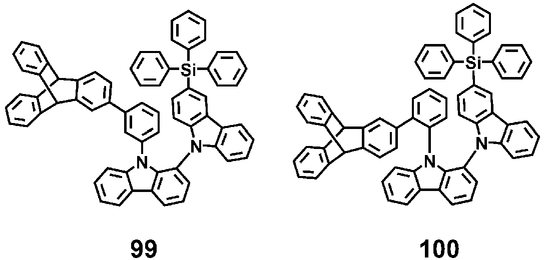

In an embodiment, the organic compound may be one of Compounds 1A, 1B, 3A, and 4A:

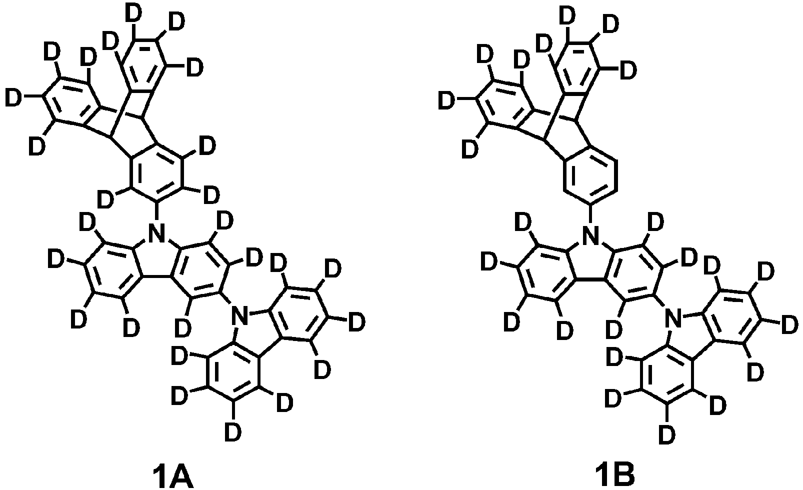

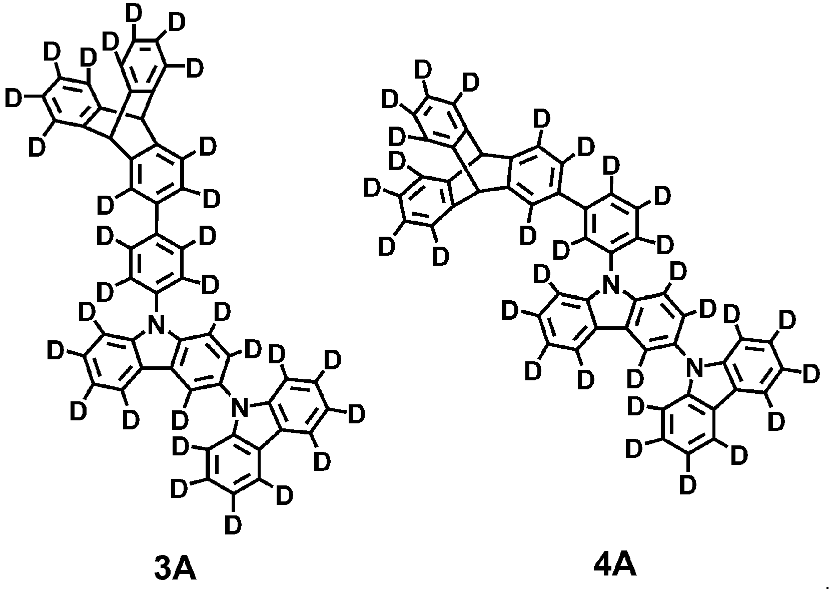

The organic compound represented by Formula 1 may have a large molecular weight while maintaining a short conjugation length by including a triptycene group (for example, the triptycene moiety in Formula 1) instead of a terphenyl group. In the organic compound, n2 may be 1 or 2 so that two or more carbazole groups (for example, the first carbazole group and the second carbazole group in Formula 1) may be included, and three benzene rings in the triptycene group may not be directly linked to -Si(Q₁)(Q₂)(Q₃), such as a triphenylsilyl group (-Si(Ph)₃). Accordingly, the organic compound may have high thermal stability and improved charge transport ability while maintaining a high glass transition temperature and a high triplet (T₁) energy level. Therefore, when the organic compound is applied to a light-emitting device, a light-emitting device may have a low driving voltage and high efficiency while having an effectively improved lifespan.

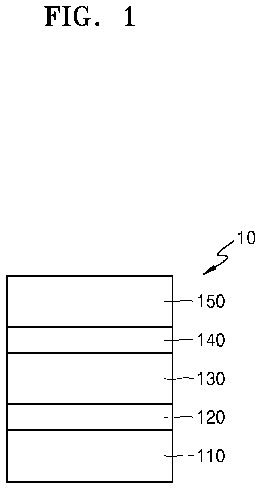

### [Description of FIG. 1]

FIG. 1 is a schematic cross-sectional view of a light-emitting device 10 according to an embodiment. The light-emitting device 10 includes a first electrode 110, an interlayer, and a second electrode 150. The interlayer may include the hole transport region 120, the emission layer 130, and the electron transport region 140.

Hereinafter, a structure of the light-emitting device 10 according to an embodiment and a method of manufacturing the light-emitting device 10 may be described with reference to FIG. 1.

### [First electrode 110]

In FIG. 1, a substrate may be further included under the first electrode 110 or on the second electrode 150. In an embodiment, the substrate may be a glass substrate or a plastic substrate. The substrate may be a flexible substrate. For example, the substrate may include plastics with excellent heat resistance and durability, such as polyimide, polyethylene terephthalate (PET), polycarbonate, polyethylene naphthalate, polyarylate (PAR), polyetherimide, or any combination thereof.

The first electrode 110 may be formed by depositing or sputtering a material for forming the first electrode 110 on the substrate. When the first electrode 110 is an anode, a material for forming the first electrode 110 may be a high-work function material that facilitates injection of holes.

The first electrode 110 may be a reflective electrode, a semi-transmissive electrode, or a transmissive electrode. When the first electrode 110 is a transmissive electrode, a material for forming the first electrode 110 may include indium tin oxide (ITO), indium zinc oxide (IZO), tin oxide (SnO₂), zinc oxide (ZnO), or any combination thereof. When the first electrode 110 is a semi-transmissive electrode or a reflective electrode, a material for forming the first electrode 110 may include magnesium (Mg), silver (Ag), aluminium (Al), aluminium-lithium (Al-Li), calcium (Ca), magnesium-indium (Mg-In), magnesium-silver (Mg-Ag), or any combination thereof.

The first electrode 110 may have a structure consisting of a single layer or a structure including multiple layers. In an embodiment, the first electrode 110 may have a three-layered structure of ITO/Ag/ITO.

### [Interlayer]

The interlayer may be arranged on the first electrode 110. The interlayer may include the hole transport region 120, the emission layer 130, and the electron transport region 140.

The interlayer may include various organic materials, a metal-containing compound such as an organometallic compound, an inorganic material such as quantum dots, and the like.

In an embodiment, the interlayer may include two or more emitting units stacked between the first electrode 110 and the second electrode 150, and at least one charge generation layer between adjacent units among the two or more emitting units. When the interlayer includes the two or more light-emitting units and the at least one charge generation layer, the light-emitting device 10 may be a tandem light-emitting device.

### [Hole transport region 120]

The hole transport region 120 may have a structure consisting of a layer consisting of a single material, a structure consisting of a layer including different materials, or a structure including multiple layers including different materials.

The hole transport region 120 may include a hole injection layer, a hole transport layer, an emission auxiliary layer, an electron-blocking layer, or any combination thereof.

In an embodiment, the hole transport region 120 may have a hole injection layer/hole transport layer structure, a hole injection layer/hole transport layer/emission auxiliary layer structure, a hole injection layer/emission auxiliary layer structure, a hole transport layer/emission auxiliary layer structure, or a hole injection layer/hole transport layer/electron-blocking layer structure, wherein the layers of each structure may be stacked from the first electrode 110 in its respective stated order, but the structure of the hole transport region 120 is not limited thereto.

In embodiments, the hole transport region 120 may include a compound represented by Formula 201, a compound represented by Formula 202, or any combination thereof:

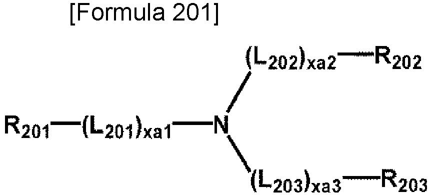

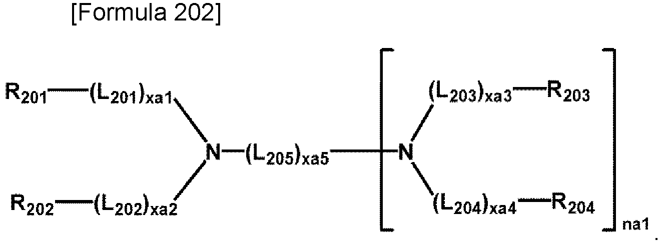

In Formulae 201 and 202,
L₂₀₁ to L₂₀₄ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
L₂₀₅ may be *-O-*', *-S-*', *-N(Q₂₀₁)-*', a C₁-C₂₀ alkylene group unsubstituted or substituted with at least one R₁₀ₐ, a C₂-C₂₀ alkenylene group unsubstituted or substituted with at least one R₁₀ₐ, a C₃-C₆₀ carbocyclic group unsubstituted or substituted with at least one R₁₀ₐ, or a C₁-C₆₀ heterocyclic group unsubstituted or substituted with at least one R₁₀ₐ,
xa1 to xa4 may each independently be an integer from 0 to 5,
xa5 may be an integer from 1 to 10,
R₂₀₁ to R₂₀₄ and Q₂₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
R₂₀₁ and R₂₀₂ may optionally be linked to each other via a single bond, a C₁-C₅ alkylene group that is unsubstituted or substituted with at least one R₁₀ₐ, or a C₂-C₅ alkenylene group that is unsubstituted or substituted with at least one R₁₀ₐ to form a C₈-C₆₀ polycyclic group (for example, a carbazole group) that is unsubstituted or substituted with at least one R₁₀ₐ (for example, Compound HT16),
R₂₀₃ and R₂₀₄ may optionally be linked to each other via a single bond, a C₁-C₅ alkylene group unsubstituted or substituted with at least one R₁₀ₐ, or a C₂-C₅ alkenylene group unsubstituted or substituted with at least one R₁₀ₐ, to form a C₈-C₆₀ polycyclic group unsubstituted or substituted with at least one R₁₀ₐ, and
na1 may be an integer from 1 to 4.

In an embodiment, the compound represented by Formula 201 and the compound represented by Formula 202 may each independently include at least one of groups represented by Formulae CY201 to CY217:

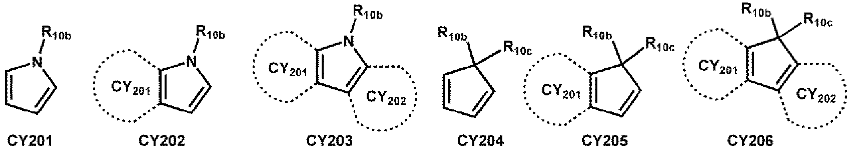

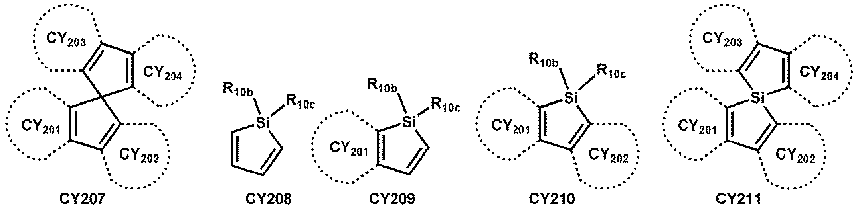

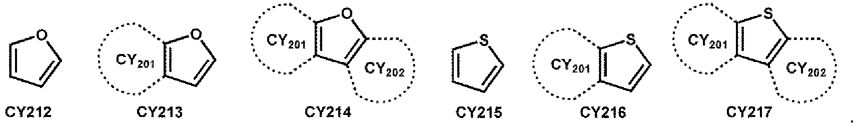

In Formulae CY201 to CY217, R_{10b} and R_{10c} may each independently be the same as described in connection with R₁₀ₐ, ring CY₂₀₁ to ring CY₂₀₄ may each independently be a C₃-C₂₀ carbocyclic group or a C₁-C₂₀ heterocyclic group, and at least one hydrogen in Formulae CY201 to CY217 may be unsubstituted or substituted with R₁₀ₐ.

In an embodiment, in Formulae CY201 to CY217, ring CY₂₀₁ to ring CY₂₀₄ may each independently be a benzene group, a naphthalene group, a phenanthrene group, or an anthracene group.

In an embodiment, the compound represented by Formula 201 and the compound represented by Formula 202 may include at least one of groups represented by Formulae CY201 to CY203.

In embodiments, the compound represented by Formula 201 may include at least one of groups represented by Formulae CY201 to CY203 and at least one of groups represented by Formulae CY204 to CY217.

In embodiments, in Formula 201, xa1 may be 1, R₂₀₁ may be a group represented by one of Formulae CY201 to CY203, xa2 may be 0, and R₂₀₂ may be a group represented by one of Formulae CY204 to CY207.

In embodiments, the compound represented by Formula 201 and the compound represented by Formula 202 may each not include groups represented by Formulae CY201 to CY203.

In embodiments, the compound represented by Formula 201 and the compound represented by Formula 202 may each not include groups represented by Formulae CY201 to CY203, and may each independently include at least one of groups represented by Formulae CY204 to CY217.

In embodiments, the compound represented by Formula 201 and the compound represented by Formula 202 may each not include groups represented by Formulae CY201 to CY217.

In an embodiment, the hole transport region 120 may include one of Compounds HT1 to HT46, m-MTDATA, TDATA, 2-TNATA, NPB (NPD), β-NPB, TPD, Spiro-TPD, Spiro-NPB, methylated NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), or any combination thereof:

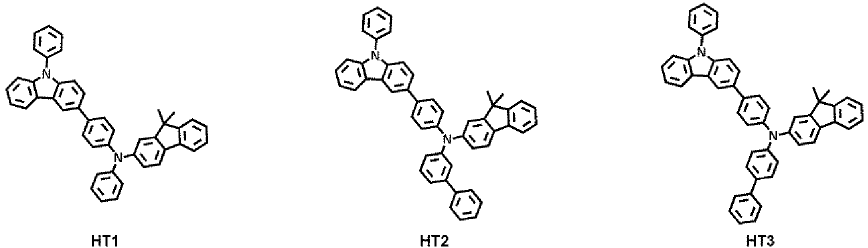

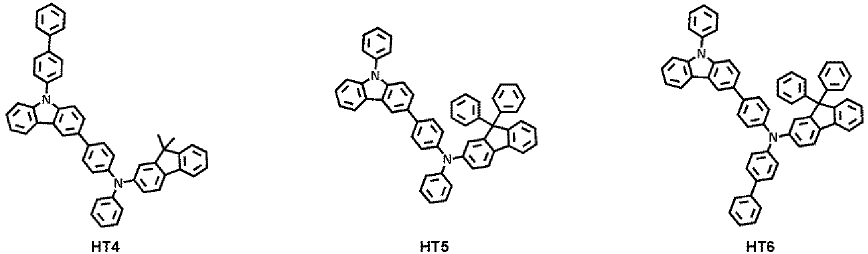

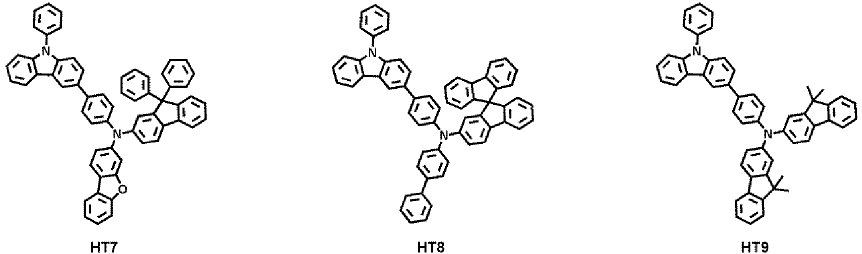

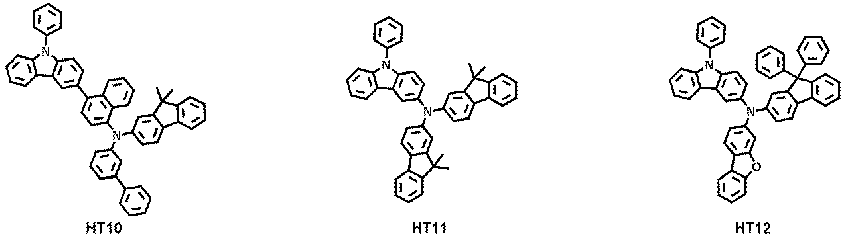

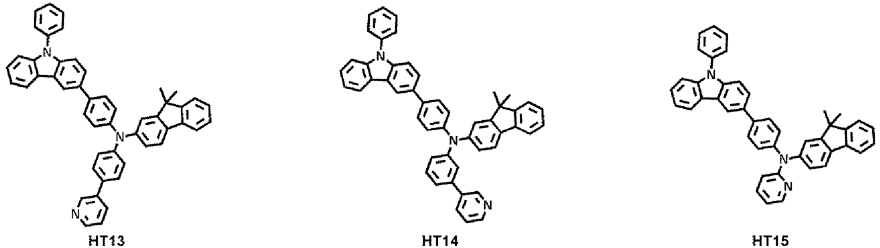

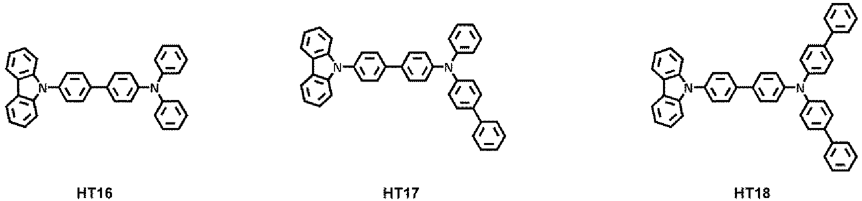

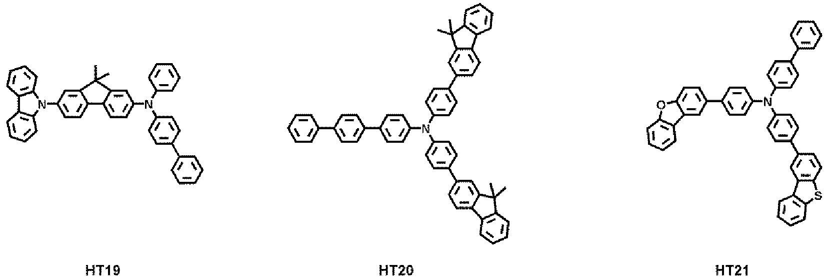

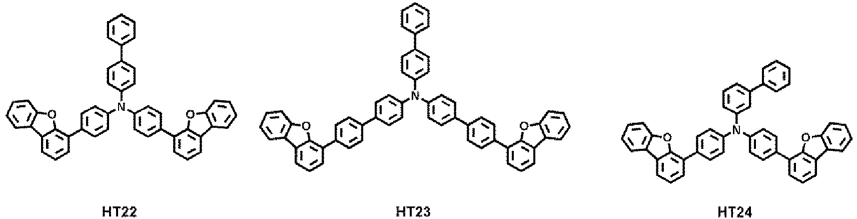

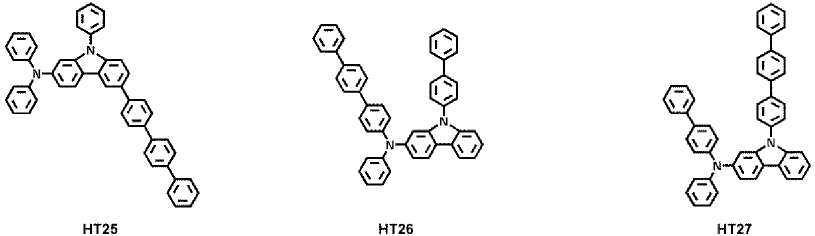

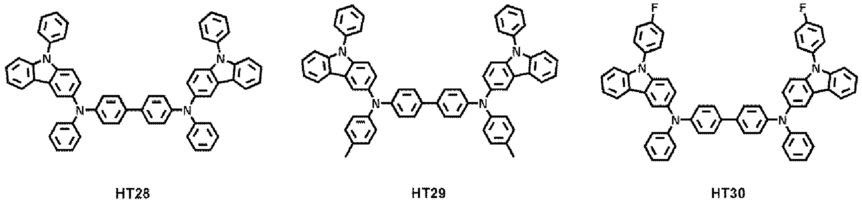

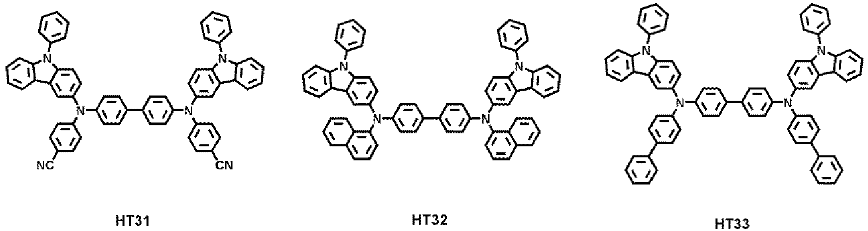

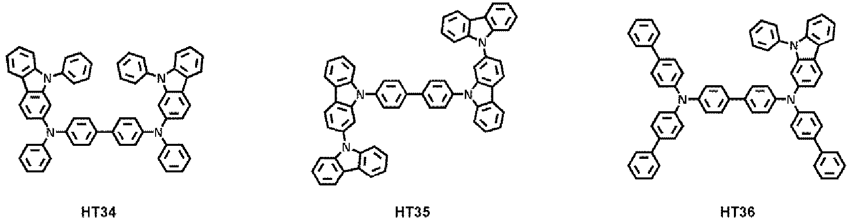

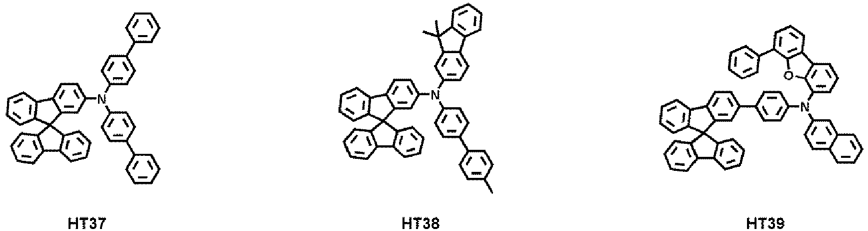

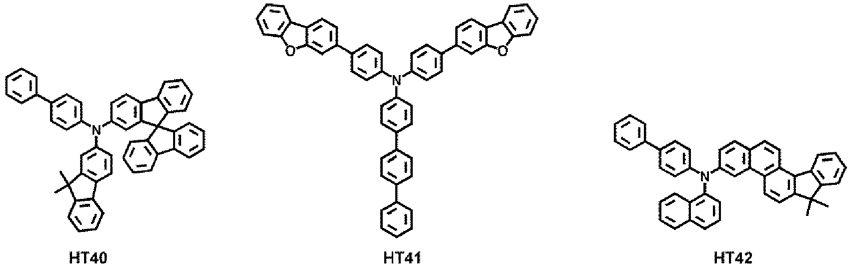

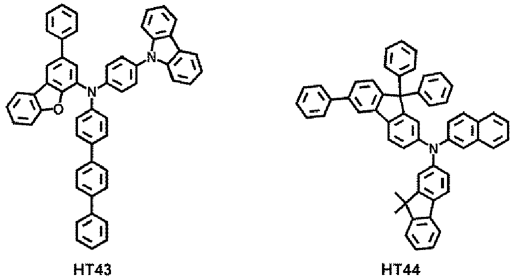

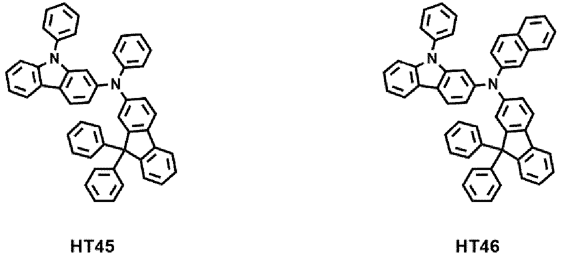

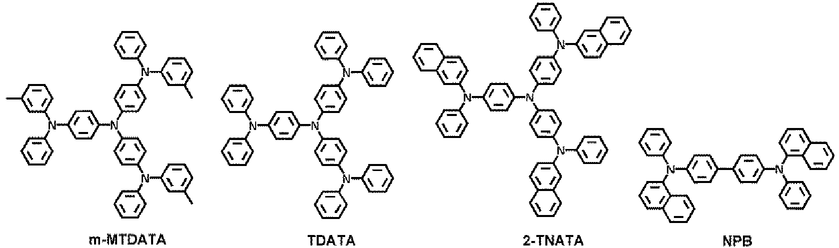

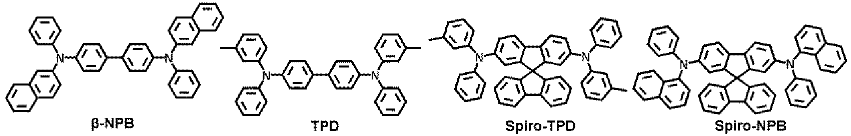

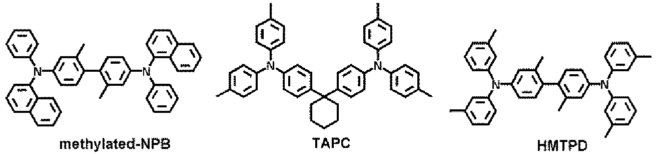

A thickness of the hole transport region 120 may be in a range of about 50 Å to about 10,000 Å. For example, the thickness of the hole transport region 120 may be in a range of about 100 Å to about 4,000 Å. When the hole transport region 120 includes a hole injection layer, a hole transport layer, or any combination thereof, a thickness of the hole injection layer may be in a range of about 100 Å to about 9,000 Å, and a thickness of the hole transport layer may be in a range of about 50 Å to about 2,000 Å. For example, the thickness of the hole injection layer may be in a range of about 100 Å to about 1,000 Å. For example, the thickness of the hole transport layer may be in a range of about 100 Å to about 1,500 Å. When the thicknesses of the hole transport region 120, the hole injection layer, and the hole transport layer are within the ranges described above, satisfactory hole transporting characteristics may be obtained without a substantial increase in driving voltage.

The emission auxiliary layer may increase light-emission efficiency by compensating for an optical resonance distance according to a wavelength of light emitted by the emission layer. The electron blocking layer may prevent electron leakage from the emission layer to the hole transport region 120. Materials that may be included in the hole transport region 120 may be included in the emission auxiliary layer and the electron blocking layer.

### [p-dopant]

The hole transport region 120 may further include, in addition to the aforementioned materials, a charge-generation material for the improvement of conductive properties. The charge-generating material may be substantially homogeneously or non-homogeneously dispersed (for example, as a single layer consisting of charge generating material) in the hole transport region 120.

The charge-generation material may be, for example, a p-dopant.

For example, the p-dopant may have a lowest unoccupied molecular orbital (LUMO) energy level less than or equal to about -3.5 eV.

In an embodiment, the p-dopant may include a quinone derivative, a cyano group-containing compound, a compound including element EL1 and element EL2, or any combination thereof.

Examples of a quinone derivative may include TCNQ and F4-TCNQ.

Examples of a cyano group-containing compound may include HAT-CN and a compound represented by Formula 221.

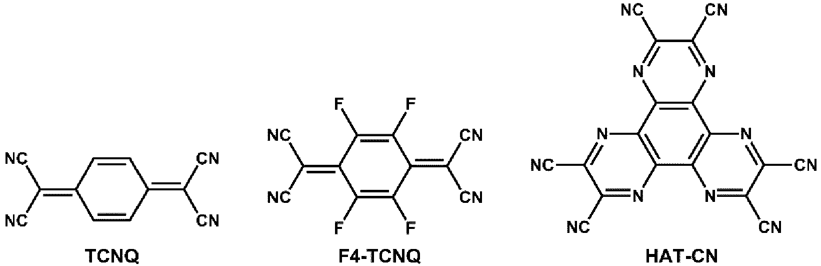

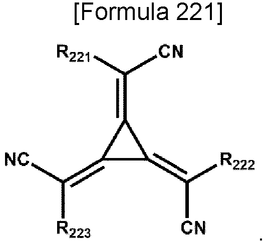

In Formula 221,
R₂₂₁ to R₂₂₃ may each independently be a C₃-C₆₀ carbocyclic group unsubstituted or substituted with at least one R₁₀ₐ or a C₁-C₆₀ heterocyclic group unsubstituted or substituted with at least one R₁₀ₐ, and
at least one of R₂₂₁ to R₂₂₃ may each independently be a C₃-C₆₀ carbocyclic group or a C₁-C₆₀ heterocyclic group, each substituted with: a cyano group; -F; -Cl; - Br; -I; a C₁-C₂₀ alkyl group substituted with a cyano group, -F, -Cl, -Br, -I, or any combination thereof; or any combination thereof.

In the compound including element EL1 and element EL2, element EL1 may be a metal, a metalloid, or any combination thereof, and element EL2 may be a non-metal, a metalloid, or any combination thereof.

Examples of a metal may include: an alkali metal (for example, lithium (Li), sodium (Na), potassium (K), rubidium (Rb), cesium (Cs), etc.); an alkaline earth metal (for example, beryllium (Be), magnesium (Mg), calcium (Ca), strontium (Sr), barium (Ba), etc.); a transition metal (for example, titanium (Ti), zirconium (Zr), hafnium (Hf), vanadium (V), niobium (Nb), tantalum (Ta), chromium (Cr), molybdenum (Mo), tungsten (W), manganese (Mn), technetium (Tc), rhenium (Re), iron (Fe), ruthenium (Ru), osmium (Os), cobalt (Co), rhodium (Rh), iridium (Ir), nickel (Ni), palladium (Pd), platinum (Pt), copper (Cu), silver (Ag), gold (Au), etc.); a post-transition metal (for example, zinc (Zn), indium (In), tin (Sn), etc.); and a lanthanide metal (for example, lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), lutetium (Lu), etc.).

Examples of a metalloid may include silicon (Si), antimony (Sb), and tellurium (Te).

Examples of a non-metal may include oxygen (O) and halogen (for example, F, Cl, Br, I, etc.).

Examples of a compound including element EL1 and element EL2 may include a metal oxide, a metal halide (for example, a metal fluoride, a metal chloride, a metal bromide, a metal iodide, etc.), a metalloid halide (for example, a metalloid fluoride, a metalloid chloride, a metalloid bromide, a metalloid iodide, etc.), a metal telluride, or any combination thereof.

Examples of a metal oxide may include a tungsten oxide (for example, WO, W₂O₃, WO₂, WO₃, W₂O₅, etc.), a vanadium oxide (for example, VO, V₂O₃, VO₂, V₂O₅, etc.), a molybdenum oxide (for example, MoO, Mo₂O₃, MoO₂, MoO₃, Mo₂O₅, etc.), a rhenium oxide (for example, ReO₃, etc.), etc.

Examples of a metal halide may include an alkali metal halide, an alkaline earth metal halide, a transition metal halide, a post-transition metal halide, and a lanthanide metal halide.

Examples of an alkali metal halide may include LiF, NaF, KF, RbF, CsF, LiCl, NaCl, KCI, RbCl, CsCl, LiBr, NaBr, KBr, RbBr, CsBr, Lil, Nal, KI, Rbl, and Csl.

Examples of an alkaline earth metal halide may include BeF₂, MgF₂, CaF₂, SrF₂, BaF₂, BeCl₂, MgCl₂, CaCl₂, SrCl₂, BaCl₂, BeBr₂, MgBr₂, CaBr₂, SrBr₂, BaBr₂, BeI₂, MgI₂, CaI₂, SrI₂, and BaI₂.

Examples of a transition metal halide may include a titanium halide (for example, TiF₄, TiCl₄, TiBr₄, TiI₄, etc.), a zirconium halide (for example, ZrF₄, ZrCl₄, ZrBr₄, ZrI₄, etc.), a hafnium halide (for example, HfF₄, HfCl₄, HfBr₄, HfI₄, etc.), a vanadium halide (for example, VF₃, VCl₃, VBr₃, VI₃, etc.), a niobium halide (for example, NbF₃, NbCl₃, NbBr₃, NbI₃, etc.), a tantalum halide (for example, TaF₃, TaCl₃, TaBr₃, TaI₃, etc.), a chromium halide (for example, CrF₃, CrCl₃, CrBr₃, CrI₃, etc.), a molybdenum halide (for example, MoF₃, MoCl₃, MoBr₃, MoI₃, etc.), a tungsten halide (for example, WF₃, WCl₃, WBr₃, WI₃, etc.), a manganese halide (for example, MnF₂, MnCl₂, MnBr₂, MnI₂, etc.), a technetium halide (for example, TcF₂, TcCl₂, TcBr₂, TcI₂, etc.), a rhenium halide (for example, ReF₂, ReCl₂, ReBr₂, ReI₂, etc.), an iron halide (for example, FeF₂, FeCl₂, FeBr₂, FeI₂, etc.), a ruthenium halide (for example, RuF₂, RuCl₂, RuBr₂, RuI₂, etc.), an osmium halide (for example, OsF₂, OsCl₂, OsBr₂, OsI₂, etc.), a cobalt halide (for example, CoF₂, CoCl₂, CoBr₂, CoI₂, etc.), a rhodium halide (for example, RhF₂, RhCl₂, RhBr₂, RhI₂, etc.), an iridium halide (for example, IrF₂, IrCl₂, IrBr₂, IrI₂, etc.), a nickel halide (for example, NiF₂, NiCl₂, NiBr₂, NiI₂, etc.), a palladium halide (for example, PdF₂, PdCl₂, PdBr₂, PdI₂, etc.), a platinum halide (for example, PtF₂, PtCl₂, PtBr₂, PtI₂, etc.), a copper halide (for example, CuF, CuCl, CuBr, Cul, etc.), a silver halide (for example, AgF, AgCl, AgBr, Agl, etc.), and a gold halide (for example, AuF, AuCl, AuBr, Aul, etc.).

Examples of a post-transition metal halide may include a zinc halide (for example, ZnF₂, ZnCl₂, ZnBr₂, ZnI₂, etc.), an indium halide (for example, InI₃, etc.), a tin halide (for example, SnI₂, etc.), etc.

Examples of a lanthanide metal halide may include YbF, YbF₂, YbF₃, SmF₃, YbCl, YbCl₂, YbCl₃, SmCl₃, YbBr, YbBr₂, YbBr₃, SmBr₃, Ybl, YbI₂, YbI₃, SmI₃, etc.

Examples of a metalloid halide may include an antimony halide (for example, SbCl₅, etc.).

Examples of a metal telluride may include an alkali metal telluride (for example, Li₂Te, Na₂Te, K₂Te, Rb₂Te, Cs₂Te, etc.), an alkaline earth metal telluride (for example, BeTe, MgTe, CaTe, SrTe, BaTe, etc.), a transition metal telluride (for example, TiTe₂, ZrTe₂, HfTe₂, V₂Te₃, Nb₂Te₃, Ta₂Te₃, Cr₂Te₃, Mo₂Te₃, W₂Te₃, MnTe, TcTe, ReTe, FeTe, RuTe, OsTe, CoTe, RhTe, IrTe, NiTe, PdTe, PtTe, Cu₂Te, CuTe, Ag₂Te, AgTe, Au₂Te, etc.), a post-transition metal telluride (for example, ZnTe, etc.), and a lanthanide metal telluride (for example, LaTe, CeTe, PrTe, NdTe, PmTe, EuTe, GdTe, TbTe, DyTe, HoTe, ErTe, TmTe, YbTe, LuTe, etc.).

### [Emission layer 130]

When the light-emitting device 10 is a full-color light-emitting device, the emission layer 130 may be patterned into a red emission layer, a green emission layer, and/or a blue emission layer, according to a subpixel. In an embodiment, the emission layer 130 may have a stacked structure in which two or more layers among a red emission layer, a green emission layer, and a blue emission layer may contact each other or may be separated from each other to emit white light. In embodiments, the emission layer may have a structure in which two or more materials among a red light-emitting material, a green light-emitting material, and a blue light-emitting material may be mixed with each other in a single layer to emit white light.

The emission layer 130 may include a host and a dopant. The dopant may include a phosphorescent dopant, a fluorescent dopant, or any combination thereof.

An amount of the dopant in the emission layer 130 may be in a range of about 0.01 parts by weight to about 15 parts by weight, with respect to 100 parts by weight of the host.

The emission layer 130 may include quantum dots.

The emission layer 130 may include a delayed fluorescence material. The delayed fluorescence material may serve as a host or as a dopant in the emission layer.

A thickness of the emission layer 130 may be in a range of about 100 Å to about 1,000 Å. For example, the thickness of the emission layer 130 may be in a range of about 200 Å to about 600 Å. When the thickness of the emission layer 130 is within any of the ranges described above, excellent luminescence characteristics may be obtained without a substantial increase in driving voltage.

### [Host]

In an embodiment, the host may include a compound represented by Formula 301:

[Formula 301] [Ar₃₀₁]x_{b11}-[(L₃₀₁)_{xb1}-R₃₀₁]_{xb21}

In Formula 301,
Ar₃₀₁ and L₃₀₁ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xb11 may be 1, 2, or 3,
xb1 may be an integer from 0 to 5,
R₃₀₁ may be hydrogen, deuterium, -F, -Cl, -Br, -I, a hydroxyl group, a cyano group, a nitro group, a C₁-C₆₀ alkyl group that is unsubstituted or substituted with at least one R₁₀ₐ, a C₂-C₆₀ alkenyl group that is unsubstituted or substituted with at least one R₁₀ₐ, a C₂-C₆₀ alkynyl group that is unsubstituted or substituted with at least one R₁₀ₐ, a C₁-C₆₀ alkoxy group that is unsubstituted or substituted with at least one R₁₀ₐ, a C₃-C₆₀ carbocyclic group that is unsubstituted or substituted with at least one R₁₀ₐ, a C₁-C₆₀ heterocyclic group that is unsubstituted or substituted with at least one R₁₀ₐ,-Si(Q₃₀₁)(Q₃₀₂)(Q₃₀₃), -N(Q₃₀₁)(Q₃₀₂), -B(Q₃₀₁)(Q₃₀₂), -C(=O)(Q₃₀₁), -S(=O)₂(Q₃₀₁), or - P(=O)(Q₃₀₁)(Q₃₀₂),
xb21 may be an integer from 1 to 5, and
Q₃₀₁ to Q₃₀₃ may each independently be the same as described in connection with Q1.

In an embodiment, in Formula 301, when xb11 is 2 or more, two or more of Ar₃₀₁ may be linked to each other via a single bond.

In an embodiment, the host may include a compound represented by Formula 301-1, a compound represented by Formula 301-2, or any combination thereof:

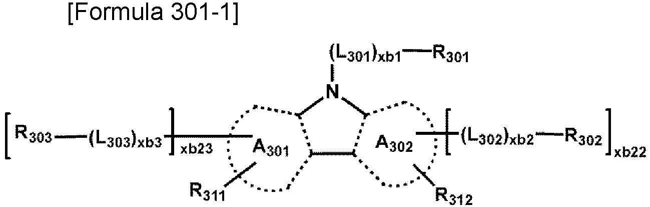

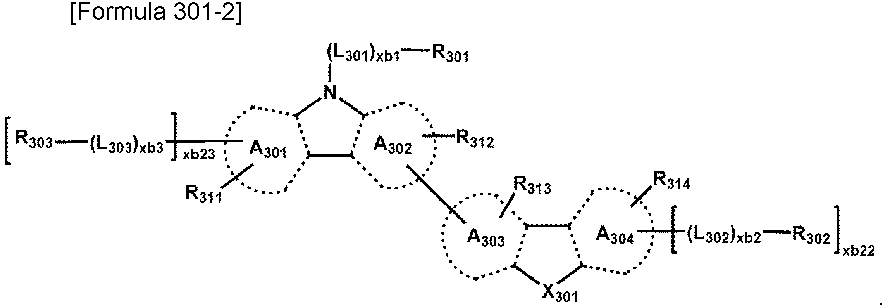

In Formulae 301-1 and 301-2,
ring A₃₀₁ to ring A₃₀₄ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
X₃₀₁ may be O, S, N[(L₃₀₄)_{xb4}-R₃₀₄], C(R₃₀₄)(R₃₀₅), or Si(R₃₀₄)(R₃₀₅),
xb22 and xb23 may each independently be 0, 1, or 2,
L₃₀₁, xb1, and R₃₀₁ may each independently be the same as described in the specification,
L₃₀₂ to L₃₀₄ may each independently be the same as described in connection with L₃₀₁,
xb2 to xb4 may each independently be the same as described in connection with xb1, and
R₃₀₂ to R₃₀₅ and R₃₁₁ to R₃₁₄ may each independently be the same as described in connection with R₃₀₁.

In an embodiment, the host may include an alkali earth metal complex, a post-transition metal complex, or any combination thereof. In an embodiment, the host may include a Be complex (for example, Compound H55), an Mg complex, a Zn complex, or any combination thereof.

In embodiments, the host may include one of Compounds H1 to H128, 9,10-di(2-naphthyl)anthracene (ADN); 2-methyl-9,10-bis(naphthalen-2-yl)anthracene (MADN), 9,10-di-(2-naphthyl)-2-t-butyl-anthracene (TBADN), 4,4'-bis(N-carbazolyl)-1,1'-biphenyl (CBP). 1,3-di(carbazol-9-yl)benzene (mCP), 1,3,5-tri(carbazol-9-yl)benzene (TCP), or any combination thereof:

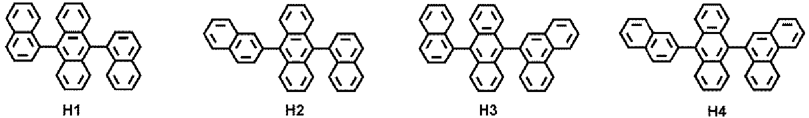

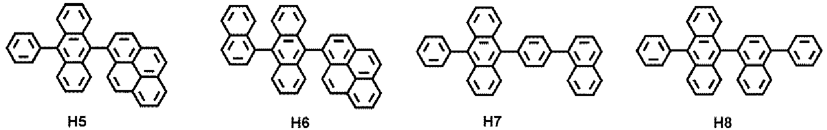

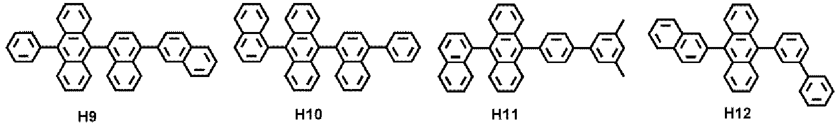

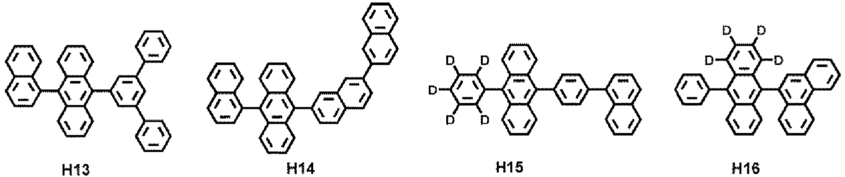

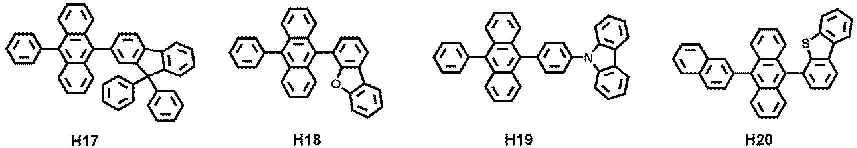

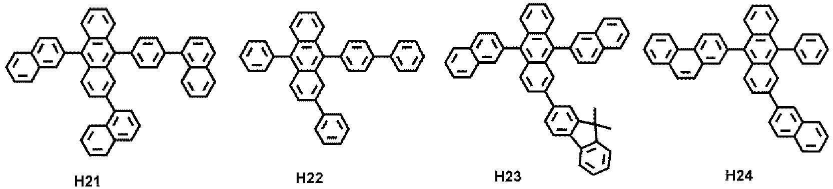

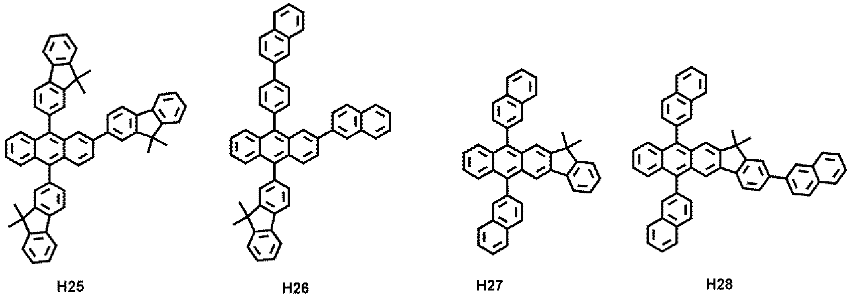

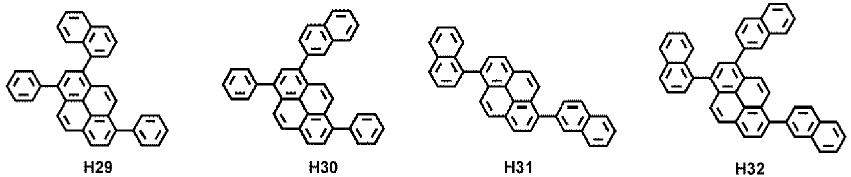

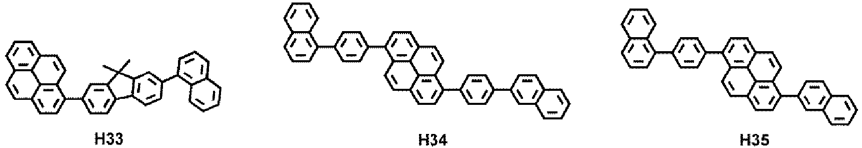

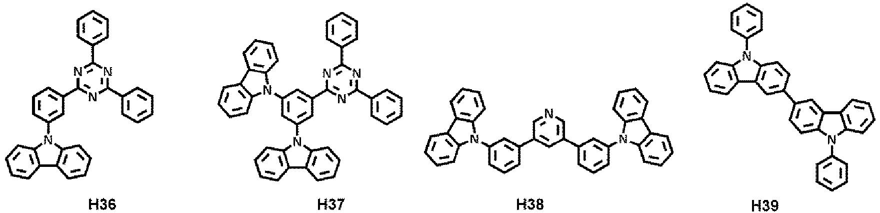

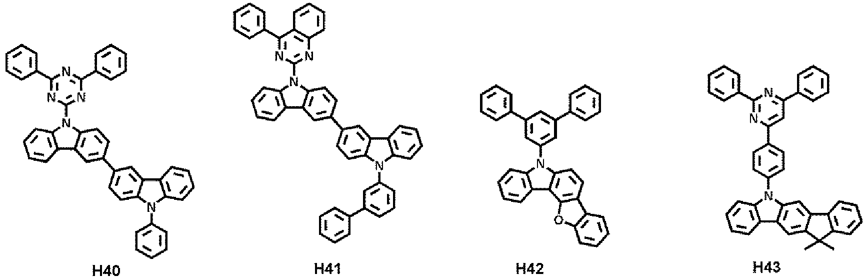

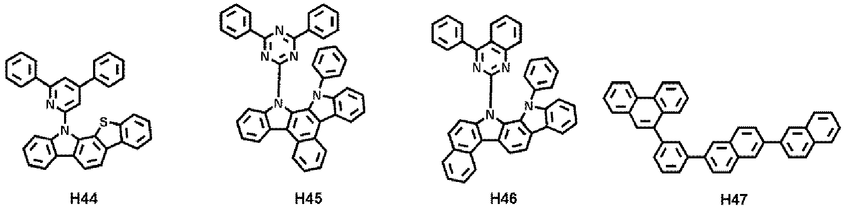

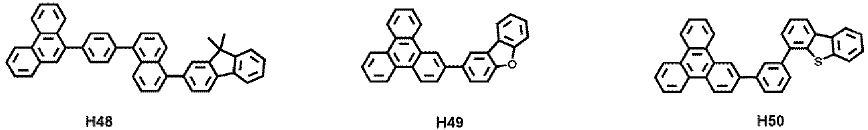

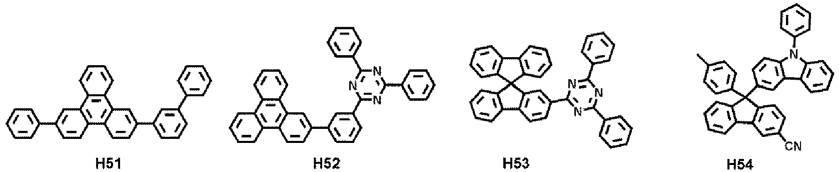

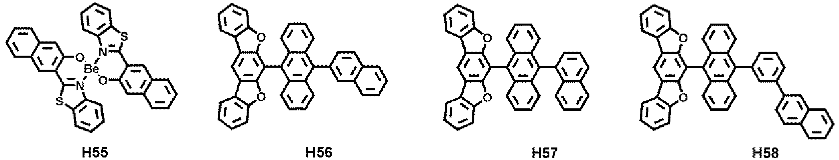

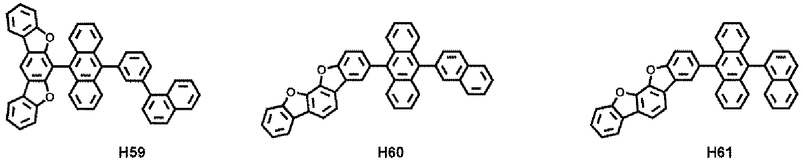

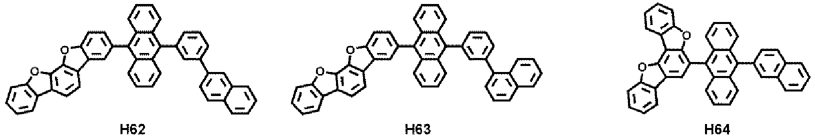

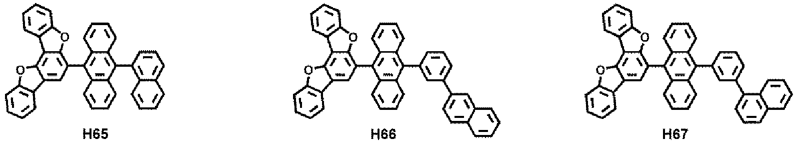

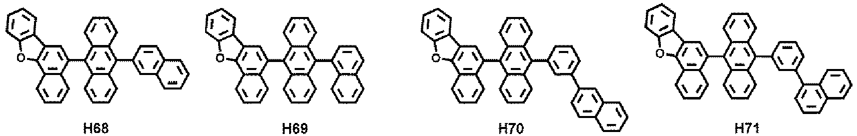

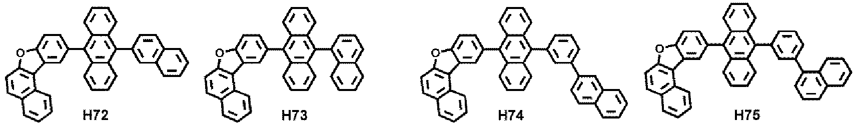

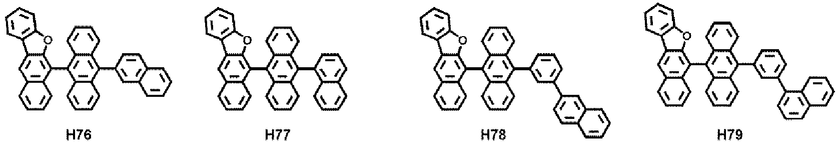

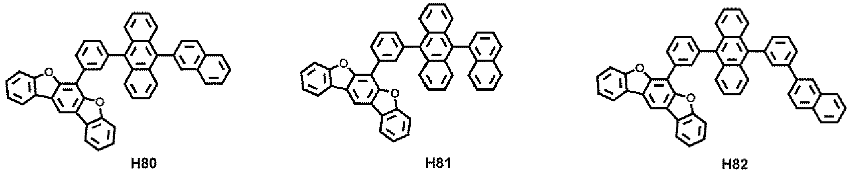

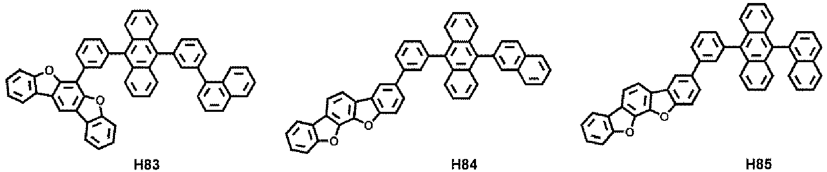

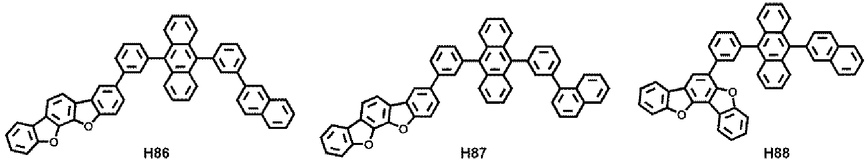

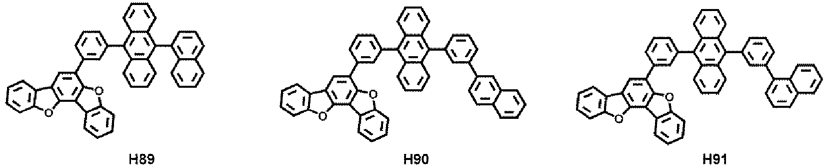

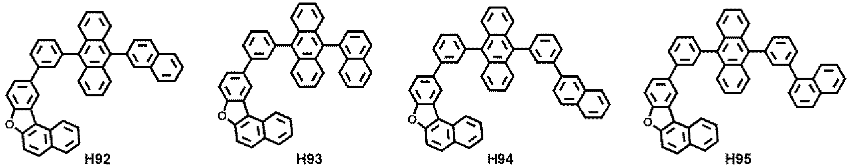

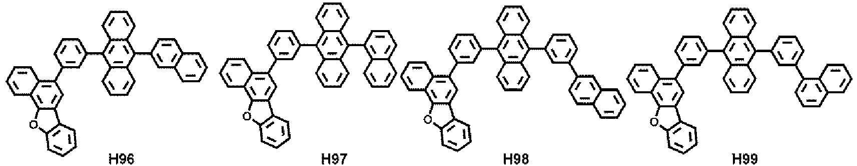

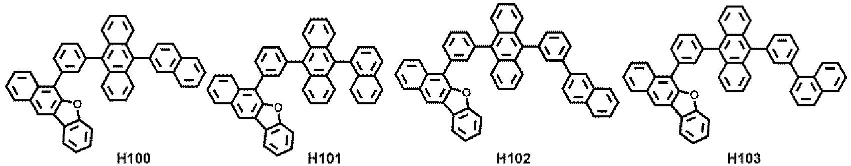

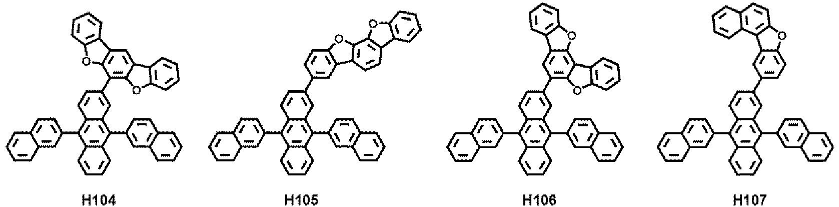

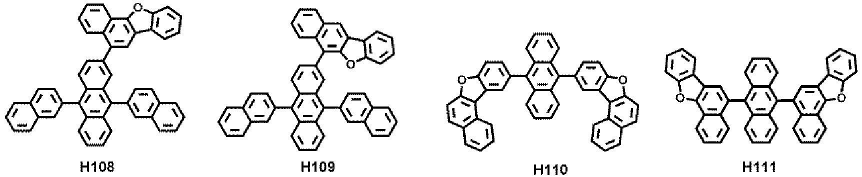

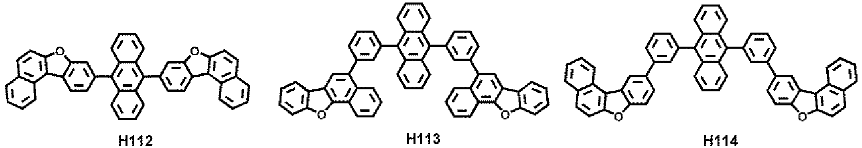

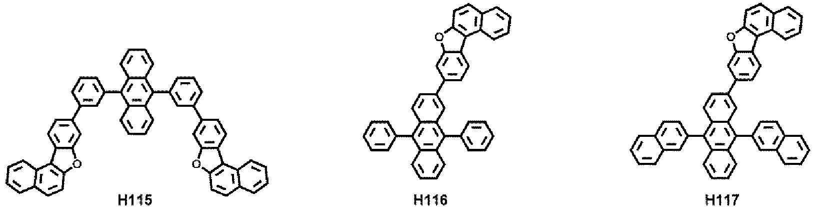

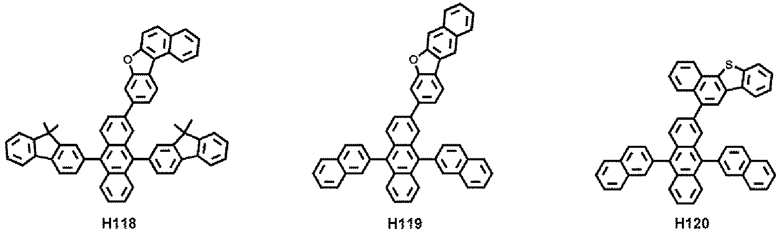

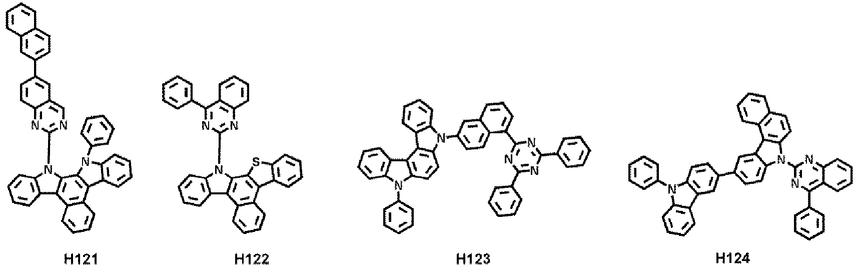

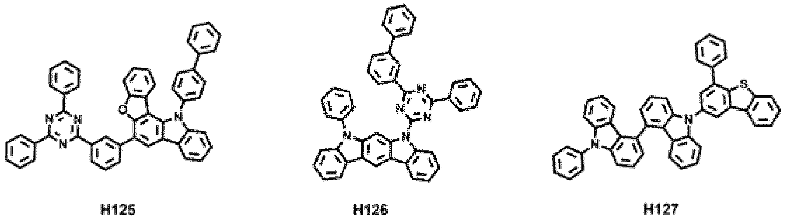

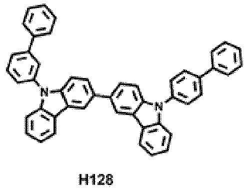

### [Phosphorescent dopant]

The phosphorescent dopant may include at least one transition metal as a central metal.

The phosphorescent dopant may include a monodentate ligand, a bidentate ligand, a tridentate ligand, a tetradentate ligand, a pentadentate ligand, a hexadentate ligand, or any combination thereof.

The phosphorescent dopant may be electrically neutral.

In an embodiment, the phosphorescent dopant may include an organometallic compound represented by Formula 401:

[Formula 401] M(L₄₀₁)_{xc1}(L₄₀₂)_{xc2}

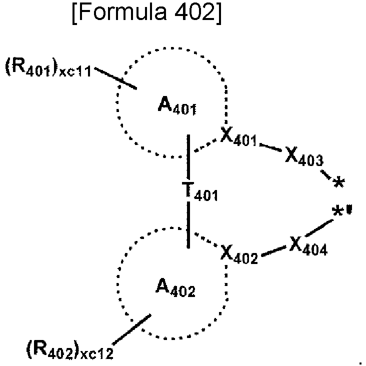

In Formulae 401 and 402,
M may be a transition metal (e.g., Ir, Pt, Pd, Os, Ti, Au, Hf, Eu, Tb, Rh, Re, or Tm),
L₄₀₁ may be a ligand represented by Formula 402, and xc1 is 1, 2, or 3, wherein when xc1 is 2 or more, two or more of L₄₀₁ may be identical to or different from each other,
L₄₀₂ may be an organic ligand, and xc2 may be 0, 1, 2, 3, or 4, wherein when xc2 is 2 or more, two or more of L₄₀₂ may be identical to or different from each other,
X₄₀₁ and X₄₀₂ may each independently be nitrogen or carbon,
ring A₄₀₁ and ring A₄₀₂ may each independently be a C₃-C₆₀ carbocyclic group or a C₁-C₆₀ heterocyclic group,
T₄₀₁ may be a single bond, *-O-*', *-S-*', *-C(=O)-*', *-N(Q₄₁₁)-*', *-C(Q₄₁₁)(Q₄₁₂)-*', *-C(Q₄₁₁)=C(Q₄₁₂)-*', *-C(Q₄₁₁)=*', or *=C=*',
X₄₀₃ and X₄₀₄ may each independently be a chemical bond (for example, a covalent bond or a coordinate bond), O, S, N(Q₄₁₃), B(Q₄₁₃), P(Q₄₁₃), C(Q₄₁₃)(Q₄₁₄), or Si(Q₄₁₃)(Q₄₁₄),
Q₄₁₁ to Q₄₁₄ may each independently be the same as described in connection with Q₁,
R₄₀₁ and R₄₀₂ may each independently be hydrogen, deuterium, -F, -Cl, -Br, -I, a hydroxyl group, a cyano group, a nitro group, a C₁-C₂₀ alkyl group unsubstituted or substituted with at least one R₁₀ₐ, a C₁-C₂₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), - S(=O)₂(Q₄₀₁), or -P(=O)(Q₄₀₁)(Q₄₀₂),
Q₄₀₁ to Q₄₀₃ may each independently be the same as described in connection with Q₁,
xc11 and xc12 may each independently be an integer from 0 to 10, and
   * and *' in Formula 402 each indicate a binding site to M in Formula 401.

In an embodiment, in Formula 402, X₄₀₁ may be nitrogen, and X₄₀₂ may be carbon, or X₄₀₁ and X₄₀₂ may each be nitrogen.

In an embodiment, in Formula 401, when xc1 is 2 or more, two ring A₄₀₁ among two or more of L₄₀₁ may be optionally linked together via T₄₀₂, which is a linking group, and two ring A₄₀₂ may be optionally linked together via T₄₀₃, which is a linking group (see Compounds PD1 to PD4 and PD7). T₄₀₂ and T₄₀₃ may each be the same as described in connection with T₄₀₁.

In Formula 401, L₄₀₂ may be an organic ligand. In an embodiment, L₄₀₂ may include a halogen group, a diketone group (for example, an acetylacetonate group), a carboxylic acid group (for example, a picolinate group), -C(=O), an isonitrile group, a - CN group, a phosphorus group (for example, a phosphine group, a phosphite group, etc.), or any combination thereof.

In an embodiment, the phosphorescent dopant may include, for example, one of Compounds PD1 to PD39, or any combination thereof:

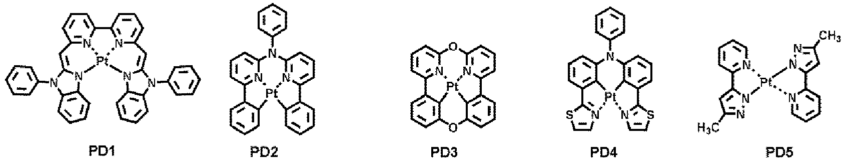

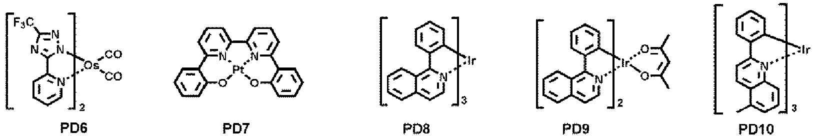

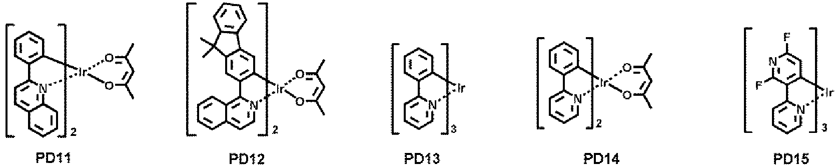

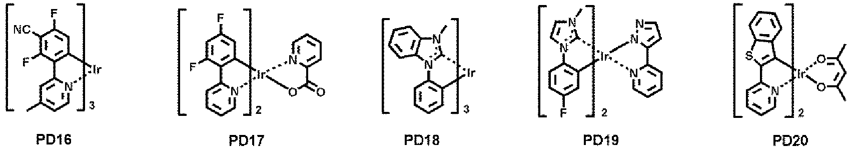

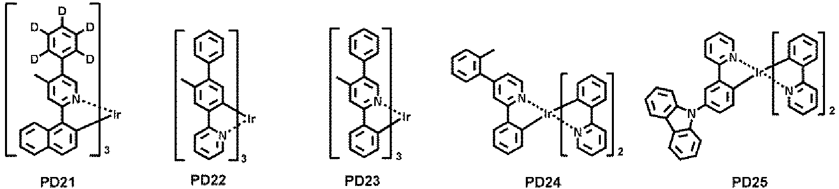

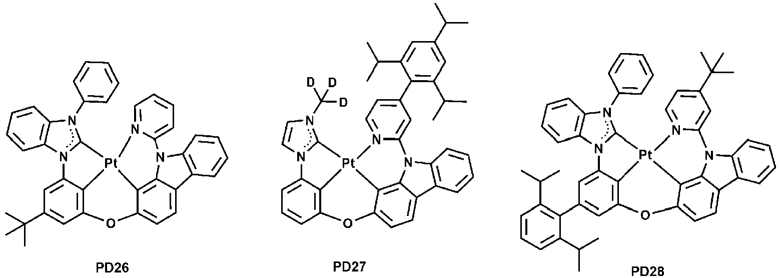

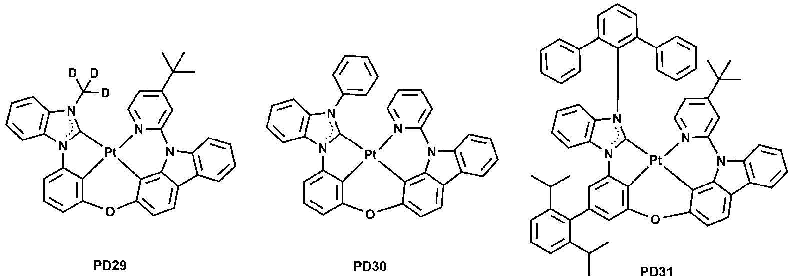

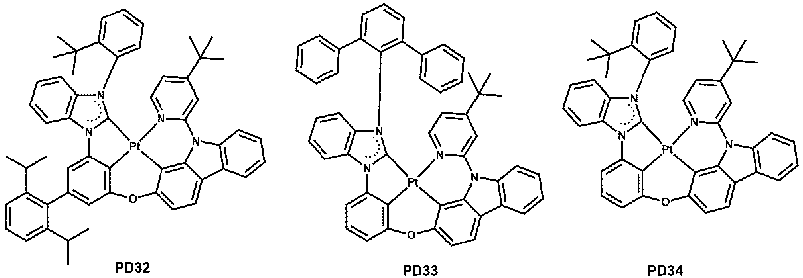

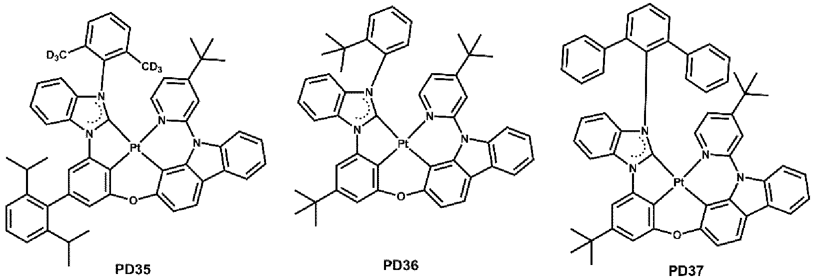

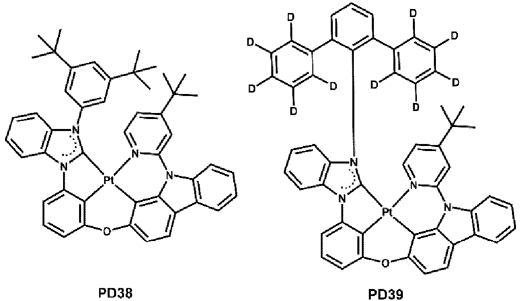

### [Fluorescent dopant]

The fluorescent dopant may include an amine group-containing compound, a styryl group-containing compound, or any combination thereof.

In an embodiment, the fluorescent dopant may include a compound represented by Formula 501:

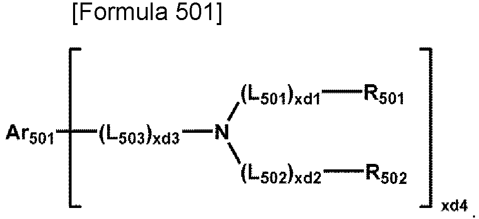

In Formula 501,
Ar₅₀₁, L₅₀₁ to L₅₀₃, R₅₀₁, and R₅₀₂ may each independently be a C₃-C₆₀ carbocyclic group unsubstituted or substituted with at least one R₁₀ₐ or a C₁-C₆₀ heterocyclic group unsubstituted or substituted with at least one R₁₀ₐ,
xd1 to xd3 may each independently be 0, 1, 2, or 3, and
xd4 may be 1, 2, 3, 4, 5, or 6.

In an embodiment, in Formula 501, Ar₅₀₁ may be a condensed cyclic group (for example, an anthracene group, a chrysene group, a pyrene group, etc.) in which three or more monocyclic groups are condensed together.

In an embodiment, in Formula 501, xd4 may be 2.

In an embodiment, the fluorescent dopant may include one of Compounds FD1 to FD37, DPVBi, DPAVBi, or any combination thereof:

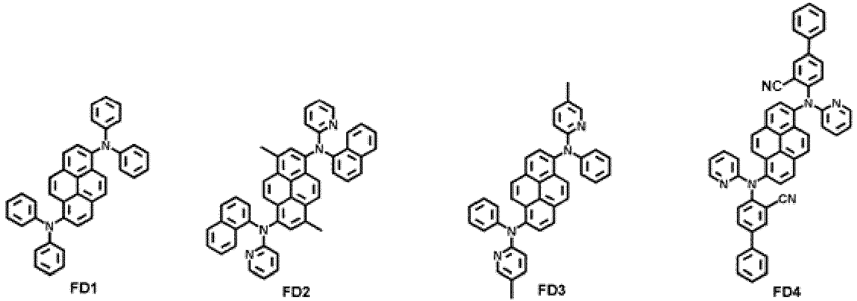

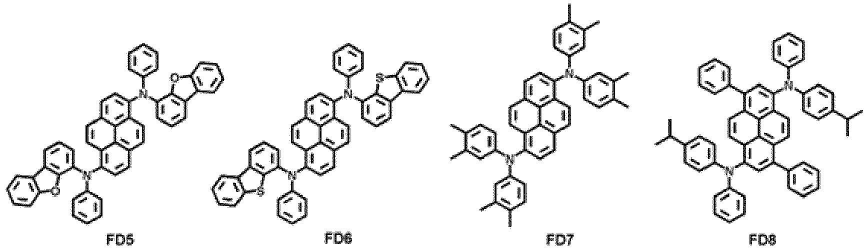

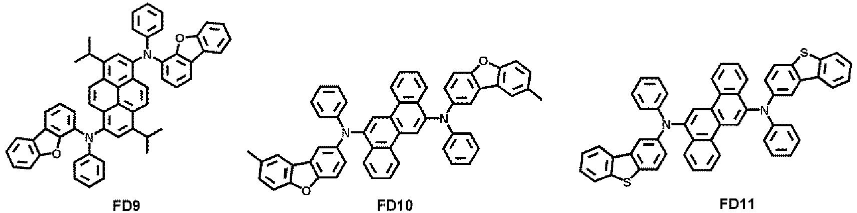

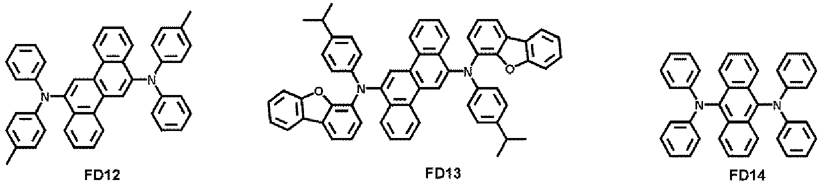

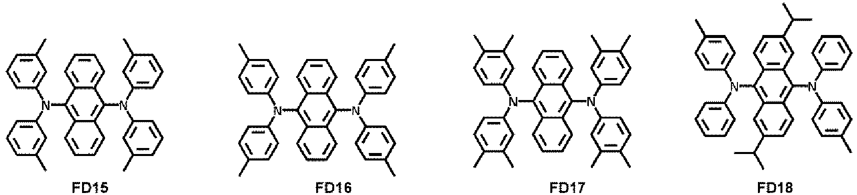

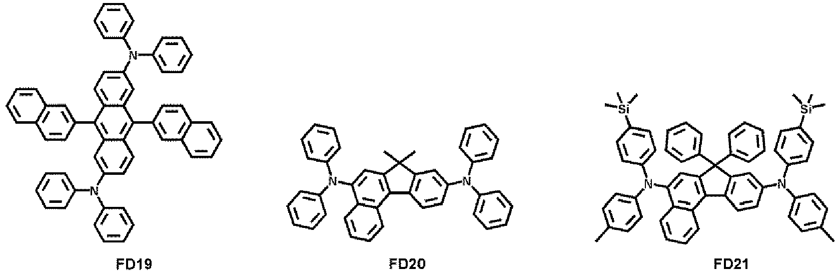

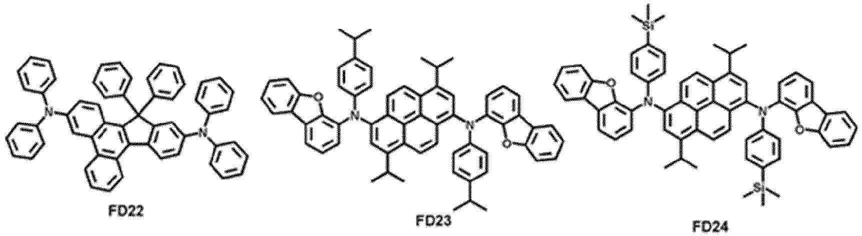

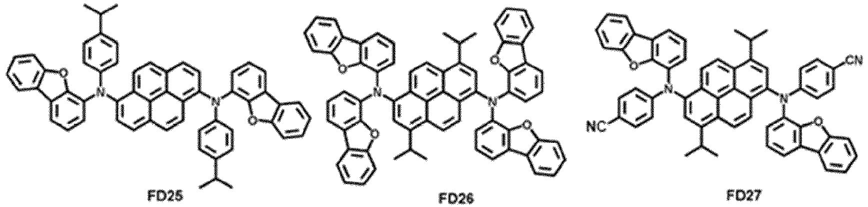

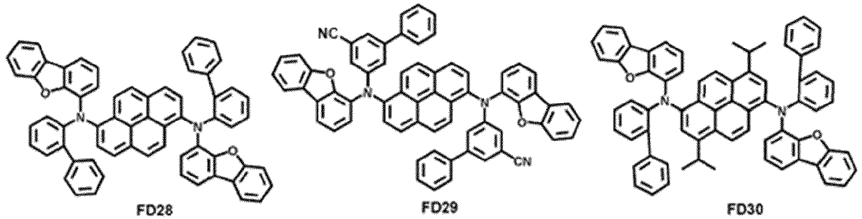

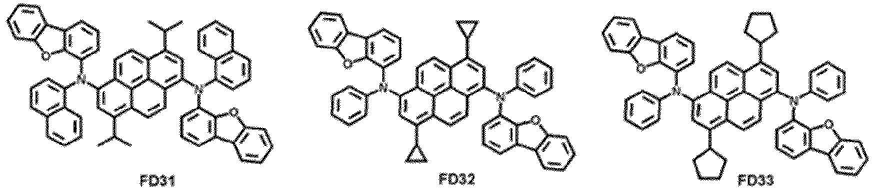

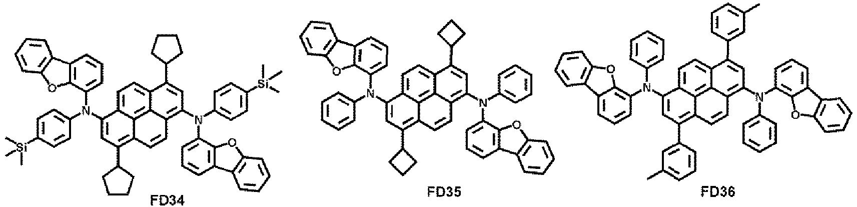

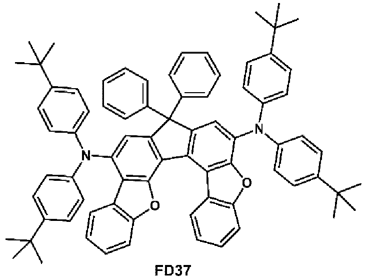

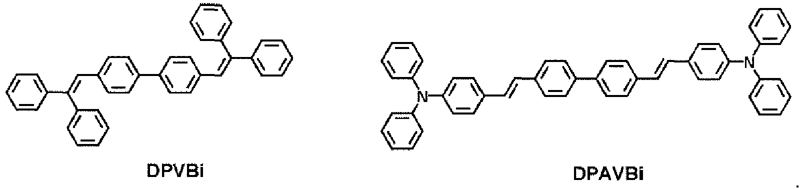

### [Delayed fluorescence material]

The emission layer 130 may include a delayed fluorescence material.

In embodiments, the delayed fluorescence material may be selected from compounds capable of emitting delayed fluorescence, based on a delayed fluorescence emission mechanism.

The delayed fluorescence material included in the emission layer 130 may serve as a host or as a dopant, depending on the types of other materials included in the emission layer 130.

In an embodiment, a difference between a triplet energy level (eV) of the delayed fluorescence material and a singlet energy level (eV) of the delayed fluorescence material may be in a range of about 0 eV to about 0.5 eV. When a difference between a triplet energy level (eV) of the delayed fluorescence material and a singlet energy level (eV) of the delayed fluorescence material is satisfied within the range described above, up-conversion from the triplet state to the singlet state of the delayed fluorescence materials may effectively occur, and thus, the light-emitting device 10 may have improved luminescence efficiency.

In an embodiment, the delayed fluorescence material may include: a material including at least one electron donor (for example, a π electron-rich C₃-C₆₀ cyclic group such as a carbazole group) and at least one electron acceptor (for example, a sulfoxide group, a cyano group, a π electron-deficient nitrogen-containing C₁-C₆₀ cyclic group, and the like); or a material including a C₈-C₆₀ polycyclic group including at least two cyclic groups that are condensed with each other while sharing boron (B).

In an embodiment, the delayed fluorescence material may include, for example, at least one of Compounds DF1 to DF14:

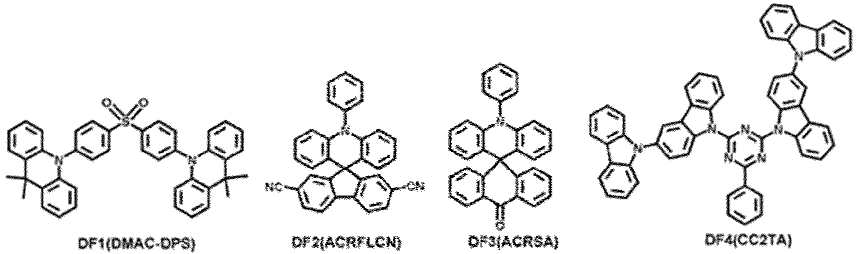

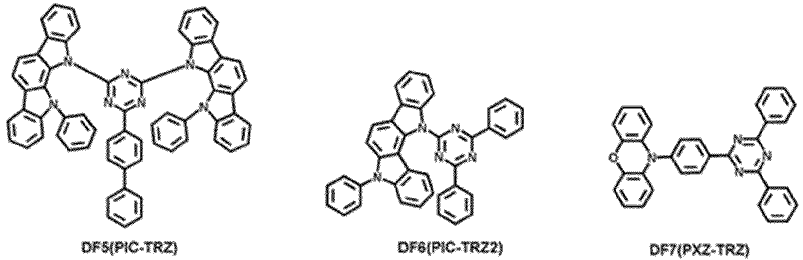

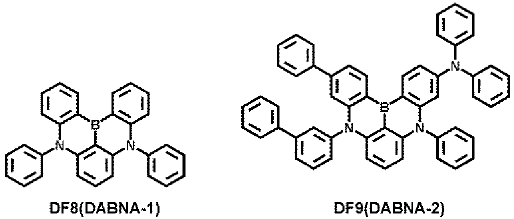

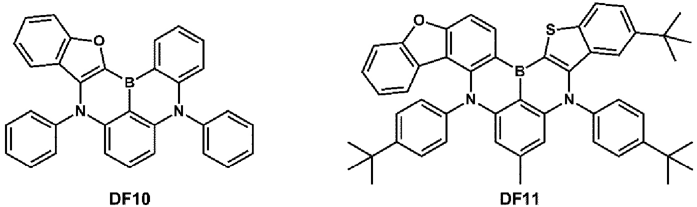

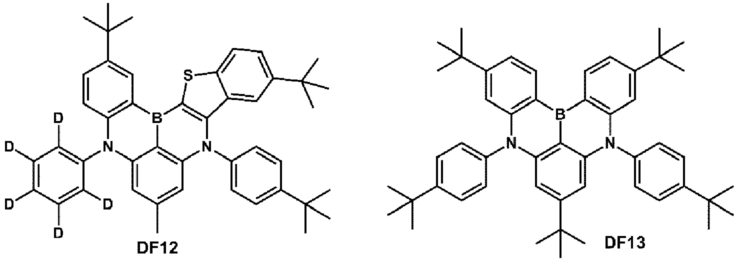

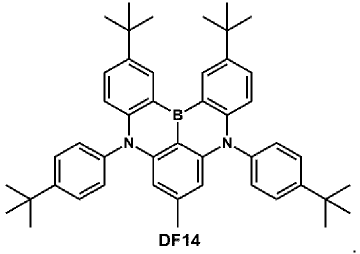

### [Quantum dots]

The emission layer 130 may include quantum dots.

In the specification, quantum dots may be crystals of a semiconductor compound. Quantum dots may emit light of various emission wavelengths depending on a size of crystals. Quantum dots may emit light of various emission wavelengths by adjusting a ratio of elements constituting the quantum dots.

A diameter of the quantum dot may be, for example, in a range of about 1 nm to about 10 nm.

The quantum dots may be synthesized by a wet chemical process, a metal organic chemical vapor deposition process, a molecular beam epitaxy process, or any process similar thereto.

The wet chemical process is a method that includes mixing a precursor material with an organic solvent and growing quantum dot particle crystals. When the crystals grow, the organic solvent naturally serves as a dispersant coordinated on the surface of the quantum dot crystals and controls the growth of the crystals so that the growth of quantum dot particles may be controlled through a process which costs less and may be more readily performed than vapor deposition methods, such as metal organic chemical vapor deposition (MOCVD) or molecular beam epitaxy (MBE).

The quantum dots may include a Group II-VI semiconductor compound, a Group III-V semiconductor compound, a Group III-VI semiconductor compound, a Group I-III-VI semiconductor compound, a Group IV-VI semiconductor compound, a Group IV element or compound, or any combination thereof.

Examples of a Group II-VI semiconductor compound may include: a binary compound, such as CdS, CdSe, CdTe, ZnS, ZnSe, ZnTe, ZnO, HgS, HgSe, HgTe, MgSe, MgS, and the like; a ternary compound, such as CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and the like; a quaternary compound, such as CdZnSeS, CdZnSeTe, CdZnSTe, CdHgSeS, CdHgSeTe, CdHgSTe, HgZnSeS, HgZnSeTe, HgZnSTe, and the like; and any combination thereof.

Examples of a Group III-V semiconductor compound may include: a binary compound, such as GaN, GaP, GaAs, GaSb, AIN, AlP, AlAs, AlSb, InN, InP, InAs, InSb, etc.; a ternary compound, such as GaNP, GaNAs, GaNSb, GaPAs, GaPSb, AINP, AlNAs, AlNSb, AlPAs, AlPSb, InGaP, InNP, InAlP, InNAs, InNSb, InPAs, InPSb, etc.; a quaternary compound, such as GaAlNP, GaAINAs, GaAINSb, GaAlPAs, GaAlPSb, GalnNP, GalnNAs, GalnNSb, GalnPAs, GalnPSb, InAlNP, InAlNAs, InAlNSb, InAlPAs, InAlPSb, etc.; and any combination thereof. In an embodiment, a Group III-V semiconductor compound may further include a Group II element. Examples of a Group IlI-V semiconductor compound further including a Group II element may include InZnP, InGaZnP, InAlZnP, etc.

Examples of a Group III-VI semiconductor compound may include: a binary compound, such as GaS, Ga₂Se₃, GaTe, InS, InSe, In₂S₃, In₂Se₃, InTe, etc.; a ternary compound, such as InGaS₃, InGaSe₃, etc.; and any combination thereof.

Examples of a Group I-III-VI semiconductor compound may include: a ternary compound, such as AgInS, AgInS₂, AgInSe₂, AgGaS, AgGaS₂, AgGaSe₂, CulnS, CuInS₂, CuInSe₂, CuGaS₂, CuGaSe₂, CuGaO₂, AgGaO₂, AgAlO₂, etc.; a quaternary compound, such as AgInGaS, AgInGaS₂, AgInGaSe, AgInGaSe₂, CulnGaS, CuInGaS₂, etc.; and any combination thereof.

Examples of a Group IV-VI semiconductor compound may include: a binary compound, such as SnS, SnSe, SnTe, PbS, PbSe, or PbTe; a ternary compound, such as SnSeS, SnSeTe, SnSTe, PbSeS, PbSeTe, PbSTe, SnPbS, SnPbSe, SnPbTe, etc.; a quaternary compound, such as SnPbSSe, SnPbSeTe, SnPbSTe, etc.; and any combination thereof.

Examples of a Group IV element or compound may include: a single element, such as Si, Ge, etc.; a binary compound, such as SiC, SiGe, etc.; and any combination thereof.

Each element included in a compound, such as a binary compound, a ternary compound, or a quaternary compound, may be present in a particle at a uniform concentration or at a non-uniform concentration. For example, the formulae above refer to types of elements included in the compound, and the element ratios within the compound may vary. For example, AgInGaS₂ may be AgInₓGa₁₋ₓS₂ (where x is a real number between 0 and 1).

In embodiments, the quantum dots may have a single structure in which the concentration of each element in the quantum dots is uniform, or the quantum dots may have a core-shell structure. In an embodiment, in the case that the quantum dots have a core-shell structure, materials included in the core and materials included in the shell may be different from each other.

The shell of the quantum dots may serve as a protective layer that prevents chemical degeneration of the core to maintain semiconductor characteristics, and/or may serve as a charging layer that imparts electrophoretic characteristics to the quantum dots. The shell may be a single-layer or a multi-layer. An interface between the core and the shell may have a concentration gradient in which the concentration of a material that is present in the shell decreases toward the core.

Examples of a shell of the quantum dots may include a metal oxide, a non-metal oxide, a semiconductor compound, and any combination thereof. Examples of a metal oxide or a non-metal oxide may include: a binary compound, such as SiO₂, Al₂O₃, TiO₂, ZnO, MnO, Mn₂O₃, Mn₃O₄, CuO, FeO, Fe₂O₃, Fe₃O₄, CoO, Co₃O₄, NiO, etc.; a ternary compound, such as MgAl₂O₄, CoFe₂O₄, NiFe₂O₄, CoMn₂O₄, etc.; and any combination thereof.

Examples of a semiconductor compound may include, as described herein, a Group III-VI semiconductor compound, a Group II-VI semiconductor compound, a Group III-V semiconductor compound, a Group III-VI semiconductor compound, a Group I-III-VI semiconductor compound, a Group IV-VI semiconductor compound, and any combination thereof. For example, the semiconductor compound may include CdS, CdSe, CdTe, ZnS, ZnSe, ZnTe, ZnSeS, ZnTeS, GaAs, GaP, GaS, GaSe, AgGaS, AgGaS₂, GaSb, HgS, HgSe, HgTe, InAs, InP, InGaP, InSb, AlAs, AlP, AlSb, and any combination thereof.

A full width at half maximum (FWHM) of an emission wavelength spectrum of the quantum dots may be less than or equal to about 45 nm. For example, the quantum dots may have an FWHM of an emission wavelength spectrum less than or equal to about 40 nm. For example, the quantum dots may have an FWHM of an emission wavelength spectrum less than or equal to about 30 nm. When the FWHM is within any of these ranges, the color purity or color reproducibility of the quantum dots may be improved. Light emitted through the quantum dots may be emitted in all directions, so that a wide viewing angle may be improved.

In an embodiment, the quantum dots may be nanoparticles, nanotubes, nanowires, nanofibers, nanoplates, and the like, or the quantum dots may be in the form of spherical particles, pyramidal particles, multi-arm particles, or cubic particles.

Since the energy band gap may be controlled by adjusting the size of the quantum dots or the ratio of elements in the quantum dot compound, light of various wavelengths may be obtained from the quantum dot-containing emission layer. Therefore, by using the aforementioned quantum dots (using quantum dots of different sizes or having different element ratios in the quantum dot compound), a light-emitting device emitting light of various wavelengths may be implemented. In an embodiment, the size of the quantum dots or the ratio of elements in the quantum dot compound may be selected to emit red light, green light, and/or blue light. In an embodiment, the size of the quantum dots may be configured to emit white light by combining light of various colors.

### [Electron transport region 140]

The electron transport region 140 may have a structure consisting of a layer consisting of a single material, a structure consisting of a layer including different materials, or a structure including multiple layers including different materials.

The electron transport region 140 may include a buffer layer, a hole blocking layer, an electron control layer, an electron transport layer, an electron injection layer, or any combination thereof.

In an embodiment, the electron transport region 140 may have an electron transport layer/electron injection layer structure, a hole blocking layer/electron transport layer/electron injection layer structure, an electron control layer/electron transport layer/electron injection layer structure, or a buffer layer/electron transport layer/electron injection layer structure, wherein the layers in each structure may be stacked from the emission layer 130 in its respective stated order, but the structure of the electron transport region 140 is not limited thereto.

In an embodiment, the electron transport region 140 (e.g., the buffer layer, the hole blocking layer, the electron control layer, or the electron transport layer in the electron transport region 140) may include a metal-free compound including at least one π electron-deficient nitrogen-containing C₁-C₆₀ cyclic group.

In an embodiment, the electron transport region 140 may include a compound represented by Formula 601:

[Formula 601] [Ar₆₀₁]ₓₑ₁₁-[(L₆₀₁)ₓₑ₁-R₆₀₁]ₓₑ₂₁

In Formula 601,
Ar₆₀₁ and L₆₀₁ may each independently be a C₃-C₆₀ carbocyclic group that is unsubstituted or substituted with at least one R₁₀ₐ or a C₁-C₆₀ heterocyclic group that is unsubstituted or substituted with at least one R₁₀ₐ,
xe11 may be 1, 2, or 3,
xe1 may be 0, 1, 2, 3, 4, or 5,
R₆₀₁ may be a C₃-C₆₀ carbocyclic group that is unsubstituted or substituted with at least one R₁₀ₐ, a C₁-C₆₀ heterocyclic group that is unsubstituted or substituted with at least one R₁₀ₐ, -Si(Q₆₀₁)(Q₆₀₂)(Q₆₀₃), -C(=O)(Q₆₀₁), -S(=O)₂(Q₆₀₁), or - P(=O)(Q₆₀₁)(Q₆₀₂),
Q₆₀₁ to Q₆₀₃ may each independently be the same as described in connection with Q₁,
xe21 may be 1, 2, 3, 4, or 5, and
at least one of Ar₆₀₁, L₆₀₁, and R₆₀₁ may each independently be a π electron-deficient nitrogen-containing C₁-C₆₀ cyclic group unsubstituted or substituted with at least one R₁₀ₐ.

In an embodiment, in Formula 601, when xe11 is 2 or more, two or more of Ar₆₀₁ may be linked together via a single bond.

In an embodiment, in Formula 601, Ar₆₀₁ in Formula 601 may be an anthracene group that is unsubstituted or substituted with at least one R₁₀ₐ.

In embodiments, the electron transport region 140 may include a compound represented by Formula 601-1:

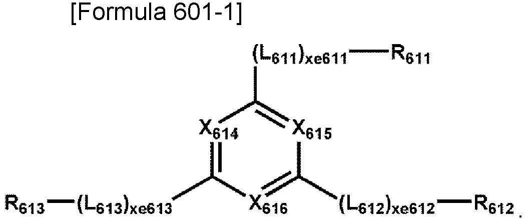

In Formula 601-1,
X₆₁₄ may be N or C(R₆₁₄), X₆₁₅ may be N or C(R₆₁₅), X₆₁₆ may be N or C(R₆₁₆), and at least one of X₆₁₄ to X₆₁₆ may be N,
L₆₁₁ to L₆₁₃ may each independently be the same as described in connection with L₆₀₁,
xe611 to xe613 may each independently be the same as described in connection with xe1,
R₆₁₁ to R₆₁₃ may each independently be the same as described in connection with R₆₀₁, and
R₆₁₄ to R₆₁₆ may each independently be hydrogen, deuterium, -F, -Cl, -Br, -I, a hydroxyl group, a cyano group, a nitro group, a C₁-C₂₀ alkyl group, a C₁-C₂₀ alkoxy group, a C₃-C₆₀ carbocyclic group that is unsubstituted or substituted with at least one R₁₀ₐ, or a C₁-C₆₀ heterocyclic group that is unsubstituted or substituted with at least one R₁₀ₐ.

In an embodiment, in Formula 601 and 601-1, xe1 and xe611 to xe613 may each independently be 0, 1, or 2.

The electron transport region 140 may include one of Compounds ET1 to ET45, 2,9-dimethyl-4,7-diphenyl-1,10-phenanthroline (BCP), 4,7-diphenyl-1,10-phenanthroline (Bphen), Alq₃, BAlq, TAZ, NTAZ, or any combination thereof:

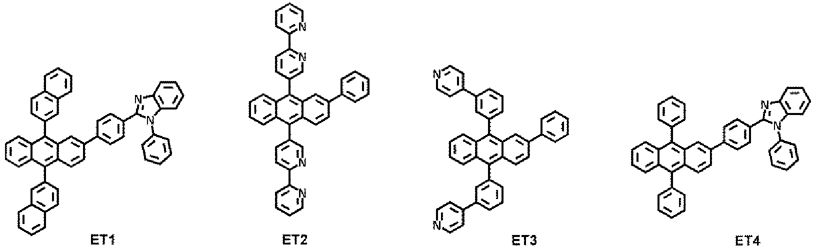

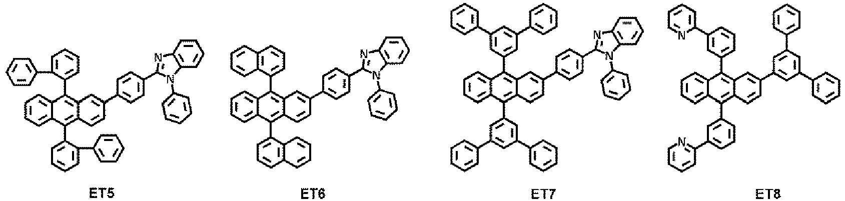

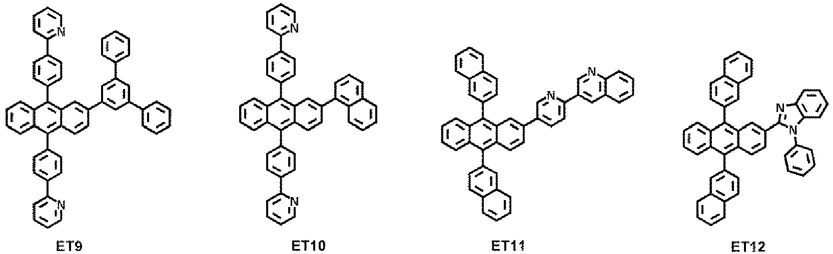

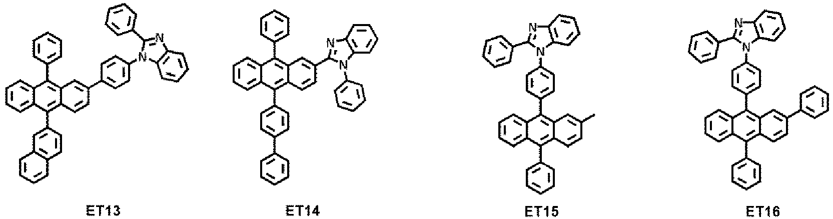

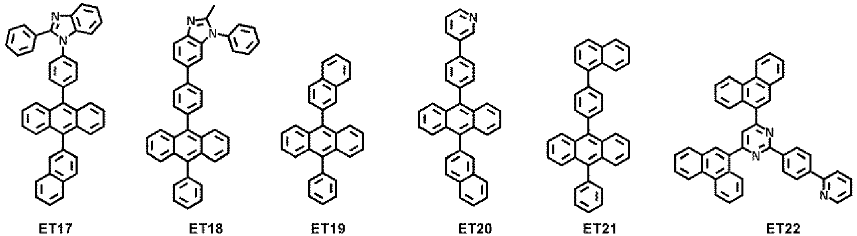

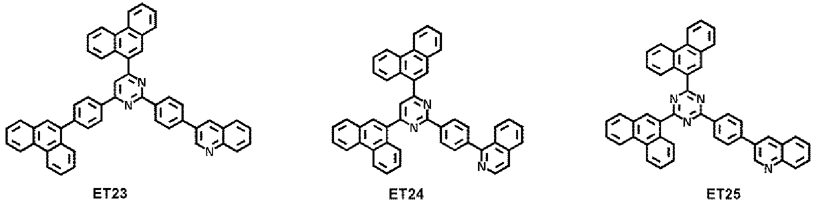

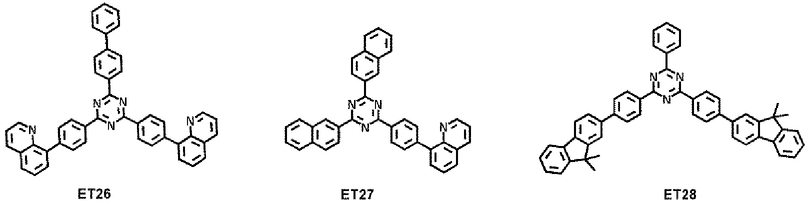

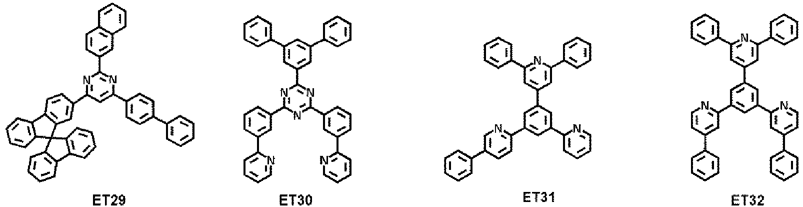

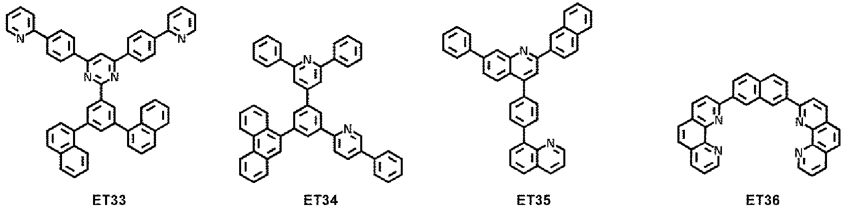

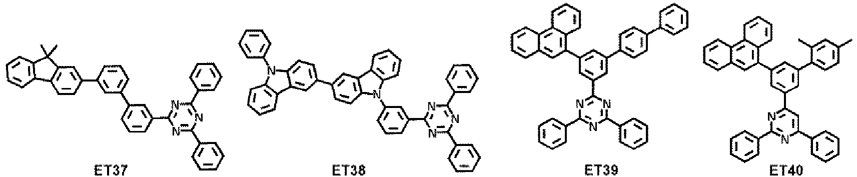

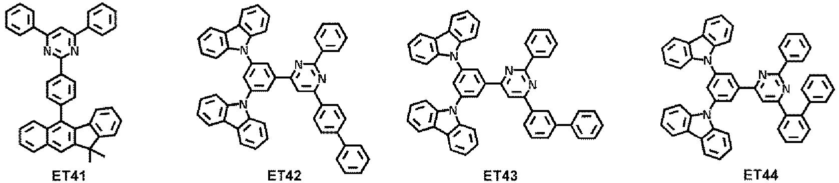

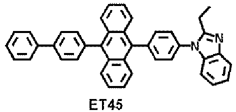

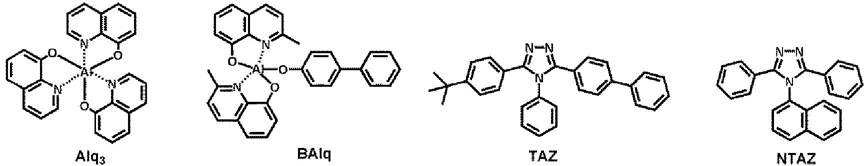

A thickness of the electron transport region 140 may be in a range of about 100 Å to about 5,000 Å. For example, the thickness of the electron transport region 140 may be in a range of about 160 Å to about 4,000 Å. When the electron transport region 140 includes a buffer layer, a hole blocking layer, an electron control layer, an electron transport layer, or any combination thereof, a thickness of the buffer layer, the hole blocking layer, or the electron control layer may each independently be in a range of about 20 Å to about 1,000 Å, and a thickness of the electron transport layer may be in a range of about 100 Å to about 1,000 Å. For example, the thickness of the buffer layer, the hole blocking layer, or the electron control layer may each independently be in a range of about 30 Å to about 300 Å. For example, the thickness of the electron transport layer may be in a range of about 150 Å to about 500 Å. When the thicknesses of the buffer layer, the hole blocking layer, the electron control layer, the electron transport layer, and/or the electron transport region 140 are within these ranges, satisfactory electron transporting characteristics may be obtained without a substantial increase in driving voltage.

The electron transport region 140 (e.g., an electron transport layer in the electron transport region 140) may further include, in addition to the aforementioned materials, a metal-containing material.

The metal-containing material may include an alkali metal complex, an alkaline earth metal complex, or any combination thereof. A metal ion of the alkali metal complex may be a Li ion, a Na ion, a K ion, a Rb ion, or a Cs ion, and a metal ion of the alkaline earth metal complex may be a Be ion, a Mg ion, a Ca ion, a Sr ion, or a Ba ion. A ligand coordinated with the metal ion of an alkali metal complex or an alkaline earth-metal complex may each independently include a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxyphenyloxadiazole, a hydroxyphenylthiadiazole, a hydroxyphenylpyridine, a hydroxyphenylbenzimidazole, a hydroxyphenylbenzothiazole, a bipyridine, a phenanthroline, a cyclopentadiene, or any combination thereof.

In an embodiment, the metal-containing material may include a Li complex. The Li complex may include, for example, Compound ET-D1 (LiQ) or Compound ET-D2:

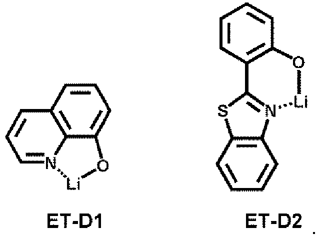

The electron transport region 140 may include an electron injection layer that facilitates the injection of electrons from the second electrode 150. The electron injection layer may directly contact the second electrode 150.

The electron injection layer may have a structure consisting of a layer consisting of a single material, a structure consisting of a layer including different materials, or a structure including multiple layers including different materials.

The electron injection layer may include an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof.

The alkali metal may include Li, Na, K, Rb, Cs, or any combination thereof. The alkaline earth metal may include Mg, Ca, Sr, Ba, or any combination thereof. The rare earth metal may include Sc, Y, Ce, Tb, Yb, Gd, or any combination thereof.

The alkali metal-containing compound, the alkaline earth metal-containing compound, and the rare earth metal-containing compound may be oxides, halides (e.g., fluorides, chlorides, bromides, or iodides), or tellurides of the alkali metal, the alkaline earth metal, and the rare earth metal, or any combination thereof.

The alkali metal-containing compound may include: an alkali metal oxide, such as Li₂O, Cs₂O, K₂O, etc.; an alkali metal halide, such as LiF, NaF, CsF, KF, Lil, Nal, Csl, Kl, etc.; or any combination thereof. The alkaline earth metal-containing compound may include an alkaline earth metal oxide, such as BaO, SrO, CaO, BaₓSr₁₋ₓO (wherein x is a real number satisfying 0<x<1), BaₓCa₁₋ₓO (wherein x is a real number satisfying 0<x<1), and the like. The rare earth metal-containing compound may include YbF₃, ScF₃, Sc₂O₃, Y₂O₃, Ce₂O₃, GdF₃, TbF₃, YbI₃, ScI₃, TbI₃, or any combination thereof. In an embodiment, the rare earth metal-containing compound may include a lanthanide metal telluride. Examples of a lanthanide metal telluride may include LaTe, CeTe, PrTe, NdTe, PmTe, SmTe, EuTe, GdTe, TbTe, DyTe, HoTe, ErTe, TmTe, YbTe, LuTe, La₂Te₃, Ce₂Te₃, Pr₂Te₃, Nd₂Te₃, Pm₂Te₃, Sm₂Te₃, Eu₂Te₃, Gd₂Te₃, Tb₂Te₃, Dy₂Te₃, Ho₂Te₃, Er₂Te₃, Tm₂Te₃, Yb₂Te₃, Lu₂Te₃, and the like.

The alkali metal complex, the alkaline earth-metal complex, and the rare earth metal complex may include: an alkali metal ion, an alkaline earth metal ion, and a rare earth metal ion; and a ligand bonded to the metal ion (for example, a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxyphenyloxadiazole, a hydroxyphenylthiadiazole, a hydroxyphenylpyridine, a hydroxyphenyl benzimidazole, a hydroxyphenylbenzothiazole, a bipyridine, a phenanthroline, a cyclopentadiene, or any combination thereof).

In an embodiment, the electron injection layer may consist of an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof, as described above. In embodiments, the electron injection layer may further include an organic material (e.g., the compound represented by Formula 601).

In embodiments, the electron injection layer may consist of an alkali metal-containing compound (e.g., an alkali metal halide); or the electron injection layer may consist of an alkali metal-containing compound (e.g., an alkali metal halide), and an alkali metal, an alkaline earth metal, a rare earth metal, or any combination thereof. In an embodiment, the electron injection layer may be a KI:Yb co-deposited layer, an Rbl:Yb co-deposited layer, a LiF:Yb co-deposited layer, or the like.

When the electron injection layer further includes an organic material, an alkali metal, an alkaline earth metal, a rare earth metal, an alkali metal-containing compound, an alkaline earth metal-containing compound, a rare earth metal-containing compound, an alkali metal complex, an alkaline earth-metal complex, a rare earth metal complex, or any combination thereof may be uniformly or non-uniformly dispersed in a matrix including the organic material.

A thickness of the electron injection layer may be in a range of about 1 Å to about 100 Å. For example, the thickness of the electron injection layer may be in a range of about 3 Å to about 90 Å. When the thickness of the electron injection layer is within any of these ranges, satisfactory electron injection characteristics may be obtained without a substantial increase in driving voltage.

### [Second electrode 150]

The second electrode 150 may be arranged on the electron transport region 140. The second electrode 150 may be a cathode, which is an electron injection electrode. When the second electrode 150 is a cathode, a material for forming the second electrode may include a material having a low-work function, such as a metal, an alloy, an electrically conductive compound, or any combination thereof.

The second electrode 150 may include Li, Ag, Mg, Al, Al-Li, Ca, Mg-In, Mg-Ag, Yb, Ag-Yb, ITO, IZO, or any combination thereof. The second electrode 150 may be a transmissive electrode, a semi-transmissive electrode, or a reflective electrode.

The second electrode 150 may have a single-layered structure or a multilayered structure.

### [Capping layer]

The light-emitting device 10 may further include a capping layer outside the first electrode 110 and/or outside the second electrode 150.

In an embodiment, the capping layer may include the organic compound.

In an embodiment, the light-emitting device 10 may further include a first capping layer outside the first electrode 110. The first capping layer may include the organic compound.

In embodiments, the light-emitting device 10 may further include a second capping layer outside the second electrode 150. The second capping layer may include the organic compound.

In embodiments, the light-emitting device 10 may further include both a first capping layer outside the first electrode 110 and a second capping layer outside the second electrode 150. At least one of the first capping layer and the second capping layer may include the organic compound.

Light generated in the emission layer 130 of the light-emitting device 10 may pass through the first electrode 110, which may be a semi-transmissive electrode or a transmissive electrode, and through the first capping layer to the outside. Light generated in the emission layer 130 of the light-emitting device 10 may pass through the second electrode 150, which may be a semi-transmissive electrode or a transmissive electrode, and through the second capping layer to the outside.

The first capping layer and the second capping layer may each increase external emission efficiency according to the principle of constructive interference. Accordingly, the light extraction efficiency of the light-emitting device 10 may be increased, and accordingly, the luminescence efficiency of the light-emitting device 10 may be improved.

The first capping layer and the second capping layer may each include a material having a refractive index greater than or equal to about 1.2 (with respect to a wavelength of about 460 nm).

The first capping layer and the second capping layer may each independently be an organic capping layer including an organic material, an inorganic capping layer including an inorganic material, or an organic-inorganic composite capping layer including an organic material and an inorganic material.

At least one of the first capping layer and the second capping layer may each independently include a carbocyclic compound, a heterocyclic compound, an amine group-containing compound, a porphine derivative, a phthalocyanine derivative, a naphthalocyanine derivative, an alkali metal complex, an alkaline earth metal complex, or any combination thereof. The carbocyclic compound, the heterocyclic compound, and the amine group-containing compound may each optionally be substituted with a substituent including O, N, S, Se, Si, F, CI, Br, I, or any combination thereof. In an embodiment, at least one of the first capping layer and the second capping layer may each independently include an amine group-containing compound.

In an embodiment, at least one of the first capping layer and the second capping layer may each independently include a compound represented by Formula 201, a compound represented by Formula 202, or any combination thereof.

In an embodiment, at least one of the first capping layer and the second capping layer may each independently include one of Compounds HT28 to HT33, one of Compounds CP1 to CP6, β-NPB, or any combination thereof:

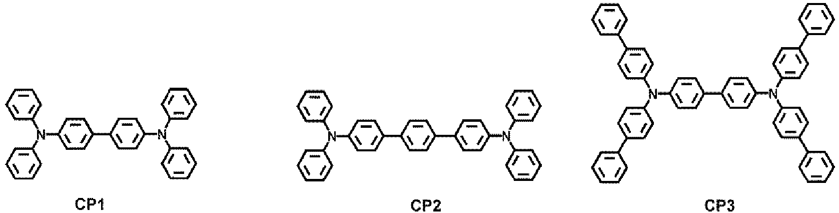

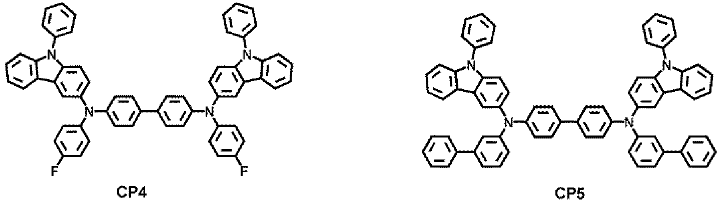

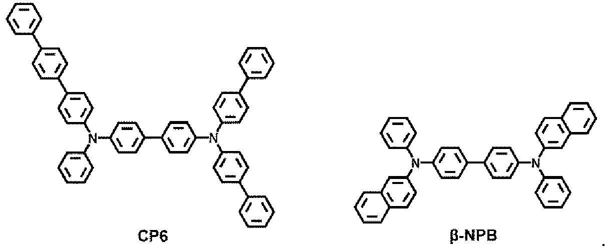

### [Film]

The electronic apparatus may further include a film. The film may be, for example, an optical member (or a light control means) (e.g., a color filter, a color conversion layer, a capping layer, a light extraction efficiency enhancement layer, a selective light absorbing layer, a polarizing layer, a quantum dot-containing layer, or like), a light blocking member (e.g., a light reflective layer, a light absorbing layer, or the like), a protective member (e.g., an insulating layer, a dielectric layer, or the like).

### [Electronic apparatus]

The light-emitting device 10 may be included in various electronic apparatuses. For example, an electronic apparatus including the light-emitting device 10 may be a display apparatus, an authentication apparatus, and the like.

The electronic apparatus (e.g., a display apparatus) may further include, in addition to the light-emitting device 10, a color filter, a color conversion layer, or a color filter and a color conversion layer. The color filter and/or the color-conversion layer may be disposed in at least one direction in which light emitted from the light-emitting device 10 travels. For example, light emitted from the light-emitting device 10 may be blue light or white light. The light-emitting device 10 may be the same as described herein.

The electronic apparatus may include a substrate. The substrate may include subpixels, the color filter may include color filter areas respectively corresponding to the subpixels, and the color conversion layer may include color conversion areas respectively corresponding to the subpixels.

A pixel-defining film may be arranged between the subpixels to define each subpixel.

The color filter may further include color filter areas and light-shielding patterns arranged between the color filter areas, and the color conversion layer may further include color conversion areas and light-shielding patterns arranged between the color conversion areas.

The color filter areas (or the color conversion areas) may include a first area emitting first color light, a second area emitting second color light, and/or a third area emitting third color light, wherein the first color light, the second color light, and/or the third color light may have different maximum emission wavelengths from one another. In an embodiment, the first color light may be red light, the second color light may be green light, and the third color light may be blue light. In an embodiment, the color filter areas (or the color conversion areas) may include quantum dots. In an embodiment, the first area may include red quantum dots, the second area may include green quantum dots, and the third area may not include quantum dots. The quantum dots may be the same as described herein. The first area, the second area, and/or the third area may each further include a scatterer.

In an embodiment, in the light-emitting device 10 emitting first light, the first area may absorb the first light to emit first-first color light, the second area may absorb the first light to emit second-first color light, and the third area may absorb the first light to emit third-first color light. For example, the first-first color light, the second-first color light, and the third-first color light may have different maximum emission wavelengths. For example, the first light may be blue light, the first-first color light may be red light, the second-first color light may be green light, and the third-first color light may be blue light.

The electronic apparatus may further include a thin-film transistor, in addition to the light-emitting device 10. The thin-film transistor may include a source electrode, a drain electrode, and an active layer, wherein any one of the source electrode and the drain electrode may be electrically connected to any one of the first electrode 110 and the second electrode 150 of the light-emitting device 10.

The thin-film transistor may further include a gate electrode, a gate insulating film, or the like.

The active layer may include crystalline silicon, amorphous silicon, an organic semiconductor, an oxide semiconductor, and the like.

The electronic apparatus may further include an encapsulation unit for sealing the light-emitting device 10. The sealing portion may be arranged between the color filter and/or the color conversion layer and the light-emitting device 10. The sealing portion may allow light from the light-emitting device 10 to be extracted to the outside, and may prevent ambient air and moisture from penetrating into the light-emitting device 10. The sealing portion may be a sealing substrate including a transparent glass substrate or a plastic substrate. The sealing portion may be a thin-film encapsulation layer including at least one layer of an organic layer and/or an inorganic layer. When the sealing portion is a thin film encapsulation layer, the electronic apparatus may be flexible.

Various functional layers may be further included on the sealing portion, in addition to the color filter and/or the color conversion layer, according to the use of the electronic apparatus. Examples of the functional layers may include a touch screen layer and a polarizing layer. The touch screen layer may be a pressure-sensitive touch screen layer, a capacitive touch screen layer, or an infrared touch screen layer. The authentication apparatus may be, for example, a biometric authentication apparatus that authenticates an individual by using biometric information of a living body (for example, fingertips, pupils, etc.).

The authentication apparatus may further include, in addition to the light-emitting device as described above, a biometric information collector.

The electronic apparatus may be applied to various displays, light sources, lighting, personal computers (for example, a mobile personal computer), mobile phones, digital cameras, electronic organizers, electronic dictionaries, electronic game machines, medical instruments (for example, electronic thermometers, sphygmomanometers, blood glucose meters, pulse measurement devices, pulse wave measurement devices, electrocardiogram displays, ultrasonic diagnostic devices, or endoscope displays), fish finders, various measuring instruments, meters (for example, meters for a vehicle, an aircraft, and a vessel), projectors, and the like.

### [Electronic equipment]

The light-emitting device 10 may be included in various types of electronic equipment. For example, the electronic apparatus including the light-emitting device 10 may be included in various types of electronic equipment.

For example, the electronic equipment including the light-emitting device 10 may be a flat panel display, a curved display, a computer monitor, a medical monitor, a television, a billboard, an indoor light, an outdoor light, a signal light, a head-up display, a fully transparent display, a partially transparent display, a flexible display, a rollable display, a foldable display, a stretchable display, a laser printer, a telephone, a mobile phone, a tablet computer, a phablet, a personal digital assistant (PDA), a wearable device, a laptop computer, a digital camera, a camcorder, a viewfinder, a micro display, a three-dimensional (3D) display, a virtual reality display, an augmented reality display, a vehicle, a video wall with multiple displays tiled together, a theater screen, a stadium screen, a phototherapy device, or a signboard.

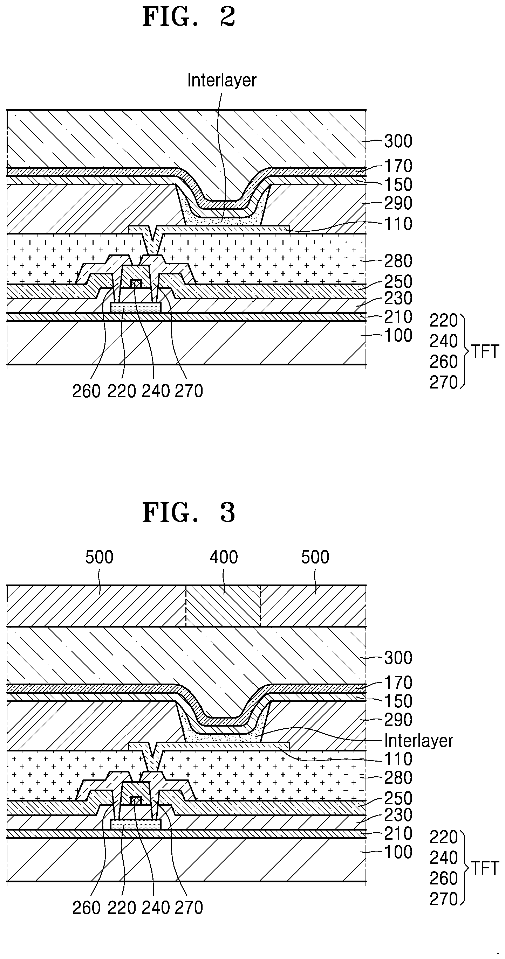

### [Description of FIGS. 2 and 3]

FIG. 2 is a schematic cross-sectional view of an electronic apparatus according to an embodiment.

The electronic apparatus of FIG. 2 may include a substrate 100, a thin-film transistor (TFT), a light-emitting device, and an encapsulation unit 300.

The substrate 100 may be a flexible substrate, a glass substrate, or a metal substrate. A buffer layer 210 may be arranged on the substrate 100. The buffer layer 210 may prevent penetration of impurities through the substrate 100 and may provide a flat surface on the substrate 100.

The TFT may be arranged on the buffer layer 210. The TFT may include an active layer 220, a gate electrode 240, a source electrode 260, and a drain electrode 270.

The active layer 220 may include an inorganic semiconductor, such as silicon or polysilicon, an organic semiconductor, or an oxide semiconductor, and may include a source region, a drain region, and a channel region.

A gate insulating film 230 for insulating the active layer 220 from the gate electrode 240 may be disposed on the active layer 220, and the gate electrode 240 may be disposed on the gate insulating film 230.

An interlayer insulating film 250 may be arranged on the gate electrode 240. The interlayer insulating film 250 may be located between the gate electrode 240 and the source electrode 260 to insulate the gate electrode 240 from the source electrode 260 and between the gate electrode 240 and the drain electrode 270 to insulate the gate electrode 240 from the drain electrode 270.

The source electrode 260 and the drain electrode 270 may be arranged on the interlayer insulating film 250. The interlayer insulating film 250 and the gate insulating film 230 may be formed to expose a source region and a drain region of the active layer 220, and the source electrode 260 and the drain electrode 270 may respectively contact the exposed portions of the source region and the drain region of the activation layer 220.

The TFT may be electrically connected to a light-emitting device to drive the light-emitting device, and may be covered and protected by a passivation layer 280. The passivation layer 280 may include an inorganic insulating film, an organic insulating film, or any combination thereof. The light-emitting device may be provided on the passivation layer 280. The light-emitting device may include the first electrode 110, the interlayer, and the second electrode 150.

The first electrode 110 may be arranged on the passivation layer 280. The passivation layer 280 may not completely cover the drain electrode and may expose a portion of the drain electrode 270. The first electrode 110 may be connected (for example, electrically connected) to the exposed portion of the drain electrode 270.

The pixel-defining film 290 including an insulating material may be arranged on the first electrode 110. The pixel defining layer 290 may expose a region of the first electrode 110, and the interlayer may be formed in the exposed region of the first electrode 110. The pixel-defining film 290 may be a polyimide-based organic film or a polyacrylic organic film. Although not shown in FIG. 2, at least some layers of the interlayer may extend to the upper portion of the pixel defining layer 290 and may be provided in the form of a common layer.

The second electrode 150 may be arranged on the interlayer, and a capping layer 170 may be further included on the second electrode 150. The capping layer 170 may be formed to cover the second electrode 150.

The encapsulation portion 300 may be arranged on the capping layer 170. The encapsulation portion 300 may be disposed on a light-emitting device to protect the light-emitting device from moisture and/or oxygen. The encapsulation portion 300 may include: an inorganic film including silicon nitride (SiNₓ), silicon oxide (SiOₓ), indium tin oxide, indium zinc oxide, or any combination thereof; an organic film including polyethylene terephthalate, polyethylene naphthalate, polycarbonate, polyimide, polyethylene sulfonate, polyoxymethylene, polyarylate, hexamethyldisiloxane, an acrylic resin (for example, polymethyl methacrylate, polyacrylic acid, or the like), an epoxy-based resin (for example, aliphatic glycidyl ether (AGE), or the like), or any combination thereof; or any combination of the inorganic film and the organic film.

FIG. 3 is a schematic cross-sectional view of an electronic apparatus according to another embodiment.

The electronic apparatus of FIG. 3 may differ from the electronic apparatus of FIG. 2, at least in that a light-shielding pattern 500 and a functional region 400 are further included on the encapsulation unit 300. The functional region 400 may include a color filter area, a color conversion area, or a combination of a color filter area and a color conversion area. In an embodiment, the light-emitting device included in the electronic apparatus of FIG. 3 may be a tandem light-emitting device.

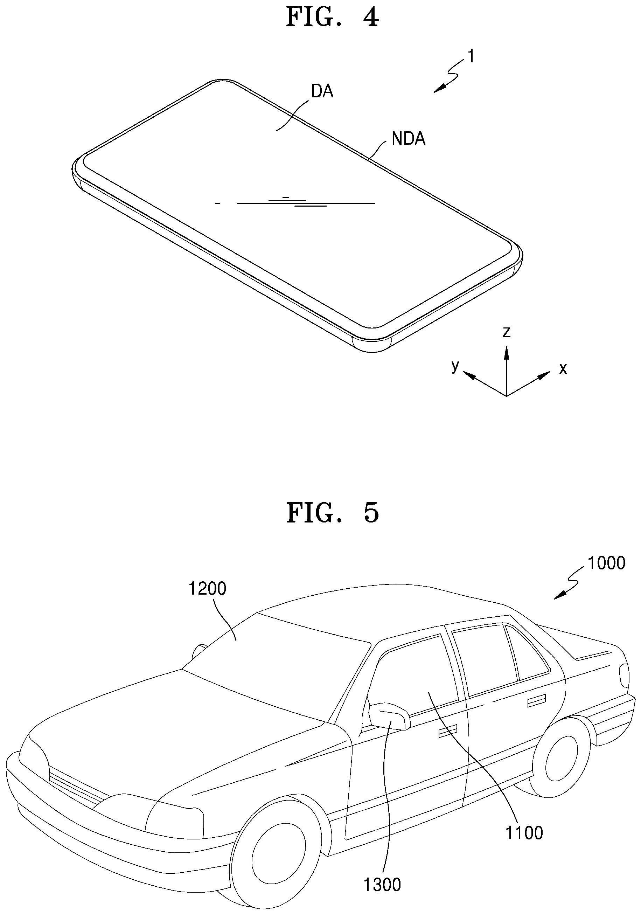

### [Description of FIG. 4]

FIG. 4 is a schematic perspective view of an electronic equipment 1 including the light-emitting device according to an embodiment.

The electronic equipment 1, which may be a device that displays a moving image or still image, may not only be a portable electronic device, such as a mobile phone, a smart phone, a tablet computer, a mobile communication terminal, an electronic notebook, an electronic book, a portable multimedia player (PMP), a navigation device, or an ultra-mobile PC (UMPC), but may also be various products, such as a television, a laptop computer, a monitor, a billboard, or an Internet of things (IoT) device. The electronic equipment 1 may be any such product as described above or a part thereof.

In an embodiment, the electronic equipment 1 may be a wearable device, such as a smart watch, a watch phone, a glasses-type display, or a head mounted display (HMD), or a part of the wearable device. However, embodiments are not limited thereto.

In an embodiment, examples of the electronic equipment 1 may include a dashboard of a vehicle, a center information display (CID) arranged on a center fascia or dashboard of a vehicle, a room mirror display instead of a side-view mirror of a vehicle, an entertainment for the back seat of a vehicle, or a display arranged on the back of the front seat of a vehicle, a head up display (HUD) installed on the front of a vehicle or projected on a front window glass, or a computer generated hologram augmented reality head up display (CGH AR HUD). FIG. 4 illustrates an embodiment in which the electronic equipment 1 is a smartphone for convenience of explanation.

The electronic equipment 1 may include a display area DA and a non-display area NDA outside the display area DA. The electronic equipment 1 may implement an image through a two-dimensional array of pixels that are arranged in the display area DA.

The non-display area NDA may be an area that does not display an image, and may surround (e.g., entirely surround) the display area DA. A driver for providing electrical signals or power to display devices arranged on the display area DA may be arranged in the non-display area NDA. A pad, which is an area to which an electronic element or a printed circuit board may be electrically connected, may be arranged in the non-display area NDA.

The electronic equipment 1 may have different lengths in an x-axis direction and in a y-axis direction. In an embodiment, as shown in FIG. 4, a length in the x-axis direction may be shorter than a length in the y-axis direction. In an embodiment, a length in the x-axis direction may be the same as a length in the y-axis direction. In an embodiment, a length in the x-axis direction may be longer than a length in the y-axis direction.

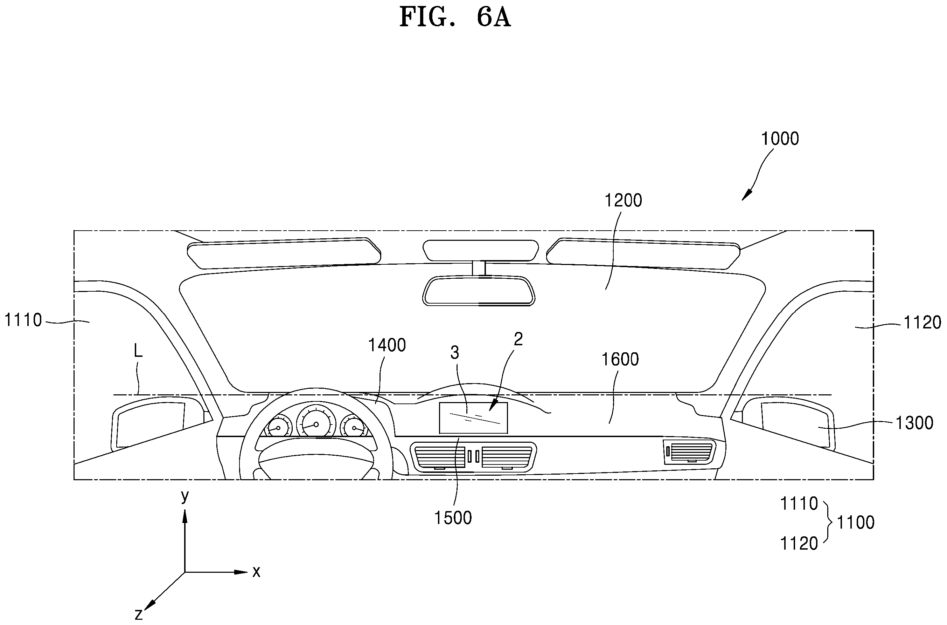

### [Descriptions of FIGS. 5 and 6A to 6C]

FIG. 5 is a schematic perspective view of an exterior of a vehicle 1000 as an electronic equipment including the light-emitting device, according to an embodiment. FIGS. 6A to 6C are each a schematic diagram of an interior of the vehicle 1000 according to embodiments.

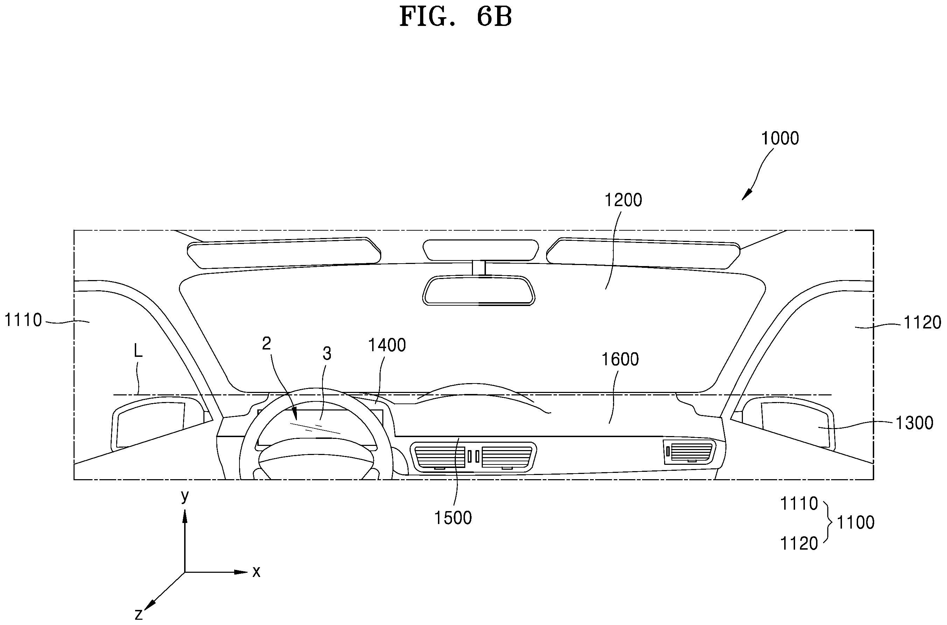

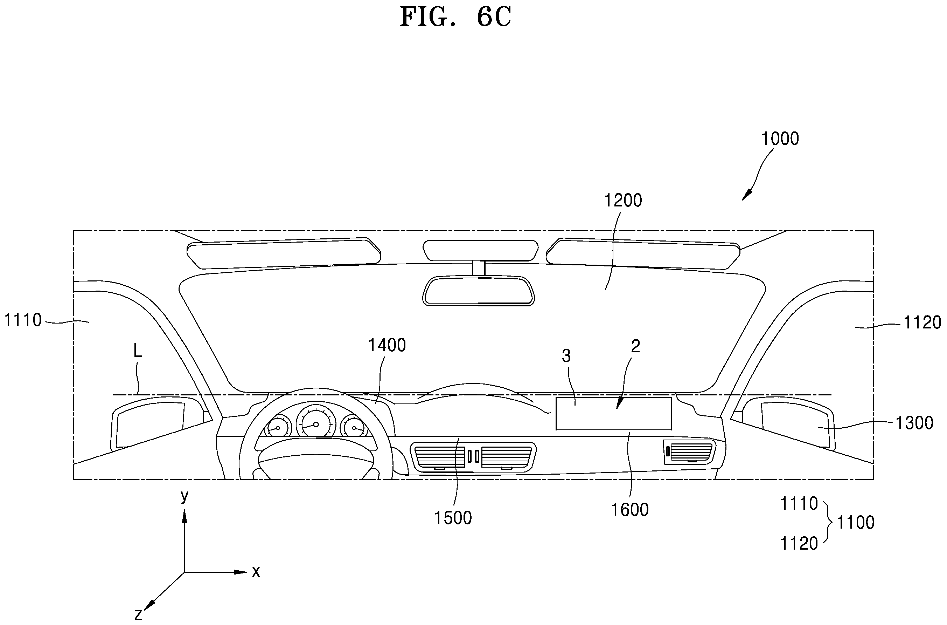

Referring to FIGS. 5, 6A, 6B, and 6C, embodiments of the vehicle 1000 may refer to various apparatuses for moving a subject to be transported, such as a person, an object, or an animal, from a departure point to a destination point. The vehicle 1000 may include a vehicle traveling on a road or a track, a vessel moving over the sea or river, an airplane flying in the sky using the action of air, and the like.

The vehicle 1000 may travel on a road or a track. The vehicle 1000 may move in a selectable direction according to rotation of at least one wheel. Examples of the vehicle 1000 may include a three-wheeled or four-wheeled vehicle, a construction machine, a two-wheeled vehicle, a prime mover device, a bicycle, and a train running on a track.

The vehicle 1000 may include a body of the vehicle 1000 having an interior and an exterior, and a chassis that is a portion excluding the body in which mechanical apparatuses necessary for driving are installed. The exterior of the body of the vehicle 1000 may include a front panel, a bonnet, a roof panel, a rear panel, a trunk, a pillar provided at a boundary between doors, and the like. The chassis of the vehicle 1000 may include a power generating device, a power transmitting device, a driving device, a steering device, a braking device, a suspension device, a transmission device, a fuel device, front and rear wheels, left and right wheels, and the like.

The vehicle 1000 may include a side window glass 1100, a front window glass 1200, a side-view mirror 1300, a cluster 1400, a center fascia 1500, a passenger seat dashboard 1600, and a display apparatus 2.

The side window glass 1100 and the front window glass 1200 may be partitioned by a pillar arranged between the side window glass 1100 and the front window glass 1200.

The side window glass 1100 may be installed on a side of the vehicle 1000. In an embodiment, the side window glass 1100 may be installed on a door of the vehicle 1000. Multiple side window glasses 1100 may be provided and may face each other. In an embodiment, the side window glass 1100 may include a first side window glass 1110 and a second side window glass 1120. In an embodiment, the first side window glass 1110 may be arranged adjacent to the cluster 1400, and the second side window glass 1120 may be arranged adjacent to the passenger seat dashboard 1600.

In an embodiment, the side window glasses 1100 may be spaced apart from each other in an x direction or a -x direction. In an embodiment, the first side window glass 1110 and the second side window glass 1120 may be spaced apart from each other in the x direction or the -x direction. For example, a virtual straight line L connecting the side window glasses 1100 may extend in the x direction or the -x direction. For example, a virtual straight line L connecting the first side window glass 1110 and the second side window glass 1120 to each other may extend in the x direction or the -x direction.

The front window glass 1200 may be installed in front of the vehicle 1000. The front window glass 1200 may be arranged between the side window glasses 1100 facing each other.

The side-view mirror 1300 may provide a rear view of the vehicle 1000. The side-view mirror 1300 may be installed on the exterior of the body of the vehicle. In an embodiment, multiple side-view mirrors 1300 may be provided. For example, one of the side-view mirrors 1300 may be arranged outside the first side window glass 1110, and another of the side-view mirrors 1300 may be arranged outside the second side window glass 1120.

The cluster 1400 may be arranged in front of a steering wheel. The cluster 1400 may include a tachometer, a speedometer, a coolant thermometer, a fuel gauge, a turn signal indicator, a high beam indicator, a warning light, a seat belt warning light, an odometer, a tachograph, an automatic shift selector indicator, a door open warning light, an engine oil warning light, and/or a low fuel warning light.

The center fascia 1500 may include a control panel on which buttons for adjusting an audio device, an air conditioning device, and a seat heater are disposed. The center fascia 1500 may be arranged on a side of the cluster 1400.

The passenger seat dashboard 1600 may be spaced apart from the cluster 1400, and the center fascia 1500 may be arranged between the cluster 1400 and the passenger seat dashboard 1600. In an embodiment, the cluster 1400 may be arranged to correspond to a driver seat (not shown), and the passenger seat dashboard 1600 may be arranged to correspond to a passenger seat (not shown). In an embodiment, the cluster 1400 may be adjacent to the first side window glass 1110, and the passenger seat dashboard 1600 may be adjacent to the second side window glass 1120.

In an embodiment, the display apparatus 2 may include a display panel 3, and the display panel 3 may display an image. The display apparatus 2 may be arranged inside the vehicle 1000. In an embodiment, the display apparatus 2 may be arranged between the side window glasses 1100 facing each other. The display apparatus 2 may be arranged on at least one of the cluster 1400, the center fascia 1500, and the passenger seat dashboard 1600.

The display apparatus 2 may include an organic light-emitting display device, an inorganic electroluminescent (EL) display device, a quantum dot display device, and the like. Hereinafter, an organic light-emitting display apparatus including the light-emitting device according to an embodiment will be described as an example of the display apparatus 2. However, various types of display apparatuses as described above may be used in embodiments.

Referring to FIG. 6A, the display apparatus 2 may be arranged on the center fascia 1500. In an embodiment, the display apparatus 2 may display navigation information. In an embodiment, the display apparatus 2 may display information regarding audio settings, video settings, or vehicle settings.

Referring to FIG. 6B, the display apparatus 2 may be arranged on the cluster 1400. In an embodiment, the cluster 1400 may display driving information and the like through the display apparatus 2. For example, the cluster 1400 may digitally implement driving information and the like. The cluster 1400 may digitally implement vehicle information and driving information as images. In an embodiment, a needle and a gauge of a tachometer and various warning lights or icons may be displayed by a digital signal.

Referring to FIG. 6C, the display apparatus 2 may be arranged on the passenger seat dashboard 1600. The display apparatus 2 may be embedded in the passenger seat dashboard 1600 or arranged on the passenger seat dashboard 1600. In an embodiment, the display apparatus 2 arranged on the passenger seat dashboard 1600 may display an image that is related to information displayed on the cluster 1400 and/or information displayed on the center fascia 1500. In an embodiment, the display apparatus 2 arranged on the passenger seat dashboard 1600 may display information that is different from information displayed on the cluster 1400 and/or information displayed on the center fascia 1500.

### [Manufacturing method]

The layers constituting the hole transport region 120, the emission layer 130, and the layers constituting the electron transport region 140 may be formed in a selected region by using various methods such as vacuum deposition, spin coating, casting, Langmuir-Blodgett (LB) deposition, ink-jet printing, laser-printing, laser-induced thermal imaging, and the like.

When the layers constituting the hole transport region 120, the emission layer 130, and the layers constituting the electron transport region 140 are formed by vacuum deposition, the deposition may be performed at a deposition temperature of about 100 °C to about 500 °C, a vacuum degree of about 10⁻⁸ torr to about 10⁻³ torr, and a deposition speed of about 0.01 Å/sec to about 100 Å/sec, depending on a material to be included in a layer to be formed and the structure of a layer to be formed.

### [Definitions of terms]

The term "C₃-C₆₀ carbocyclic group" as used herein may be a cyclic group consisting of carbon atoms as the only ring-forming atoms and having 3 to 60 carbon atoms.

The term "C₁-C₆₀ heterocyclic group" as used herein may be a cyclic group that has 1 to 60 carbon atoms and further has, in addition to carbon atoms, at least one heteroatom as a ring-forming atom.

The C₃-C₆₀ carbocyclic group and the C₁-C₆₀ heterocyclic group may each be a monocyclic group consisting of one ring or a polycyclic group in which two or more rings are condensed with each other. In an embodiment, the C₁-C₆₀ heterocyclic group may have 3 to 61 ring-forming atoms.

The term "cyclic group" as used herein may be a C₃-C₆₀ carbocyclic group or a C₁-C₆₀ heterocyclic group.

The term "π electron-rich C₃-C₆₀ cyclic group" as used herein may be a cyclic group that has 3 to 60 carbon atoms and may not include *-N=*' as a ring-forming moiety.

The term "π electron-deficient nitrogen-containing C₁-C₆₀ cyclic group" as used herein may be a heterocyclic group that has 1 to 60 carbon atoms and may include *-N=*' as a ring-forming moiety.

In an embodiment,
a C₃-C₆₀ carbocyclic group may be a T1 group or a group in which two or more T1 groups are condensed with each other (for example, a cyclopentadiene group, an adamantane group, a norbornane group, a benzene group, a pentalene group, a naphthalene group, an azulene group, an indacene group, an acenaphthylene group, a phenalene group, a phenanthrene group, an anthracene group, a fluoranthene group, a triphenylene group, a pyrene group, a chrysene group, a perylene group, a pentaphene group, a heptalene group, a naphthacene group, a picene group, a hexacene group, a pentacene group, a rubicene group, a coronene group, an ovalene group, an indene group, a fluorene group, a spiro-bifluorene group, a benzofluorene group, an indenophenanthrene group, or an indenoanthracene group),
a C₁-C₆₀ heterocyclic group may be a T2 group, a group in which two or more T2 groups are condensed with each other, or a group in which at least one T2 group and at least one T1 group are condensed with each other (e.g., a pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, a xanthene group, or the like),
a π electron-rich C₃-C₆₀ cyclic group may be a T1 group, a group in which two or more T1 groups are condensed with each other, a T3 group, a group in which two or more T3 groups are condensed with each other, or a group in which at least one T3 group and at least one T1 group are condensed with each other (e.g., a C₃-C₆₀ carbocyclic group, a 1H-pyrrole group, a silole group, a borole group, a 2H-pyrrole group, a 3H-pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, or the like),
a π electron-deficient nitrogen-containing C₁-C₆₀ cyclic group may be a T4 group, a group in which two or more T4 groups are condensed with each other, a group in which at least one T4 group and at least one T1 group are condensed with each other, a group in which at least one T4 group and at least one T3 group are condensed with each other, or a group in which at least one T4 group, at least one T1 group, and at least one T3 group are condensed with one another (for example, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, and the like),
a T1 group may be a cyclopropane group, a cyclobutane group, a cyclopentane group, a cyclohexane group, a cycloheptane group, a cyclooctane group, a cyclobutene group, a cyclopentene group, a cyclopentadiene group, a cyclohexene group, a cyclohexadiene group, a cycloheptene group, an adamantane group, a norbornane (or bicyclo[2.2.1]heptane) group, a norbornene group, a bicyclo[1.1.1]pentane group, a bicyclo[2.1.1]hexane group, a bicyclo[2.2.2]octane group, or a benzene group,
a T2 group may be a furan group, a thiophene group, a 1H-pyrrole group, a silole group, a borole group, a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, a tetrazine group, a pyrrolidine group, an imidazolidine group, a dihydropyrrole group, a piperidine group, a tetrahydropyridine group, a dihydropyridine group, a hexahydropyrimidine group, a tetrahydropyrimidine group, a dihydropyrimidine group, a piperazine group, a tetrahydropyrazine group, a dihydropyrazine group, a tetrahydropyridazine group, or a dihydropyridazine group,
a T3 group may be a furan group, a thiophene group, a 1H-pyrrole group, a silole group, or a borole group, and
a T4 group may be a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, or a tetrazine group.

The terms "cyclic group", "C₃-C₆₀ carbocyclic group", "C₁-C₆₀ heterocyclic group", "π electron-rich C₃-C₆₀ cyclic group", or "π electron-deficient nitrogen-containing C₁-C₆₀ cyclic group" as used herein may each be a group condensed to any cyclic group, a monovalent group, or a polyvalent group (e.g., a divalent group, a trivalent group, a tetravalent group, or the like), according to the structure of a formula for which the corresponding term is used.

In an embodiment, a "benzene group" may be a benzo group, a phenyl group, a phenylene group, or the like, which may be readily understood by those of ordinary skill in the art according to the structure of a formula including the "benzene group."

Examples of a monovalent C₃-C₆₀ carbocyclic group or a monovalent C₁-C₆₀ heterocyclic group may include a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group.

Examples of a divalent C₃-C₆₀ carbocyclic group or a divalent C₁-C₆₀ heterocyclic group may include a C₃-C₁₀ cycloalkylene group, a C₁-C₁₀ heterocycloalkylene group, a C₃-C₁₀ cycloalkenylene group, a C₁-C₁₀ heterocycloalkenylene group, a C₆-C₆₀ arylene group, a C₁-C₆₀ heteroarylene group, a divalent non-aromatic condensed polycyclic group, and a divalent non-aromatic condensed heteropolycyclic group.

The term "C₁-C₆₀ alkyl group" as used herein may be a linear or branched aliphatic hydrocarbon monovalent group that has one to sixty carbon atoms, and examples thereof may include a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, and a tert-decyl group.

The term "C₁-C₆₀ alkylene group" as used herein may be a divalent group having a same structure as the C₁-C₆₀ alkyl group.

The term "C₂-C₆₀ alkenyl group" as used herein may be a monovalent hydrocarbon group having at least one carbon-carbon double bond in the middle or at a terminus of a C₂-C₆₀ alkyl group, and examples thereof may include an ethenyl group, a propenyl group, and a butenyl group.

The term "C₂-C₆₀ alkenylene group" as used herein may be a divalent group having a same structure as the C₂-C₆₀ alkenyl group.

The term "C₂-C₆₀ alkynyl group" as used herein may be a monovalent hydrocarbon group having at least one carbon-carbon triple bond in the middle or at a terminus of a C₂-C₆₀ alkyl group, and examples thereof may include an ethynyl group and a propynyl group.

The term "C₂-C₆₀ alkynylene group" as used herein may be a divalent group having a same structure as the C₂-C₆₀ alkynyl group.

The term "C₁-C₆₀ alkoxy group" as used herein may be a monovalent group represented by -O(A₁₀₁) (wherein A₁₀₁ may be a C₁-C₆₀ alkyl group), and examples thereof may include a methoxy group, an ethoxy group, and an isopropyloxy group.

The term "C₃-C₁₀ cycloalkyl group" as used herein may be a monovalent saturated hydrocarbon cyclic group having 3 to 10 carbon atoms, and examples thereof may include a cyclopropyl group, a cyclobutyl group, a cyclopentyl group, a cyclohexyl group, a cycloheptyl group, a cyclooctyl group, an adamantanyl group, a norbornanyl group (or bicyclo[2.2.1]heptyl group), a bicyclo[1.1.1]pentyl group, a bicyclo[2.1.1]hexyl group, a bicyclo[2.2.2]octyl group, and the like.

The term "C₃-C₁₀ cycloalkylene group" as used herein may be a divalent group having a same structure as the C₃-C₁₀ cycloalkyl group.

The term "C₁-C₁₀ heterocycloalkyl group" as used herein may be a monovalent cyclic group that has one to ten carbon atoms and further includes, in addition to the carbon atoms, at least one heteroatom as a ring-forming atom, and examples thereof may include a 1,2,3,4-oxatriazolidinyl group, a tetrahydrofuranyl group, and a tetrahydrothiophenyl group.

The term "C₁-C₁₀ heterocycloalkylene group" as used herein may be a divalent group having a same structure as the C₁-C₁₀ heterocycloalkyl group.

The term "C₃-C₁₀ cycloalkenyl group" as used herein may be a monovalent cyclic group that has three to ten carbon atoms and at least one carbon-carbon double bond in the cyclic structure thereof and no aromaticity, and examples thereof may include a cyclopentenyl group, a cyclohexenyl group, and a cycloheptenyl group.

The term "C₃-C₁₀ cycloalkenylene group" as used herein may be a divalent group having a same structure as the C₃-C₁₀ cycloalkenyl group.

The term "C₁-C₁₀ heterocycloalkenyl group" as used herein refers to a monovalent cyclic group that has one to ten carbon atoms that further includes, in addition to the carbon atoms, at least one heteroatom as a ring-forming atom, and has at least one double bond in the cyclic structure thereof. Examples of a C₁-C₁₀ heterocycloalkenyl group may include a 4,5-dihydro-1,2,3,4-oxatriazolyl group, a 2,3-dihydrofuranyl group, and a 2,3-dihydrothiophenyl group.

The term "C₁-C₁₀ heterocycloalkenylene group" as used herein may be a divalent group having a same structure as the C₁-C₁₀ heterocycloalkenyl group.

The term "C₆-C₆₀ aryl group" as used herein may be a monovalent group having a carbocyclic aromatic system of 6 to 60 carbon atoms.

The term "C₆-C₆₀ arylene group" as used herein may be a divalent group having a carbocyclic aromatic system of 6 to 60 carbon atoms.

Examples of a C₆-C₆₀ aryl group may include a phenyl group, a pentalenyl group, a naphthyl group, an azulenyl group, an indacenyl group, an acenaphthyl group, a phenalenyl group, a phenanthrenyl group, an anthracenyl group, a fluoranthenyl group, a triphenylenyl group, a pyrenyl group, a chrysenyl group, a perylenyl group, a pentaphenyl group, a heptalenyl group, a naphthacenyl group, a picenyl group, a hexacenyl group, a pentacenyl group, a rubicenyl group, a coronenyl group, and an ovalenyl group.

When the C₆-C₆₀ aryl group and the C₆-C₆₀ arylene group each include two or more rings, the respective two or more rings may be condensed with each other.

The term "C₁-C₆₀ heteroaryl group" as used herein may be a monovalent group having a heterocyclic aromatic system of 1 to 60 carbon atoms, further including, in addition to carbon atoms, at least one heteroatom, as ring-forming atoms.

The term "C₁-C₆₀ heteroarylene group" as used herein may be a divalent group having a heterocyclic aromatic system of 1 to 60 carbon atoms, further including, in addition to carbon atoms, at least one heteroatom, as ring-forming atoms.

Examples of a C₁-C₆₀ heteroaryl group may include a pyridinyl group, a pyrimidinyl group, a pyrazinyl group, a pyridazinyl group, a triazinyl group, a quinolinyl group, a benzoquinolinyl group, an isoquinolinyl group, a benzoisoquinolinyl group, a quinoxalinyl group, a benzoquinoxalinyl group, a quinazolinyl group, a benzoquinazolinyl group, a cinnolinyl group, a phenanthrolinyl group, a phthalazinyl group, and a naphthyridinyl group.

When the C₁-C₆₀ heteroaryl group and the C₁-C₆₀ heteroarylene group each include two or more rings, the respective two or more rings may be condensed with each other.

The term "monovalent non-aromatic condensed polycyclic group" as used herein may be a monovalent group having two or more rings condensed with each other, only carbon atoms (for example, eight to sixty carbon atoms) as ring-forming atoms, and no aromaticity in its molecular structure when considered as a whole. Examples of a monovalent non-aromatic condensed polycyclic group include an indenyl group, a fluorenyl group, a spiro-bifluorenyl group, a benzofluorenyl group, an indenophenanthrenyl group, and an indeno anthracenyl group.

The term "divalent non-aromatic condensed polycyclic group" as used herein may be a divalent group having a same structure as the monovalent non-aromatic condensed polycyclic group.

The term "monovalent non-aromatic condensed heteropolycyclic group" as used herein may be a monovalent group that has two or more rings condensed with each other that further includes, in addition to carbon atoms (for example, one to sixty carbon atoms), at least one heteroatom as a ring-forming atom, and has no aromaticity in its molecular structure when considered as a whole. Examples of a monovalent non-aromatic condensed heteropolycyclic group may include a pyrrolyl group, a thiophenyl group, a furanyl group, an indolyl group, a benzoindolyl group, a naphthoindolyl group, an isoindolyl group, a benzoisoindolyl group, a naphthoisoindolyl group, a benzosilolyl group, a benzothiophenyl group, a benzofuranyl group, a carbazolyl group, a dibenzosilolyl group, a dibenzothiophenyl group, a dibenzofuranyl group, an azacarbazolyl group, an azafluorenyl group, an azadibenzosilolyl group, an azadibenzothiophenyl group, an azadibenzofuranyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a benzopyrazolyl group, a benzimidazolyl group, a benzoxazolyl group, a benzothiazolyl group, a benzoxadiazolyl group, a benzothiadiazolyl group, an imidazopyridinyl group, an imidazopyrimidinyl group, an imidazotriazinyl group, an imidazopyrazinyl group, an imidazopyridazinyl group, an indenocarbazolyl group, an indolocarbazolyl group, a benzofurocarbazolyl group, a benzothienocarbazolyl group, a benzosilolocarbazolyl group, a benzoindolocarbazolyl group, a benzocarbazolyl group, a benzonaphthofuranyl group, a benzonaphthothiophenyl group, a benzonaphthosilolyl group, a benzofurodibenzofuranyl group, a benzofurodibenzothiophenyl group, and a benzothienodibenzothiophenyl group.

The term "divalent non-aromatic condensed heteropolycyclic group" as used herein may be a divalent group having a same structure as the monovalent non-aromatic condensed heteropolycyclic group.

The term "C₆-C₆₀ aryloxy group" as used herein may be a group represented by -O(A₁₀₂) (wherein A₁₀₂ may be a C₆-C₆₀ aryl group).

The term "C₆-C₆₀ arylthio group" as used herein may be a group represented by -S(A₁₀₃) (wherein A₁₀₃ may be a C₆-C₆₀ aryl group).

The term "C₇-C₆₀ arylalkyl group" as used herein may be a group represented by -(A₁₀₄)(A₁₀₅) (wherein A₁₀₄ may be a C₁-C₅₄ alkylene group, and A₁₀₅ may be a C₆-C₅₉ aryl group).

The term "C₂-C₆₀ heteroarylalkyl group" as used herein may be a group represented by -(A₁₀₆)(A₁₀₇) (wherein A₁₀₆ may be a C₁-C₅₉ alkylene group, and A₁₀₇ may be a C₁-C₅₉ heteroaryl group).

In the specification, the group "R₁₀ₐ" may be:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂).

In the specification, Q₁ to Q₃, Q₁₁ to Q₁₃, Q₂₁ to Q₂₃ and Q₃₁ to Q₃₃ may each independently be: hydrogen; deuterium; -F; -Cl; -Br; -I; a hydroxyl group; a cyano group; a nitro group; C₁-C₆₀ alkyl group; C₂-C₆₀ alkenyl group; C₂-C₆₀ alkynyl group; C₁-C₆₀ alkoxy group; or a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, and any combination thereof.

The term "heteroatom" as used herein may be any atom other than a carbon atom or a hydrogen. Examples of a heteroatom include O, S, N, P, Si, B, Ge, Se, and any combination thereof.

In the specification, examples of a "transition metal" may include Hf, Ta, W, Re, Os, Ir, Pt, Au, and the like.

In the specification, the term "D" refers to deuterium, the term "Ph" refers to a phenyl group, the term "Me" refers to a methyl group, the term "Et" refers to an ethyl group, the terms "tert-Bu", "^{t}Bu", or "Bu^{t}" each refer to a tert-butyl group, and the term "OMe" refers to a methoxy group.

The term "biphenyl group" as used herein may be a "phenyl group that is substituted with a phenyl group". For example, the "biphenyl group" may be a substituted phenyl group having a C₆-C₆₀ aryl group as a substituent.

The term "terphenyl group" as used herein may be "phenyl group substituted with a biphenyl group". The term "terphenyl group" as used herein may be a substituted phenyl group wherein the substituent is a C₆-C₆₀ aryl group substituted with a C₆-C₆₀ aryl group, or a substituted phenyl group wherein two substituents are present, and each substituent is a C₆-C₆₀ aryl group.

The symbols * and *' as used herein, unless defined otherwise, each refer to a binding site to a neighboring atom in a corresponding formula or moiety.

In the specification, the terms "x-axis", "y-axis", and "z-axis" are not limited to three axes in an orthogonal coordinate system, and may be interpreted in a broader sense than the aforementioned three axes in an orthogonal coordinate system. For example, the x-axis, y-axis, and z-axis may describe axes that are orthogonal to each other, or may describe axes that are in different directions that are not orthogonal to each other.

Hereinafter, a light-emitting device including an organic compound according to embodiments will be described in detail with reference to the Synthesis Examples and Examples.

### Synthesis Example 1 (Synthesis of Compound 1)

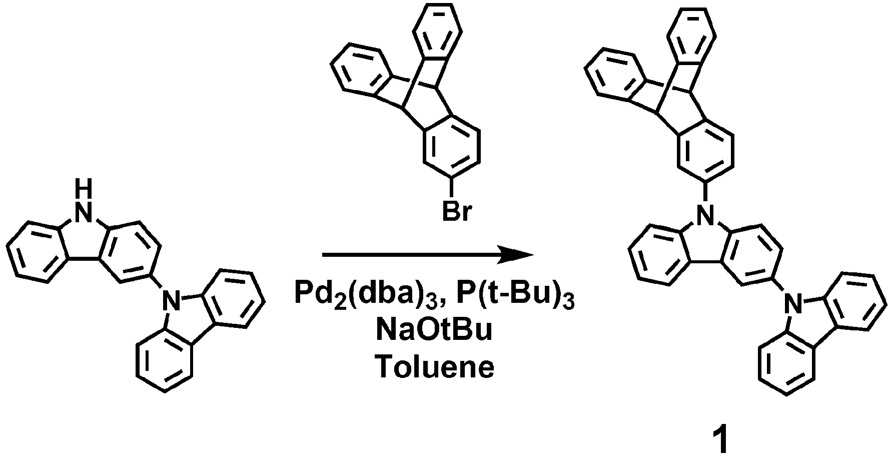

3,9'-bicarbazole (CAS No.: 18628-07-4) (10 g, 1 eq.), 2-bromotriptycene (CAS No.: 20712-00-9) (11 g, 1.1 eq.), sodium tert-butoxide (4.3 g, 1.5 eq.), Pd₂(dba)₃ (1.1 g, 0.04 eq.) and P(t-Bu)₃ (0.49 g, 0.08 eq.) were dissolved in 150 mL of toluene, and the mixed solution was stirred at 120 °C for 12 hours. After completion of the reaction, the reaction solution was subjected to an extraction process, and the organic layer thus obtained was dried. The residues were separated and purified by column chromatography, followed by recrystallization and sublimation purification, so as to obtain 14 g of Compound 1 (yield of 82%). Compound 1 was identified by LC-MS and 1H-NMR, and the results are shown in Table 1.

### Synthesis Example 2 (Synthesis of Compound 2)

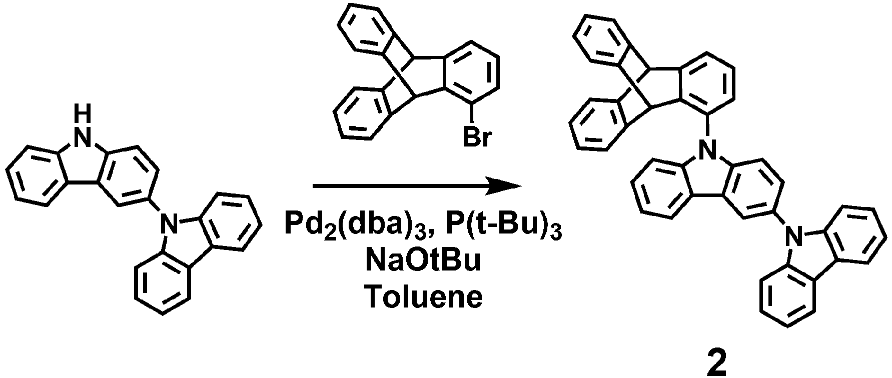

12 g of Compound 2 (yield of 69%) was obtained in the same manner as in Example 1, except that 1-bromotriptycene (CAS No.: 21192-04-1) (11 g, 1.1 eq.) was used instead of 2-bromotriptycene (CAS No.: 20712-00-9) (11 g, 1.1 eq.). Compound 2 was identified by LC-MS and 1H-NMR, and the results are shown in Table 1.

### Synthesis Example 3 (Synthesis of Compound 3)

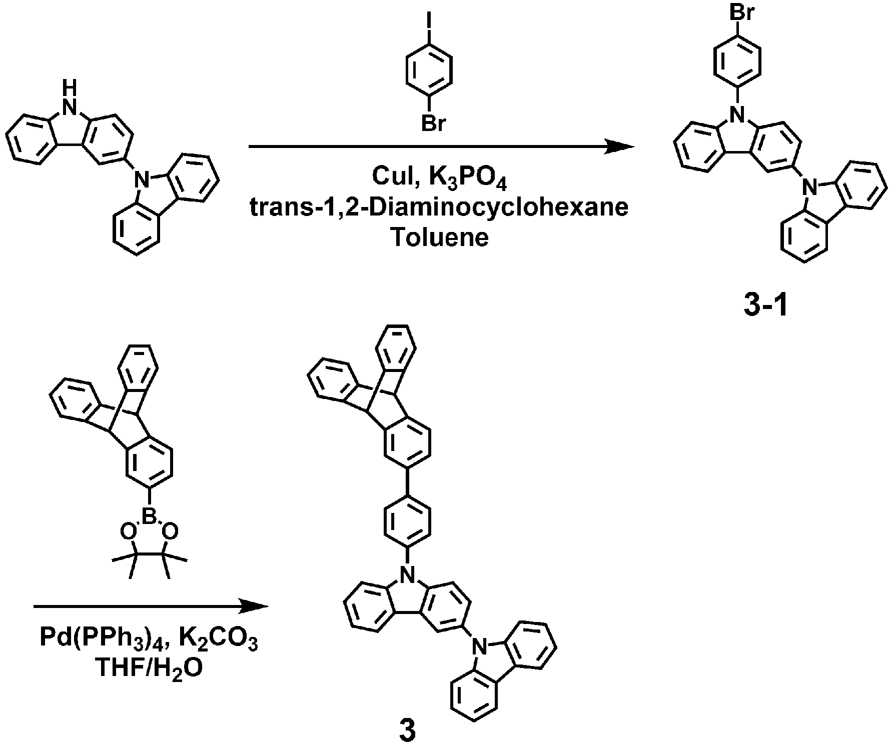

### Synthesis of Intermediate 3-1

3,9'-bicarbazole (1 eq.), 1-bromo-4-iodobenzene (CAS No.: 589-87-7) (1 eq.), Cul (0.5 eq.), K₃PO₄ (3 eq.), and trans-1,2-diaminocyclohexane (2 eq.) were dissolved in toluene, and the mixed solution was stirred overnight at 110 °C. The reaction solution was separated and purified by column chromatography, so as to obtain Intermediate 3-1, which was identified by LC-MS.
C₃₀H₁₉BrN₂ M+1 487.1

### Synthesis of Compound 3

Intermediate 3-1 (4.7 g, 1 eq.), 2-(9,10-dihydro-9,10[1',2']-benzenoanthracen-2-yl)-4,4,5,5-tetramethyl-1,3,2-dioxaborolane (CAS No.: 1803458-55-0) (4.6 g, 1.25 eq.), Pd(PPh₃)₄ (0.56 g, 0.05 eq.), and K₂CO₃ (4.0 g, 3 eq.) were dissolved in a mixed solvent containing tetrahydrofuran (THF)/water (50 mL/25 mL), and the mixed solution was stirred overnight at 85 °C. After completion of the reaction, the reaction solution was subjected to an extraction process, and the organic layer thus obtained was dried. The residues were separated and purified by column chromatography, followed by recrystallization and sublimation purification, so as to obtain 5.0 g of Compound 3 (yield of 78%). Compound 3 was identified by LC-MS and 1H-NMR, and the results are shown in Table 1.

### Synthesis Example 4 (synthesis of Compound 5)

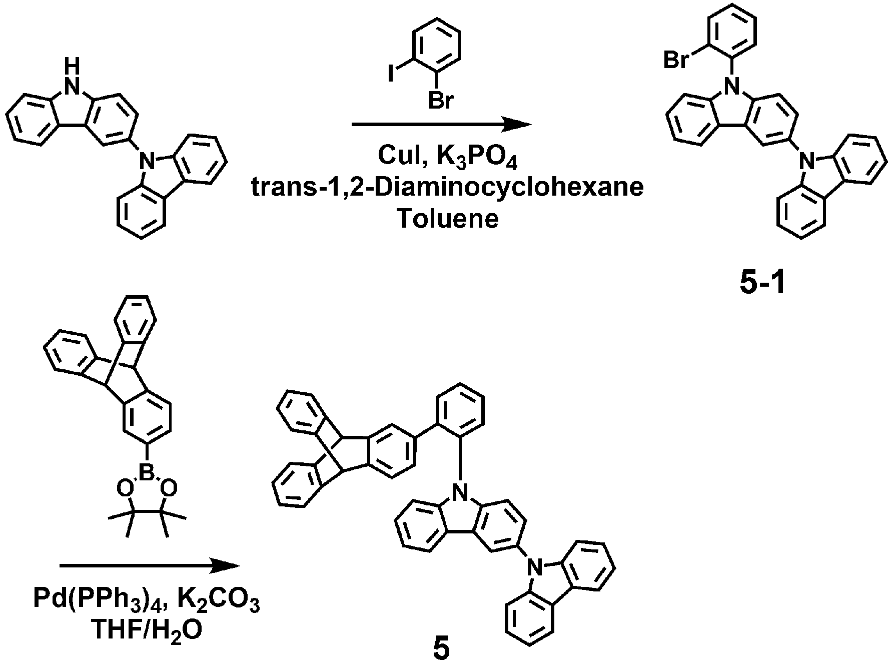

### Synthesis of Intermediate 5-1

Intermediate 5-1 was obtained in the same manner as in the synthesis of Intermediate 3-1, except that 1-bromo-2-iodobenzene (CAS No.: 583-55-1) (1 eq.) was used instead of 1-bromo-4-iodobenzene (CAS No.: 589-87-7) (1 eq.), and Intermediate 5-1 was identified by LC-MS.
C₃₀H₁₉BrN₂ M+1 487.1

### Synthesis of Compound 5

Intermediate 5-1 (6.3 g, 1 eq.), 2-(9,10-dihydro-9,10[1',2']-benzenoanthracen-2-yl)-4,4,5,5-tetramethyl-1,3,2-dioxaborolane (6.1 g, 1.25 eq.), Pd(PPh₃)₄ (0.75 g, 0.05 eq.), and K₂CO₃ (5.4 g, 3 eq.) were dissolved in a mixed solvent of THF/water (65 mL/30 mL), and the mixed solution was stirred overnight at 85 °C. After completion of the reaction, the reaction solution was subjected to an extraction process, and the organic layer thus obtained was dried. The residues were separated and purified by column chromatography, followed by recrystallization and sublimation purification, so as to obtain 5.6 g of Compound 5 (yield of 65%). Compound 5 was identified by LC-MS and 1H-NMR, and the results are shown in Table 1.

### Synthesis Example 5 (Synthesis of Compound 9)

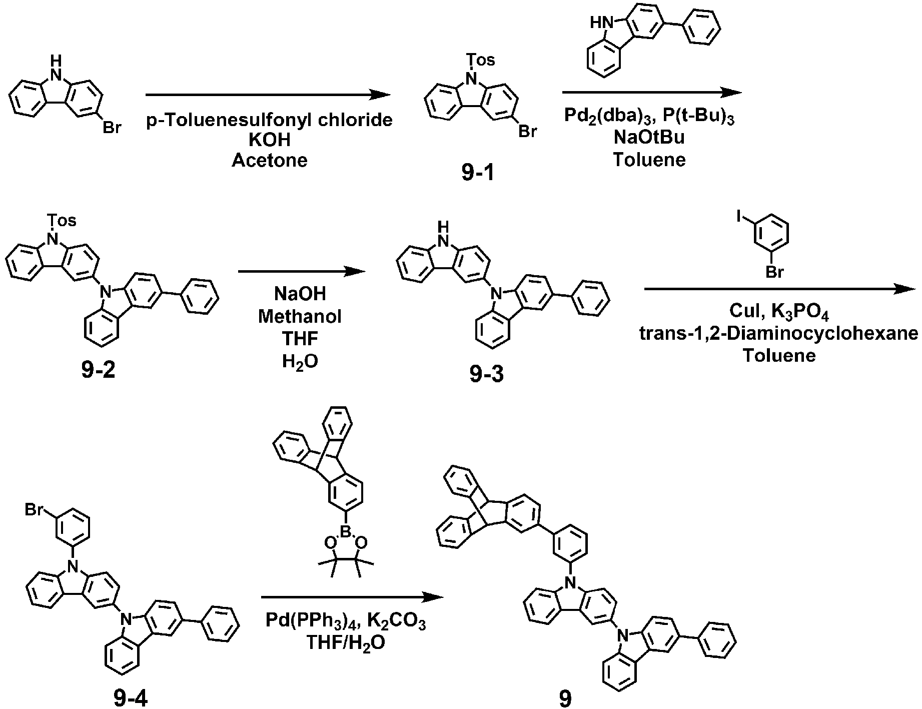

### Synthesis of Intermediate 9-1

3-bromocarbazole (CAS No.: 1592-95-6) (1 eq.) was dissolved in acetone, and p-toluenesulfonyl chloride (1.5 eq.) and KOH (2 eq.) were added thereto. The mixed solution was stirred at 60 °C for 6 hours. The reaction solution was subjected to an extraction process, and the organic layer thus obtained was dried. The reaction solution was separated and purified by column chromatography, so as to obtain Intermediate 9-1, which was identified by LC-MS.
C₁₉H₁₄BrNO₂S M+1 400.0

### Synthesis of Intermediate 9-2

Intermediate 9-1 (1 eq.), 3-phenylcarbazole (CAS No.: 103012-26-6) (1 eq.), sodium tert-butoxide (1.5 eq.), Pd₂(dba)₃ (0.04 eq.), and P(t-Bu)₃ (0.08 eq.) were dissolved in toluene, and the mixed solution was stirred at 120 °C for 12 hours. The reaction solution was subjected to an extraction process, and the organic layer thus obtained was dried. The reaction solution was separated and purified by column chromatography, so as to obtain Intermediate 9-2, which was identified by LC-MS.
C₃₇H₂₆N₂O₂S M+1 563.2

### Synthesis of Intermediate 9-3

Intermediate 9-2 was dissolved in in a mixed solvent of THF/methanol/water, and NaOH (10 eq.) was added thereto. The mixed solution was stirred at 90 °C for 12 hours. The reaction solution was subjected to an extraction process, and the organic layer thus obtained was dried. The reaction solution was separated and purified by column chromatography, so as to obtain Intermediate 9-3, which was identified by LC-MS.
C₃₀H₂₀N₂ M+1 409.2

### Synthesis of Intermediate 9-4

Intermediate 9-3 (1 eq.), 1-bromo-3-iodobenzene (CAS No.: 591-18-4) (1 eq.), Cul (0.5 eq.), K₃PO₄ (3 eq.), and trans-1,2-diaminocyclohexane (2 eq.) were dissolved in toluene, and the mixed solution was stirred overnight at 110 °C. The reaction solution was separated and purified by column chromatography, so as to obtain Intermediate 9-4, which was identified by LC-MS.
C₃₆H₂₃BrN₂ M+1 563.1

### Synthesis of Compound 9

Intermediate 9-4 (4.1 g, 1 eq.), 2-(9,10-dihydro-9,10[1',2']-benzenoanthracen-2-yl)-4,4,5,5-tetramethyl-1,3,2-dioxaborolane (3.5 g, 1.25 eq.), Pd(PPh₃)₄ (0.42 g, 0.05 eq.), and K₂CO₃ (3.0 g, 3 eq.) were dissolved in a mixed solvent of THF/water (40 mL/20 mL), and the mixed solution was stirred overnight at 85 °C. After completion of the reaction, the reaction solution was subjected to an extraction process, and the organic layer thus obtained was dried. The residues were separated and purified by column chromatography, followed by recrystallization and sublimation purification, so as to obtain 4.3 g of Compound 9 (yield of 81%). Compound 9 was identified by LC-MS and 1H-NMR, and the results are shown in Table 1.

### Synthesis Example 6 (synthesis of Compound 15)

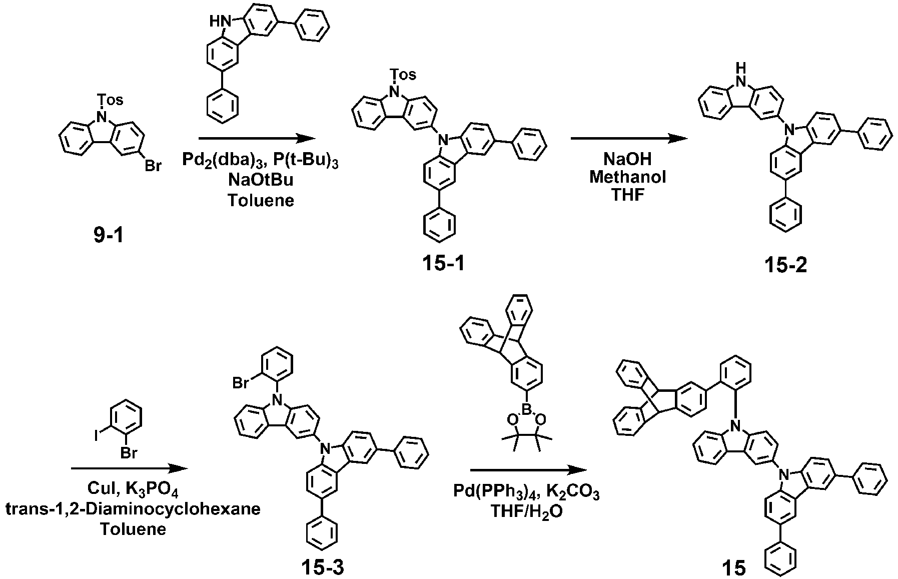

### Synthesis of Intermediate 15-1

Intermediate 9-1 (1 eq.), 3,6-diphenylcarbazole (CAS No.: 56525-79-2) (1 eq.), sodium tert-butoxide (1.5 eq.), Pd₂(dba)₃ (0.04 eq.), and P(t-Bu)₃ (0.08 eq.) were dissolved in toluene, and the mixed solution was stirred at 120 °C for 12 hours. The reaction solution was subjected to an extraction process, and the organic layer thus obtained was dried. The reaction solution was separated and purified by column chromatography, so as to obtain Intermediate 15-1, which was identified by LC-MS.
C₄₃H₃₀N₂O₂S M+1 639.2

### Synthesis of Intermediate 15-2

Intermediate 15-1 was dissolved in in a mixed solvent of THF/methanol/water, and NaOH (10 eq.) was added thereto. The mixed solution was stirred at 90 °C for 12 hours. The reaction solution was subjected to an extraction process, and the organic layer thus obtained was dried. The reaction solution was separated and purified by column chromatography, so as to obtain Intermediate 15-2, which was identified by LC-MS.
C₃₆H₂₄N₂ M+1 485.2

### Synthesis of Intermediate 15-3

Intermediate 15-2 (1 eq.), 1-bromo-2-iodobenzene (1 eq.), Cul (0.5 eq.), K₃PO₄ (3 eq.), and trans-1,2-diaminocyclohexane (2 eq.) were dissolved in toluene, and the mixed solution was stirred overnight at 110 °C. The reaction solution was separated and purified by column chromatography, so as to obtain Intermediate 15-3, which was identified by LC-MS.
C₄₂H₂₇BrN₂ M+1 639.1

### Synthesis of Compound 15

Intermediate 15-3 (2.9 g, 1 eq.), 2-(9,10-dihydro-9,10[1',2']-benzenoanthracen-2-yl)-4,4,5,5-tetramethyl-1,3,2-dioxaborolane (2.2 g, 1.25 eq.), Pd(PPh₃)₄ (0.26 g, 0.05 eq.), and K₂CO₃ (1.9 g, 3 eq.) were dissolved in a mixed solvent of THF/water (20 mL/10 mL), and the mixed solution was stirred overnight at 85 °C. After completion of the reaction, the reaction solution was subjected to an extraction process, and the organic layer thus obtained was dried. The residues were separated and purified by column chromatography, followed by recrystallization and sublimation purification, so as to obtain 2.1 g of Compound 15 (yield of 58%). Compound 15 was identified by LC-MS and 1H-NMR, and the results are shown in Table 1.

### Synthesis Example 7 (Synthesis of Compound 20)

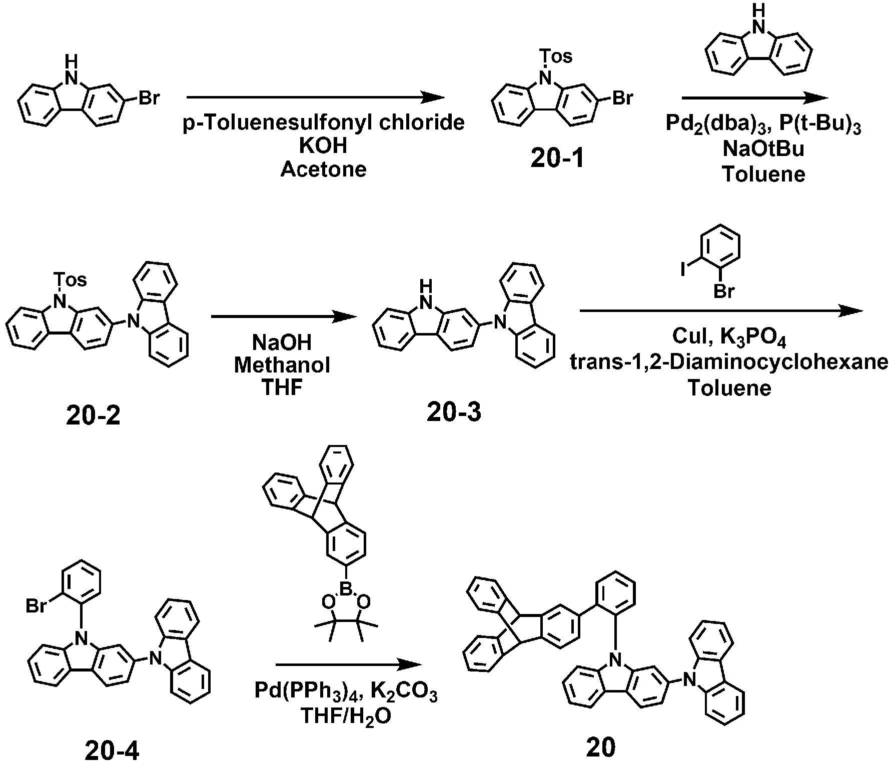

### Synthesis of Intermediate 20-1

2-bromocarbazole (CAS No.: 3652-90-2) (1 eq.) was dissolved in acetone, and p-toluenesulfonyl chloride (1.5 eq.) and KOH (2 eq.) were added thereto. The mixed solution was stirred at 60 °C for 6 hours. The reaction solution was subjected to an extraction process, and the organic layer thus obtained was dried. The reaction solution was separated and purified by column chromatography, so as to obtain Intermediate 20-1, which was identified by LC-MS.
C₁₉H₁₄BrNO₂S M+1 400.0

### Synthesis of Intermediate 20-2

Intermediate 20-1 (1 eq.), carbazole (CAS No.: 86-74-8) (1 eq.), sodium tert-butoxide (1.5 eq.), Pd₂(dba)₃ (0.04 eq.), and P(t-Bu)₃ (0.08 eq.) were dissolved in toluene, and the mixed solution was stirred at 120 °C for 12 hours. The reaction solution was subjected to an extraction process, and the organic layer thus obtained was dried. The reaction solution was separated and purified by column chromatography, so as to obtain Intermediate 20-2, which was identified by LC-MS.
C₃₁H₂₂N₂O₂S M+1 487.1

### Synthesis of Intermediate 20-3

Intermediate 20-2 was dissolved in in a mixed solvent of THF/methanol/water, and NaOH (10 eq.) was added thereto. The mixed solution was stirred at 90 °C for 12 hours. The reaction solution was subjected to an extraction process, and the organic layer thus obtained was dried. The reaction solution was separated and purified by column chromatography, so as to obtain Intermediate 20-3, which was then identified by LC-MS.
C₂₄H₁₆N₂ M+1 333.1

### Synthesis of Intermediate 20-4

Intermediate 20-3 (1 eq.), 1-bromo-2-iodobenzene (1 eq.), Cul (0.5 eq.), K₃PO₄ (3 eq.), and trans-1,2-diaminocyclohexane (2 eq.) were dissolved in toluene, and the mixed solution was stirred overnight at 110 °C. The reaction solution was separated and purified by column chromatography, so as to obtain Intermediate 20-4, which was identified by LC-MS.
C₃₀H₁₉BrN₂ M+1 487.1

### Synthesis of Compound 20

Intermediate 20-4 (6.1 g, 1 eq.), 2-(9,10-dihydro-9,10[1',2']-benzenoanthracen-2-yl)-4,4,5,5-tetramethyl-1,3,2-dioxaborolane (5.9 g, 1.25 eq.), Pd(PPh₃)₄ (0.72 g, 0.05 eq.), and K₂CO₃ (5.2 g, 3 eq.) were dissolved in a mixed solvent of THF/water (60 mL/30 mL), and the mixed solution was stirred overnight at 85 °C. After completion of the reaction, the reaction solution was subjected to an extraction process, and the organic layer thus obtained was dried. The residues were separated and purified by column chromatography, followed by recrystallization and sublimation purification, so as to obtain 4.5 g of Compound 20 (yield of 54%). Compound 20 was identified by LC-MS and 1H-NMR, and the results are shown in Table 1.

### Synthesis Example 8 (Synthesis of Compound 28)

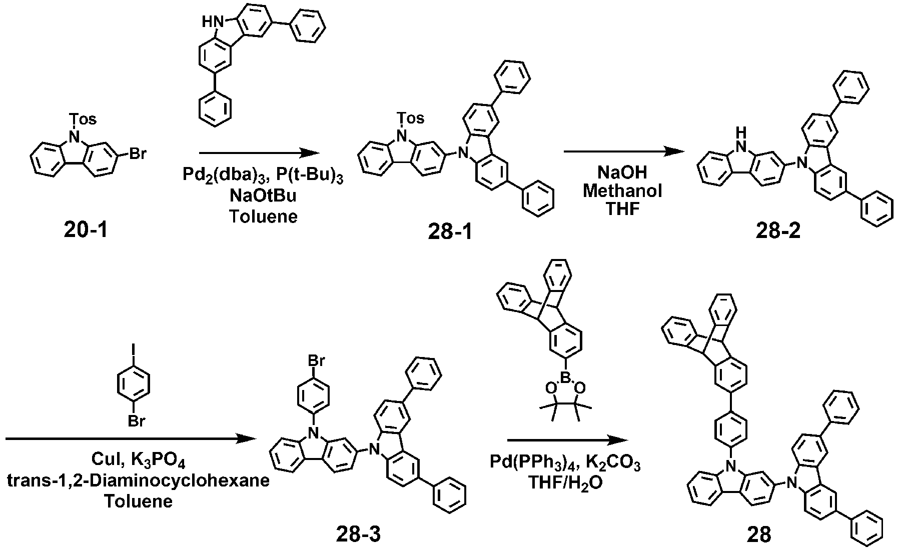

### Synthesis of Intermediate 28-1

Intermediate 20-1 (1 eq.), 3,6-diphenylcarbazole (1 eq.), sodium tert-butoxide (1.5 eq.), Pd₂(dba)₃ (0.04 eq.), and P(t-Bu)₃ (0.08 eq.) were dissolved in toluene, and the mixed solution was stirred at 120 °C for 12 hours. The reaction solution was subjected to an extraction process, and the organic layer thus obtained was dried. The reaction solution was separated and purified by column chromatography, so as to obtain Intermediate 28-1, which was identified by LC-MS.
C₄₃H₃₀N₂O₂S M+1 639.2

### Synthesis of Intermediate 28-2

Intermediate 28-1 was dissolved in in a mixed solvent of THF/methanol/water, and NaOH (10 eq.) was added thereto. The mixed solution was stirred at 90 °C for 12 hours. The reaction solution was subjected to an extraction process, and the organic layer thus obtained was dried. The reaction solution was separated and purified by column chromatography, so as to obtain Intermediate 28-2, which was identified by LC-MS.
C₃₆H₂₄N₂ M+1 485.2

### Synthesis of Intermediate 28-3

Intermediate 28-2 (1 eq.), 1-bromo-4-iodobenzene (1 eq.), Cul (0.5 eq.), K₃PO₄ (3 eq.), and trans-1,2-diaminocyclohexane (2 eq.) were dissolved in toluene, and the mixed solution was stirred overnight at 110 °C. The reaction solution was separated and purified by column chromatography, so as to obtain Intermediate 28-3, which was identified by LC-MS.
C₄₂H₂₇BrN₂ M+1 639.1

### Synthesis of Compound 28

Intermediate 28-3 (2.7 g, 1 eq.), 2-(9,10-dihydro-9,10[1',2']-benzenoanthracen-2-yl)-4,4,5,5-tetramethyl-1,3,2-dioxaborolane (2.0 g, 1.25 eq.), Pd(PPh₃)₄ (0.24 g, 0.05 eq.), and K₂CO₃ (1.7 g, 3 eq.) were dissolved in a mixed solvent of THF/water (20 mL/10 mL), and the mixed solution was stirred overnight at 85 °C. After completion of the reaction, the reaction solution was subjected to an extraction process, and the organic layer thus obtained was dried. The residues were separated and purified by column chromatography, followed by recrystallization and sublimation purification, so as to obtain 2.2 g of Compound 28 (yield of 65%). Compound 28 was identified by LC-MS and 1H-NMR, and the results are shown in Table 1.

### Synthesis Example 9 (synthesis of Compound 58)

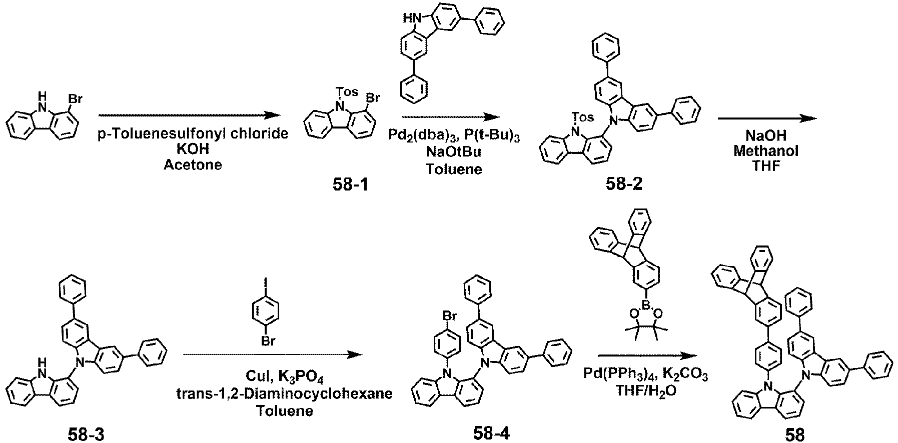

### Synthesis of Intermediate 58-1

1-bromocarbazole (CAS No.: 16807-11-7) (1 eq.) was dissolved in acetone, and p-toluenesulfonyl chloride (1.5 eq.), KOH (2 eq.) were added thereto. The mixed solution was stirred at 60 °C for 6 hours. The reaction solution was subjected to an extraction process, and the organic layer thus obtained was dried. The reaction solution was separated and purified by column chromatography, so as to obtain Intermediate 58-1, which was identified by LC-MS.
C₁₉H₁₄BrNO₂S M+1 400.0

### Synthesis of Intermediate 58-2

Intermediate 58-1 (1 eq.), 3,6-diphenylcarbazole (1 eq.), sodium tert-butoxide (1.5 eq.), Pd₂(dba)₃ (0.04 eq.), and P(t-Bu)₃ (0.08 eq.) were dissolved in toluene, and the mixed solution was stirred at 120 °C for 12 hours. The reaction solution was subjected to an extraction process, and the organic layer thus obtained was dried. The reaction solution was separated and purified by column chromatography, so as to obtain Intermediate 58-2, which was identified by LC-MS.
C₄₃H₃₀N₂O₂S M+1 639.8

### Synthesis of Intermediate 58-3

Intermediate 58-2 was dissolved in in a mixed solvent of THF/methanol/water, and NaOH (10 eq.) was added thereto. The mixed solution was stirred at 90 °C for 12 hours. The reaction solution was subjected to an extraction process, and the organic layer thus obtained was dried. The reaction solution was separated and purified by column chromatography, so as to obtain Intermediate 58-3, which was identified by LC-MS.
C₃₆H₂₄N₂ M+1 485.2

### Synthesis of Intermediate 58-4

Intermediate 58-3 (1 eq.), 1-bromo-4-iodobenzene (1 eq.), Cul (0.5 eq.), K₃PO₄ (3 eq.), and trans-1,2-diaminocyclohexane (2 eq.) were dissolved in toluene, and the mixed solution was stirred overnight at 110 °C. The reaction solution was separated and purified by column chromatography, so as to obtain Intermediate 58-4, which was identified by LC-MS.
C₄₂H₂₇BrN₂ M+1 639.1

### Synthesis of Compound 58

Intermediate 58-4 (3.5 g, 1 eq.), 2-(9,10-dihydro-9,10[1',2']-benzenoanthracen-2-yl)-4,4,5,5-tetramethyl-1,3,2-dioxaborolane (2.6 g, 1.25 eq.), Pd(PPh₃)₄ (0.32 g, 0.05 eq.), and K₂CO₃ (2.3 g, 3 eq.) were dissolved in a mixed solvent of THF/water (30 mL/15 mL), and the mixed solution was stirred overnight at 85 °C. After completion of the reaction, the reaction solution was subjected to an extraction process, and the organic layer thus obtained was dried. The residues were separated and purified by column chromatography, followed by recrystallization and sublimation purification, so as to obtain 2.1 g of Compound 58 (yield of 48%). Compound 58 was identified by LC-MS and 1H-NMR, and the results are shown in Table 1.

### Synthesis Example 10 (Synthesis of Compound 81)

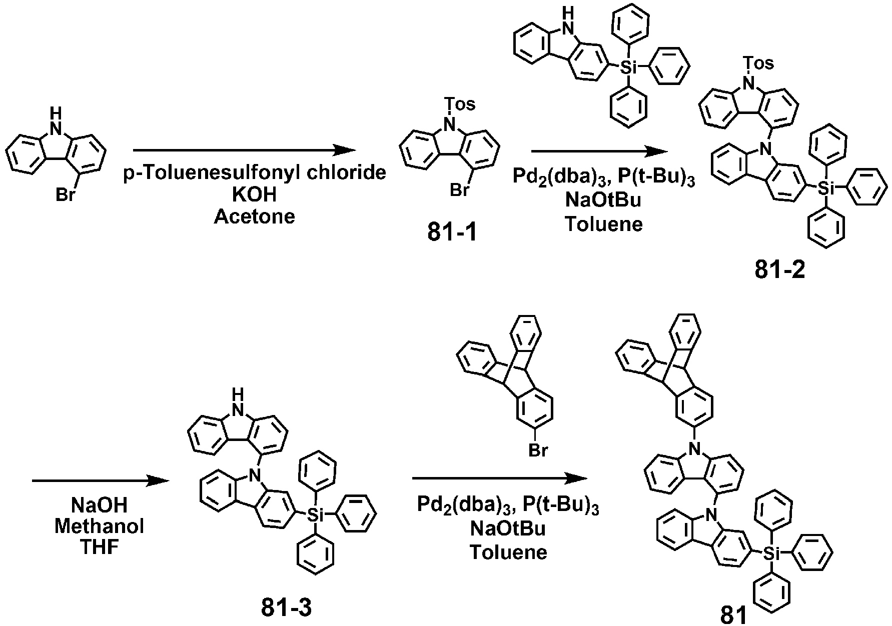

### Synthesis of Intermediate 81-1

4-bromocarbazole (CAS No.: 3652-89-9) (1 eq.) was dissolved in acetone, and p-toluenesulfonyl chloride (1.5 eq.), KOH (2 eq.) were added thereto. The mixed solution was stirred at 60 °C for 6 hours. The reaction solution was subjected to an extraction process, and the organic layer thus obtained was dried. The reaction solution was separated and purified by column chromatography, so as to obtain Intermediate 81-1, which was identified by LC-MS.
C₁₉H₁₄BrNO₂S M+1 400.0

### Synthesis of Intermediate 81-2

Intermediate 81-1 (1 eq.), 2-triphenylsilylcarbazole (CAS No.: 1262866-95-4) (1 eq.), sodium tert-butoxide (1.5 eq.), Pd₂(dba)₃ (0.04 eq.), and P(t-Bu)₃ (0.08 eq.) were dissolved in toluene, and the mixed solution was stirred at 120 °C for 12 hours. The reaction solution was subjected to an extraction process, and the organic layer thus obtained was dried. The reaction solution was separated and purified by column chromatography, so as to obtain Intermediate 81-2, which was identified by LC-MS.
C₄₉H₃₆N₂O₂SSi M+1 745.2

### Synthesis of Intermediate 81-3

Intermediate 81-2 was dissolved in in a mixed solvent of THF/methanol/water, and NaOH (10 eq.) was added thereto. The mixed solution was stirred at 90 °C for 12 hours. The reaction solution was subjected to an extraction process, and the organic layer thus obtained was dried. The reaction solution was separated and purified by column chromatography, so as to obtain Intermediate 81-3, which was identified by LC-MS.
C₄₂H₃₀N₂Si M+1 591.2

### Synthesis of Compound 81

Intermediate 81-3 (3.0 g, 1 eq.), 2-(9,10-dihydro-9,10[1',2']-benzenoanthracen-2-yl)-4,4,5,5-tetramethyl-1,3,2-dioxaborolane (2.4 g, 1.25 eq.), Pd(PPh₃)₄ (0.29 g, 0.05 eq.), and K₂CO₃ (2.1 g, 3 eq.) were dissolved in a solvent of toluene, and the mixed solution was stirred overnight at 85 °C. After completion of the reaction, the reaction solution was subjected to an extraction process, and the organic layer thus obtained was dried. The residues were separated and purified by column chromatography, followed by recrystallization and sublimation purification, so as to obtain 2.1 g of Compound 81 (yield of 50%). Compound 81 was identified by LC-MS and 1H-NMR, and the results are shown in Table 1.

The 1H NMR and LC-MS results for the compounds of Synthesis Examples 1 to 10 are shown in Table 1. Those skilled in the art may readily recognize the synthesis methods for compounds other than the compounds shown in Table 1 by referring to the aforementioned synthesis methods and raw materials.

**[Table 1]**

| Compound | 1H NMR (CDCl₃, 400 MHz) | LC-MS | |
|---|---|---|---|
| | | found [M+1] | Calcd. |
| 1 | 8.55 (d, 2H), 8.19 (d, 1H), 7.94 (d, 2H), 7.74-7.66 (m, 2H), 7.60-7.09 (m, 19H), 5.19 (s, 2H) | 585.3 | 584.2 |
| 2 | 8.56 (d, 2H), 8.20 (d, 1H), 7.94 (d, 2H), 7.73-7.65 (m, 2H), 7.59-7.08 (m, 19H), 5.19 (s, 2H) | 585.3 | 584.2 |
| 3 | 8.56 (d, 2H), 8.21 (d, 1H), 7.97-7.90 (m, 6H), 7.73-7.08 (m, 21H), 5.18 (s, 2H) | 661.2 | 660.3 |
| 5 | 8.55 (d, 2H), 8.19 (d, 1H), 7.96-7.90 (m, 4H), 7.73-7.08 (m, 23H), 5.19 (s, 2H) | 661.3 | 660.3 |
| 9 | 8.56 (d, 2H), 8.23 (dd, 1H), 8.00-7.88 (m, 4H), 7.80-7.11 (m, 27H), 5.19 (s, 2H) | 737.4 | 736.3 |
| 15 | 8.55 (d, 1H), 8.30 (dd, 1H), 8.11 (d, 1H), 8.00-7.10 (m, 35H), 5.18 (s, 2H) | 813.4 | 812.3 |
| 20 | 8.56 (d, 2H), 8.25-8.20 (m, 2H), 7.97-7.12 (m, 26H), 5.18 (s, 2H) | 661.3 | 660.3 |
| 28 | 8.56 (d, 1H), 8.30-8.26 (m, 2H), 8.13 (d, 1H), 8.01-7.11 (m, 34H), 5.19 (s, 2H) | 813.3 | 812.3 |
| 58 | 8.54 (d, 1H), 8.30 (dd, 1H), 8.16-8.12 (m, 2H), 8.00-7.09 (m, 34H), 5.19 (s, 2H) | 813.2 | 812.3 |
| 81 | 8.56 (d, 2H), 8.23 (d, 1H), 7.95 (dd, 2H), 7.70 (d, 1H), 7.55-7.10 (m, 34H), 5.19 (s, 2H) | 843.4 | 842.3 |

### Example 1

As an anode, a Corning 15 Ω/cm² (1,200 Å) ITO glass substrate was cut to a size of 50 mm x 50 mm x 0.5 mm, sonicated with isopropyl alcohol and pure water each for 5 minutes, and cleaned by exposure to ultraviolet rays and ozone for 30 minutes. The ITO glass substrate was provided to a vacuum deposition apparatus.

HAT-CN was vacuum-deposited on the substrate to form a hole injection layer having a thickness of 100 Å. BCFN was vacuum-deposited on the hole injection layer to form a first hole transport layer having a thickness of 600 Å. SiCzCz was vacuum-deposited on the first hole transport layer to form a second hole transport layer having a thickness of 50 Å.

On the second hole transport layer, Compound 1, Compound ETH2, and Compound PD38 were co-deposited at a weight ratio of 60:27:13 to form an emission layer having a thickness of 350 Å.

mSiTrz was vacuum-deposited on the emission layer to form a first electron transport layer having a thickness of 50 Å. mSiTrz and LiQ were co-deposited at a weight ratio 1:1 on the first electron transport layer to form a second electron transport layer having a thickness of 350 Å.

LiF was vacuum-deposited on the second electron transport layer to form an electron injection layer having a thickness of 15 Å. Al was vacuum-deposited on the electron injection layer to form an LiF/Al electrode having a thickness of 80 Å, thereby completing the manufacture of a light-emitting device.

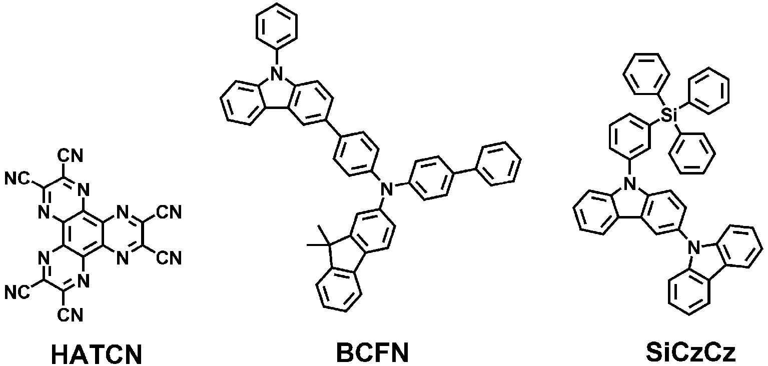

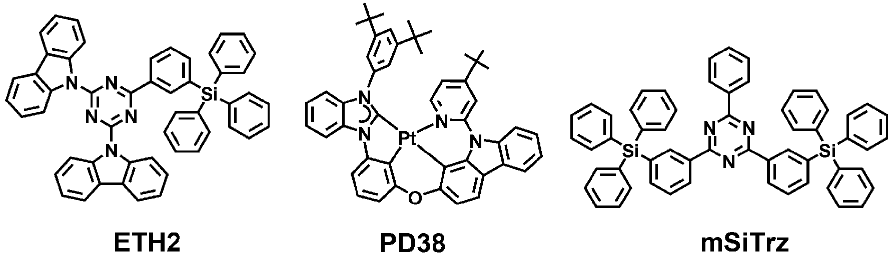

### Examples 2 to 10 and Comparative Examples 1 to 7

Light-emitting devices were manufactured in the same manner as in Example 1, except that the compounds shown in Table 2 were each used instead of Compound 1 in forming an emission layer.

### Evaluation Example 1

To evaluate the characteristics of the light-emitting devices of Examples 1 to 10 and Comparative Examples 1 to 7, a driving voltage, maximum quantum efficiency, and a lifespan (T₉₅), at a current density of 10 mA/cm², were measured. The driving voltage and the lifespan (T₉₅) of the light-emitting devices were measured by using a source meter (e.g., Keithley Instrument Company, 2400 series). Lifespan (T₉₅) refers to the time (hr) taken for the luminance to reach 95% of the initial luminance. The measured lifespan was calculated as a relative lifespan compared to Comparative Example 1. The maximum quantum efficiency may be measured by using an external quantum efficiency measuring device, C9920-2-12 from Hamamatsu Photonics Inc. In evaluating the maximum quantum efficiency, the luminance/current density was measured by using a luminance meter that was calibrated for wavelength sensitivity, and the maximum quantum efficiency was converted by assuming an angular luminance distribution (Lambertian) which introduced a perfect reflecting diffuser. The evaluation results of the characteristics of the light-emitting devices are shown in Table 2.

**[Table 2]**

| No. | Hole-transporting host | Driving voltage (V) | Maximum quantum efficiency (%) | Relative lifespan (%) | Emission color |
|---|---|---|---|---|---|
| Example 1 | Compound 1 | 5.0 | 21.2 | 127 | Blue |
| Example 2 | Compound 2 | 5.2 | 22.0 | 115 | Blue |
| Example 3 | Compound 3 | 5.2 | 22.3 | 129 | Blue |
| Example 4 | Compound 5 | 5.1 | 22.4 | 126 | Blue |
| Example 5 | Compound 9 | 5.4 | 21.5 | 113 | Blue |
| Example 6 | Compound 15 | 5.3 | 23.7 | 105 | Blue |
| Example 7 | Compound 20 | 5.2 | 21.9 | 111 | Blue |
| Example 8 | Compound 28 | 5.1 | 23.1 | 121 | Blue |
| Example 9 | Compound 58 | 5.1 | 23.2 | 122 | Blue |
| Example 10 | Compound 81 | 5.1 | 22.0 | 118 | Blue |
| Comparative Example 1 | Compound CE1 | 5.2 | 22.9 | 100 | Blue |
| Comparative Example 2 | Compound CE2 | 5.7 | 20.8 | 65 | Blue |
| Comparative Example 3 | Compound CE3 | 5.9 | 21.2 | 72 | Blue |
| Comparative Example 4 | Compound CE4 | 5.7 | 22.3 | 73 | Blue |
| Comparative Example 5 | Compound CE5 | 5.3 | 23.2 | 102 | Blue |
| Comparative Example 6 | Compound CE6 | 5.2 | 21.3 | 108 | Blue |
| Comparative Example 7 | Compound CE7 | 5.3 | 20.1 | 88 | Blue |

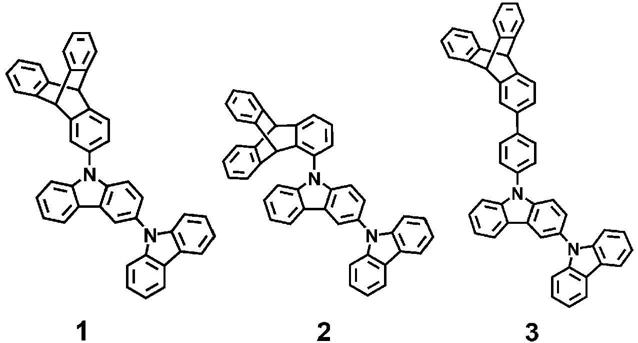

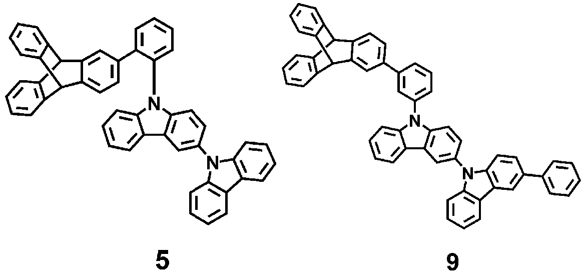

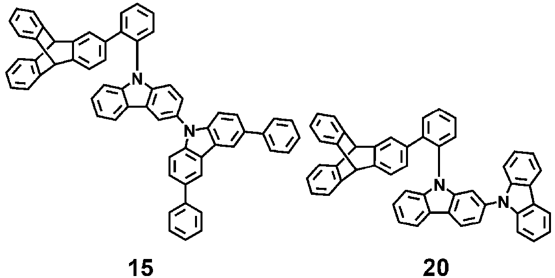

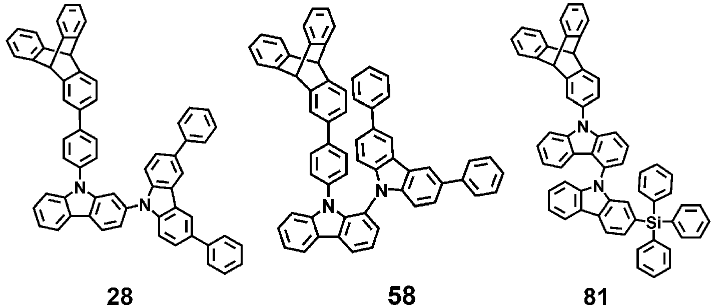

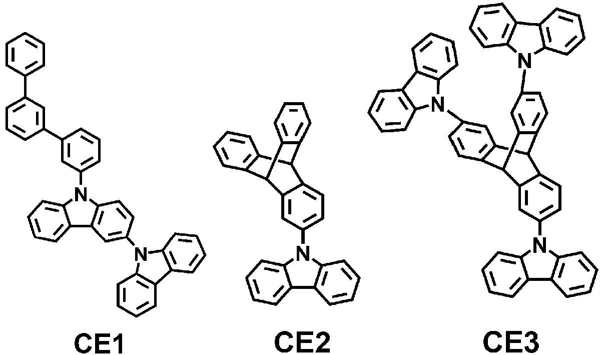

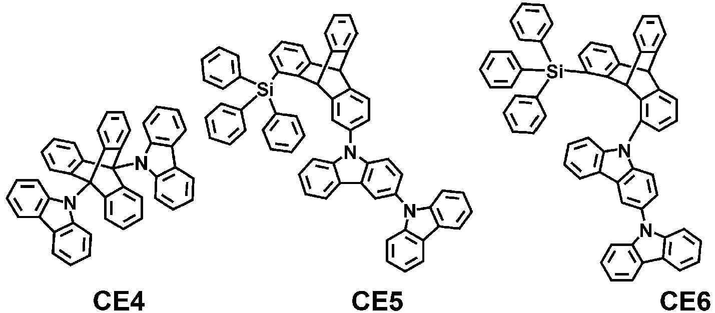

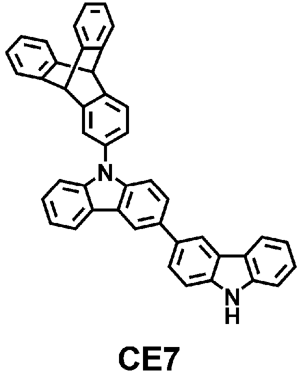

Referring to Table 2, it was confirmed that the light-emitting devices of Examples 1 to 10 including the organic compound represented by Formula 1 exhibited at least one improvement among the driving voltage, the maximum quantum efficiency, and the lifespan, as compared to the light-emitting devices of Comparative Examples 1 to 7 including the compounds that are not represented by Formula 1. For example, the light-emitting devices of the Examples were found to have effectively excellent lifespan while having a similar level of the driving voltage and a similar level of the maximum quantum efficiency, as compared with the light-emitting devices of the Comparative Examples.

When comparing Examples 1 to 10 with Comparative Example 1, it was confirmed that, due to the triptycene moiety having a larger molecular weight than the terphenyl moiety, the characteristics of the driving voltage, the maximum quantum efficiency, and/or the lifespan were improved.

When comparing Example 1 with Comparative Example 2, it was confirmed that excellent characteristics were achieved by including bicarbazole rather than a single carbazole. Referring to Comparative Example 3, it was confirmed that Example 1 including bicarbazole had excellent driving voltage and long lifespan, as compared with Comparative Example 3 in which the number of single carbazoles was increased. Referring to Comparative Example 4, it was confirmed that Example 1 had excellent driving voltage and long lifespan, as compared with Comparative Example 4 which does not include bicarbazole and have some changes in the binding positions of single carbazole and triptycene.

When comparing Example 1 with Comparative Example 5 or comparing Example 2 with Comparative Example 6, it was confirmed that, even if bicarbazole was included, the driving voltage and/or lifespan characteristics were degraded when a silyl group (e.g., a triphenylsilyl group -Si(Ph)₃) was linked to two benzene groups other than a benzene group linked to the bicarbazole in the three benzene groups included in triptycene. When comparing Example 10 with Comparative Example 5, it was confirmed that Example 10 including Compound 81 in which a silyl group (e.g., a triphenylsilyl group -Si(Ph)₃) was linked to bicarbazole had excellent characteristics in terms of driving voltage, maximum quantum efficiency, and lifespan, indicating that different characteristics were obtained depending on the position of the silyl group.

When comparing Example 1 with Comparative Example 7, it was confirmed that, even if bicarbazole was included, the characteristics may significantly change depending on the binding position among the carbazoles.

According to embodiments, an organic compound represented by Formula 1 may have a structure of "triptycene group-(linking group)-two or more carbazole groups", thereby having high thermal stability and improved charge transport ability while maintaining a high glass transition temperature and a high triplet(T₁) energy level. Therefore, a light-emitting device including the organic compound may have a low driving voltage and high efficiency while having an effectively improved lifespan.

Embodiments have been disclosed herein, and although terms are employed, they are used and are to be interpreted in a generic and descriptive sense only and not for purposes of limitation. In some instances, as would be apparent by one of ordinary skill in the art, features, characteristics, and/or elements described in connection with an embodiment may be used singly or in combination with features, characteristics, and/or elements described in connection with other embodiments unless otherwise specifically indicated. Accordingly, it will be understood by those of ordinary skill in the art that various changes in form and details may be made without departing from the scope of the disclosure.

## Claims

1. A light-emitting device comprising:
a first electrode;
a second electrode facing the first electrode;
an interlayer between the first electrode and the second electrode and comprising an emission layer; and
an organic compound represented by Formula 1: 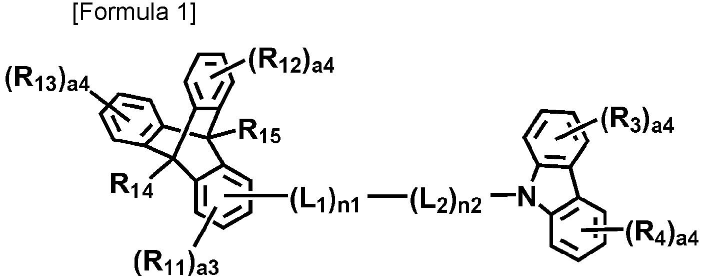
wherein in Formula 1,
L₁ is a C₃-C₆₀ carbocyclic group unsubstituted or substituted with at least one R₁ or a C₁-C₆₀ heterocyclic group unsubstituted or substituted with at least one R₁,
n1 is an integer from 0 to 3,
L₂ is a carbazole group unsubstituted or substituted with at least one R₂,
n2 is 1 or 2,
R₁ to R₄ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₁)(Q₂)(Q₃), -N(Q₁)(Q₂), -B(Q₁)(Q₂), -C(=O)(Q₁), -S(=O)₂(Q₁), or -P(=O)(Q₁)(Q₂),
R₁₁ to R₁₅ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -N(Q₁)(Q₂), - B(Q₁)(Q₂), -C(=O)(Q₁), -S(=O)₂(Q₁), or -P(=O)(Q₁)(Q₂),
a3 is an integer from 0 to 3,
a4 is an integer from 0 to 4,
R₁₀ₐ is:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or a combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), -P(=O)(Q₂₁)(Q₂₂), or a combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ are each independently:
hydrogen, deuterium, -F, -Cl, -Br, -I, a hydroxyl group, a cyano group, or a nitro group; or
a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or a combination thereof.

2. The light-emitting device of claim 1, wherein the interlayer comprises the organic compound.

3. The light-emitting device of claim 1 or claim 2, wherein the emission layer is configured to emit blue light.

4. An electronic apparatus comprising:
the light-emitting device of any one of claims 1 to 3; and
a thin-film transistor electrically connected to the light-emitting device.

5. An electronic equipment comprising:
the electronic apparatus of claim 4, wherein
the electronic equipment is a flat panel display, a curved display, a computer monitor, a medical monitor, a television, a billboard, an indoor light, an outdoor light, a signal light, a head-up display, a fully transparent display, a partially transparent display, a flexible display, a rollable display, a foldable display, a stretchable display, a laser printer, a telephone, a mobile phone, a tablet computer, a phablet, a personal digital assistant (PDA), a wearable device, a laptop computer, a digital camera, a camcorder, a viewfinder, a micro display, a three-dimensional (3D) display, a virtual reality display, an augmented reality display, a vehicle, a video wall with multiple displays tiled together, a theater screen, a stadium screen, a phototherapy device, or a signboard.

6. An organic compound represented by Formula 1: 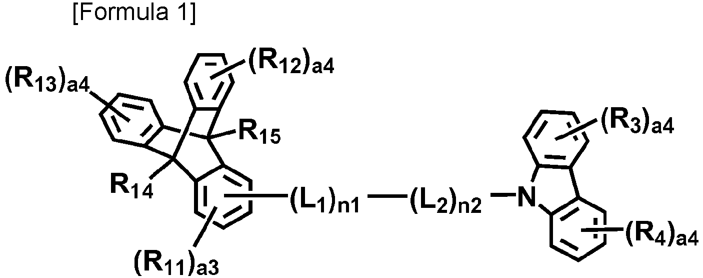
wherein in Formula 1,
L₁ is a C₃-C₆₀ carbocyclic group unsubstituted or substituted with at least one R₁ or a C₁-C₆₀ heterocyclic group unsubstituted or substituted with at least one R₁,
n1 is an integer from 0 to 3,
L₂ is a carbazole group unsubstituted or substituted with at least one R₂,
n2 is 1 or 2,
R₁ to R₄ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₁)(Q₂)(Q₃), -N(Q₁)(Q₂), -B(Q₁)(Q₂), -C(=O)(Q₁), -S(=O)₂(Q₁), or -P(=O)(Q₁)(Q₂),
R₁₁ to R₁₅ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -N(Q₁)(Q₂), - B(Q₁)(Q₂), -C(=O)(Q₁), -S(=O)₂(Q₁), or -P(=O)(Q₁)(Q₂),
a3 is an integer from 0 to 3,
a4 is an integer from 0 to 4,
R₁₀ₐ is:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or a combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), -P(=O)(Q₂₁)(Q₂₂), or a combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ are each independently:
hydrogen, deuterium, -F, -Cl, -Br, -I, a hydroxyl group, a cyano group, or a nitro group; or
a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or a combination thereof.

7. The organic compound of claim 6, wherein L₁ is a C₆-C₆₀ arylene group unsubstituted or substituted with at least one R₁.

8. The organic compound of claim 6, wherein L₁ is a group represented by one of Formulae B1 to B3: 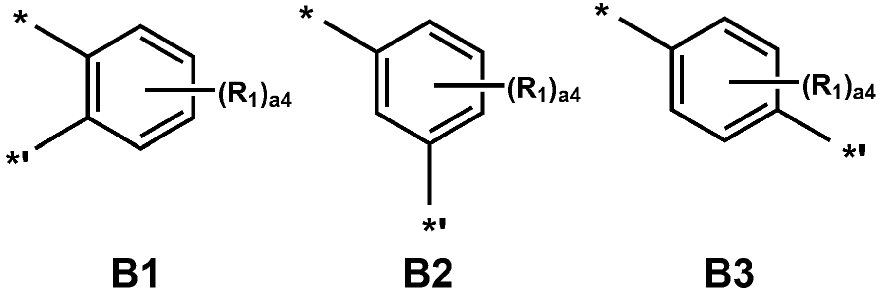
wherein in Formulae B1 to B3,
R₁ and a4 are each the same as defined in Formula 1,
* indicates a binding site to (L₂)ₙ₂ in Formula 1, and
*' indicates a binding site to a neighboring atom.

9. The organic compound of any one of claims 6 to 8, wherein in Formula 1, L₂ is a group represented by Formula C1: 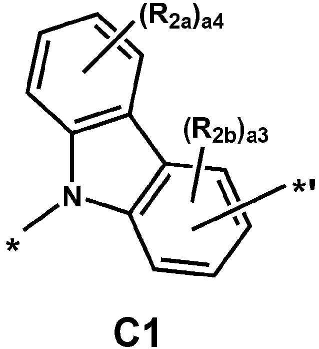
wherein in Formula C1,
a3 and a4 are each the same as defined in Formula 1,
R₂ₐ and R_{2b} are each independently the same as defined in connection with R₂ in Formula 1,
* indicates a binding site to (L₁)ₙ₁ in Formula 1, and
*' indicates a binding site to a neighboring atom.

10. The organic compound of any one of claims 6 to 9, wherein R₁ to R₄ and R₁₁ to R₁₅ each does not include a carbazole group.

11. The organic compound of any one of claims 6 to 10, wherein R₁₄ and R₁₅ are each independently hydrogen or deuterium.

12. The organic compound of any one of claims 6 to 11, wherein in Formula 1, a moiety represented by 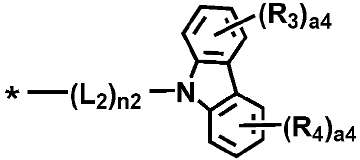 is a moiety represented by one of Formulae A1 to A8: 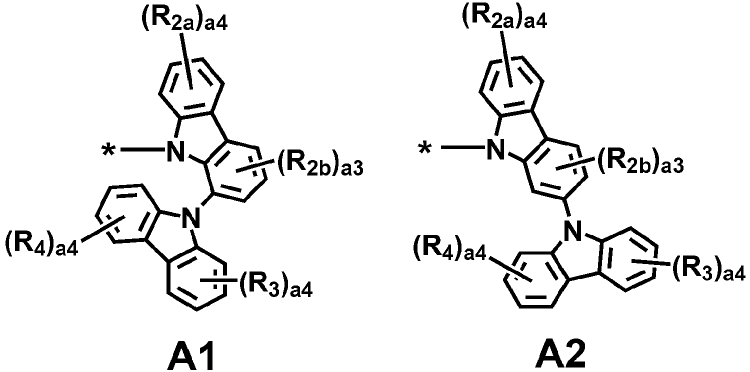 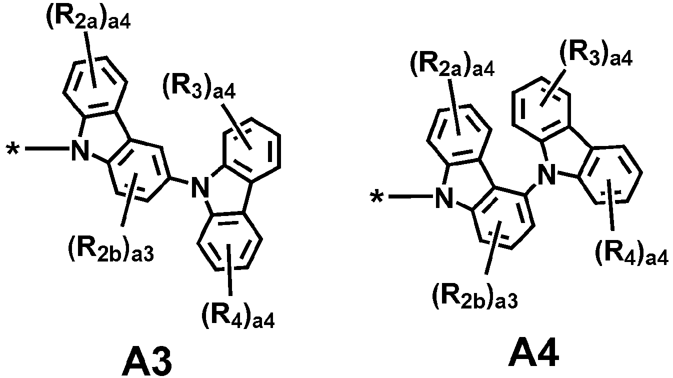 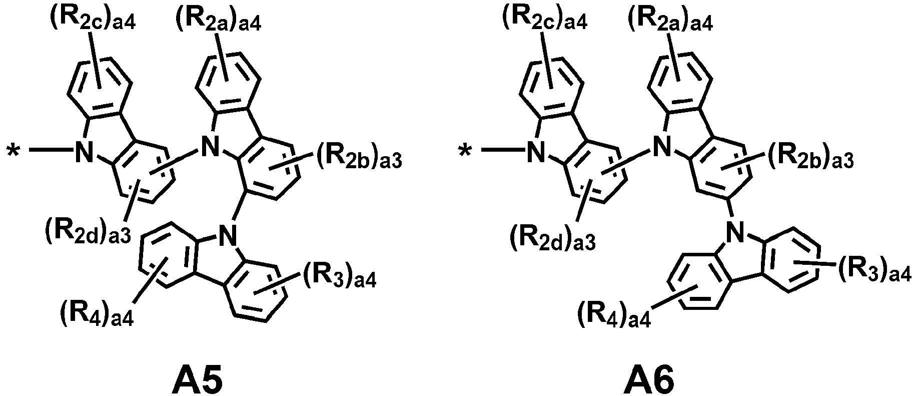 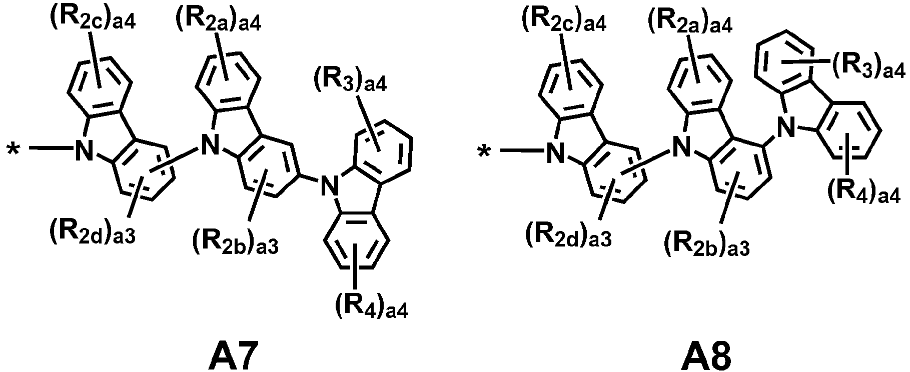 wherein in Formulae A1 to A8,
* indicates a binding site to (L₁)ₙ₁ in Formula 1,
R₂ₐ to R_{2d} are each independently the same as defined in connection with R₂ in Formula 1, and
R₃, R₄, a3, and a4 are each the same as defined in Formula 1.

13. The organic compound of any one of claims 6 to 12, wherein the organic compound is represented by Formula 1' or Formula 1": 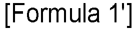 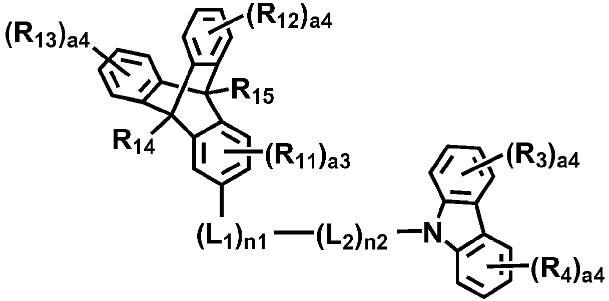 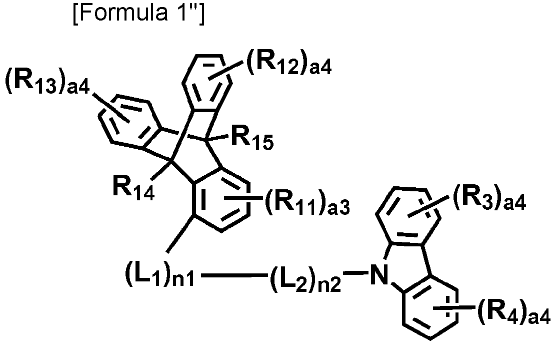
wherein in Formulae 1' and 1",
L₁, L₂, n1, n2, R₃, R₄, R₁₁ to R₁₅, a3, and a4 are each the same as defined in Formula 1.

14. The organic compound of claim 6, wherein the organic compound is represented by Formula 1-1: 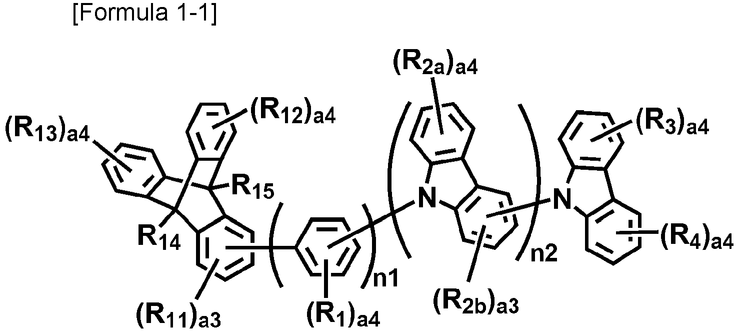
wherein in Formula 1-1,
R₁, R₃, R₄, R₁₁ to R₁₅, n1, n2, a3, and a4 are each the same as defined in Formula 1, and
R₂ₐ and R_{2b} are each independently the same as defined in connection with R₂ in Formula 1.

15. The organic compound of claim 6, wherein
the organic compound is one of Compounds 1 to 100, or
the organic compound is a compound in which at least one hydrogen atom in one of Compounds 1 to 100 is substituted with deuterium:

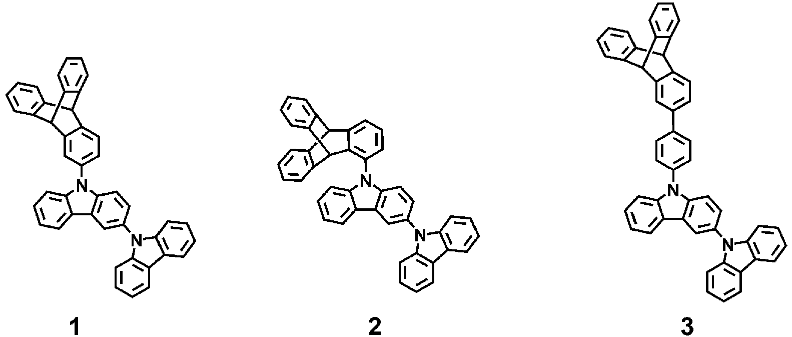

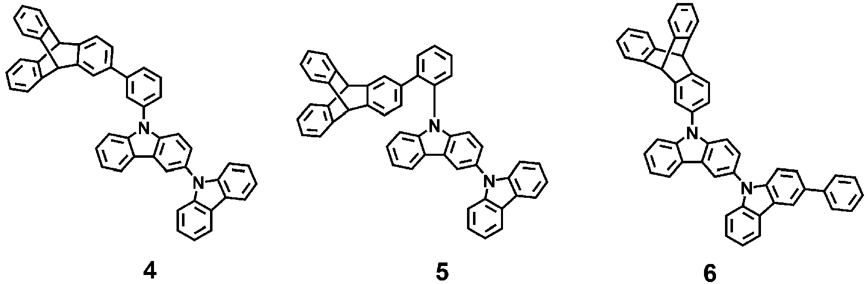

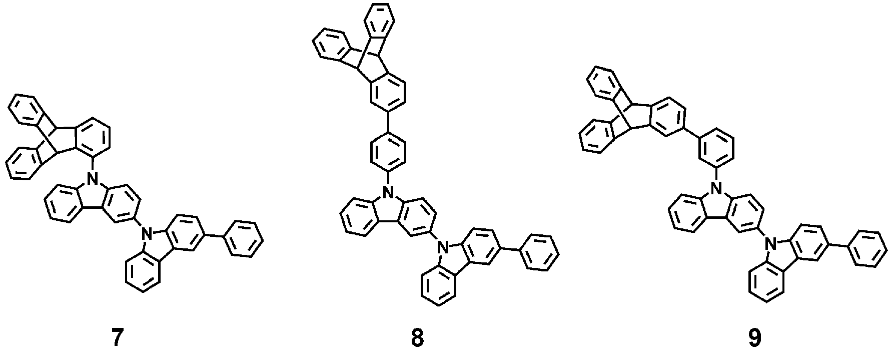

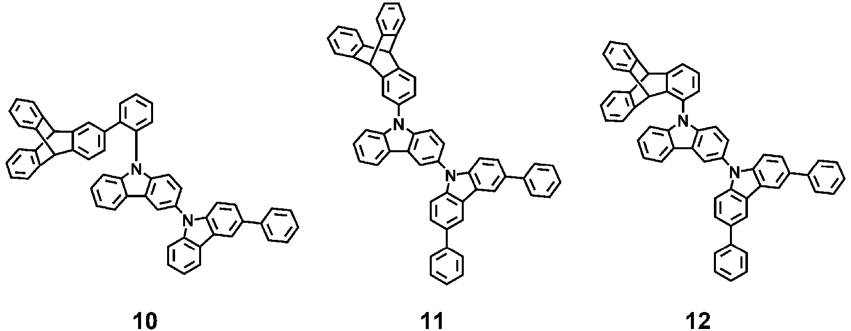

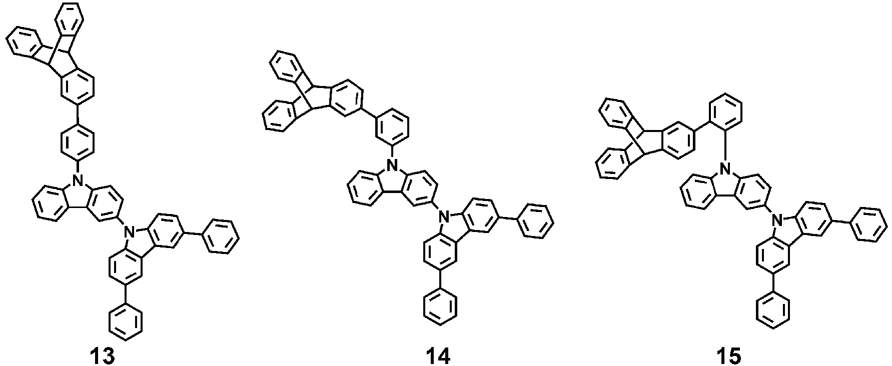

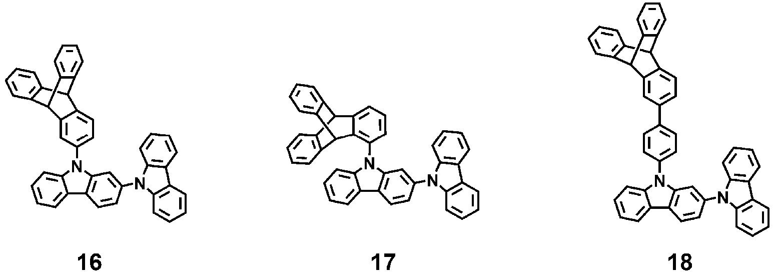

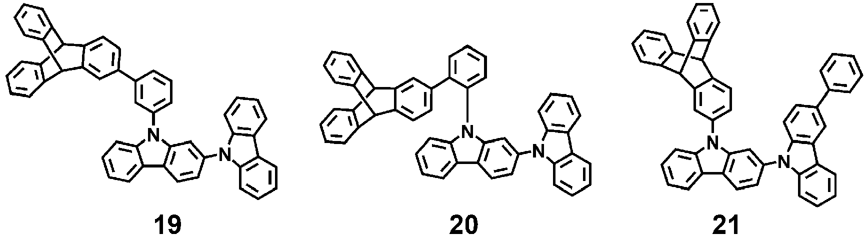

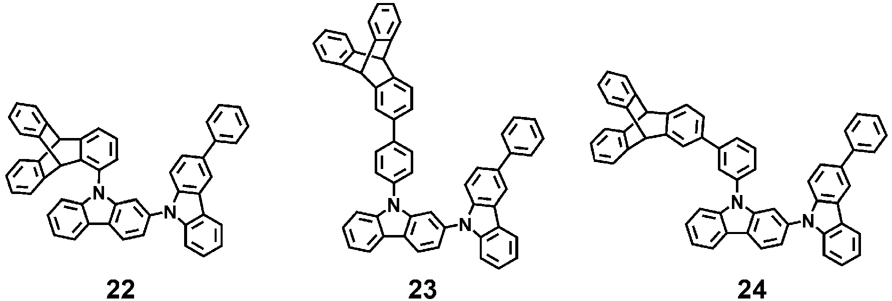

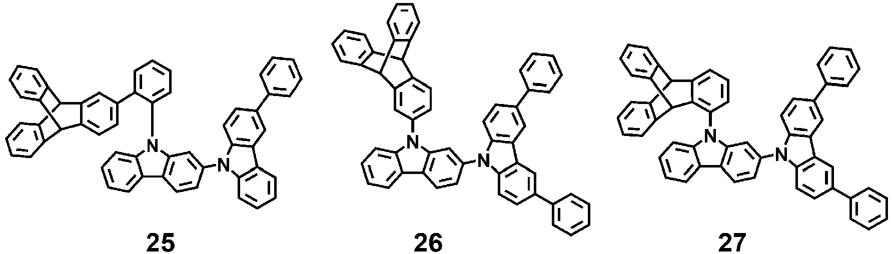

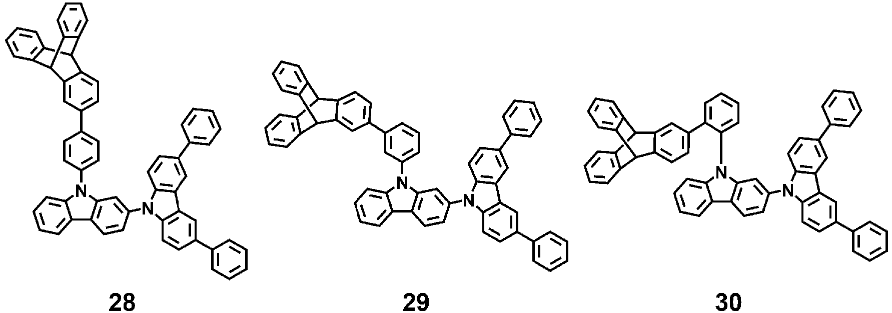

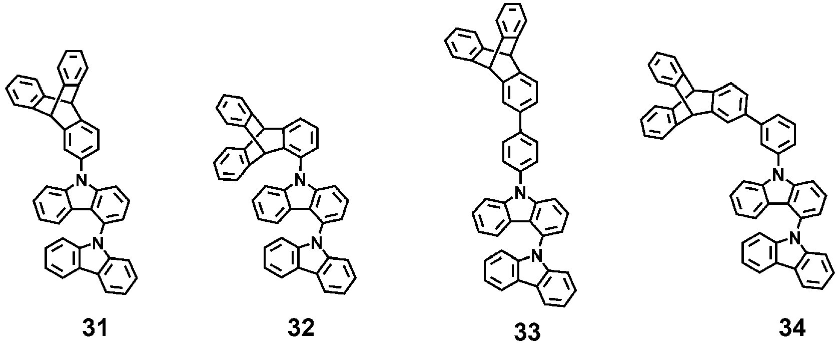

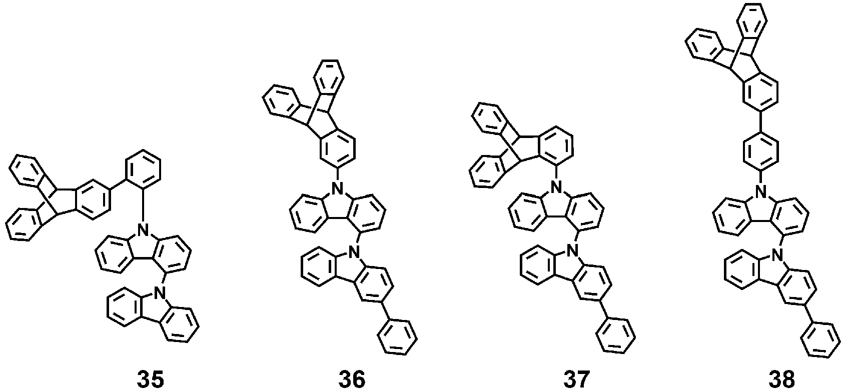

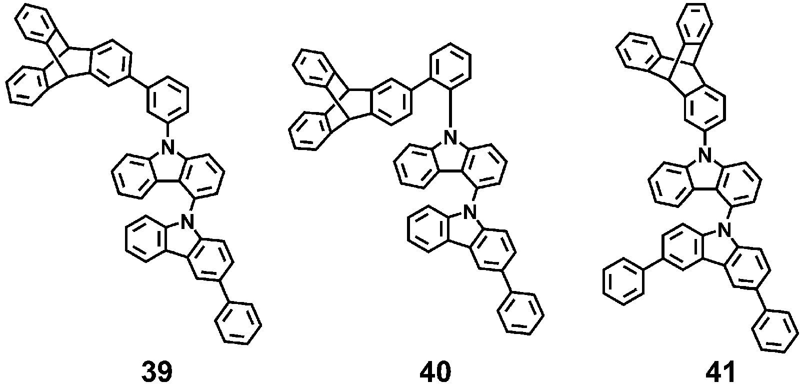

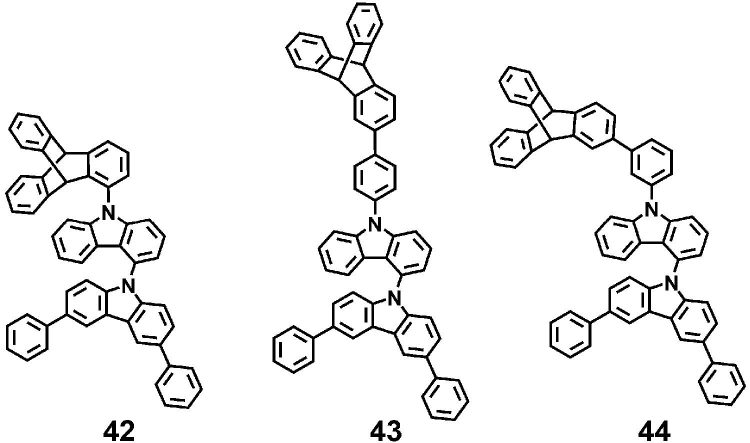

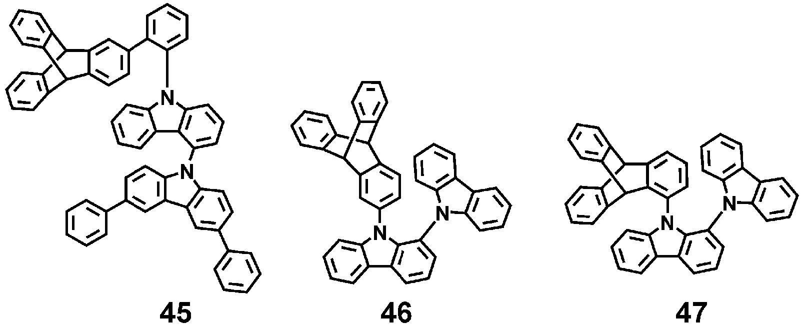

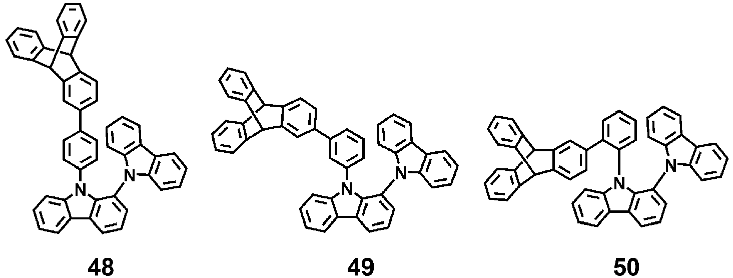

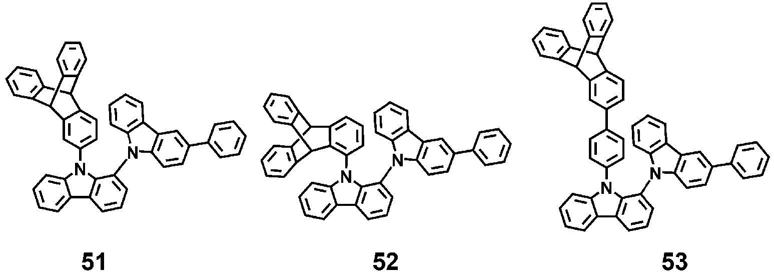

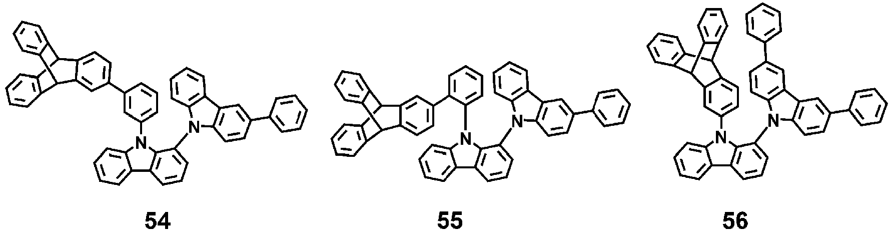

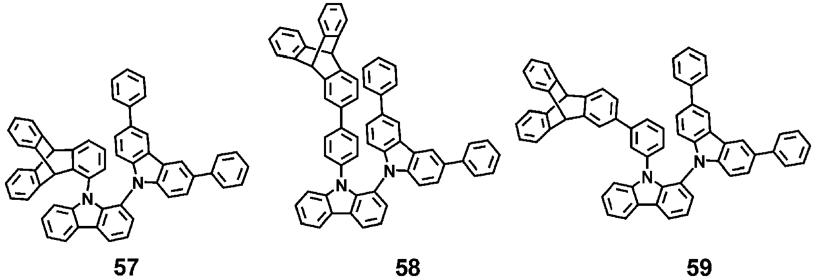

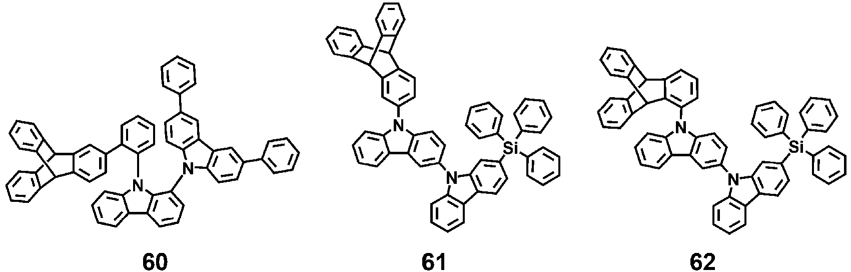

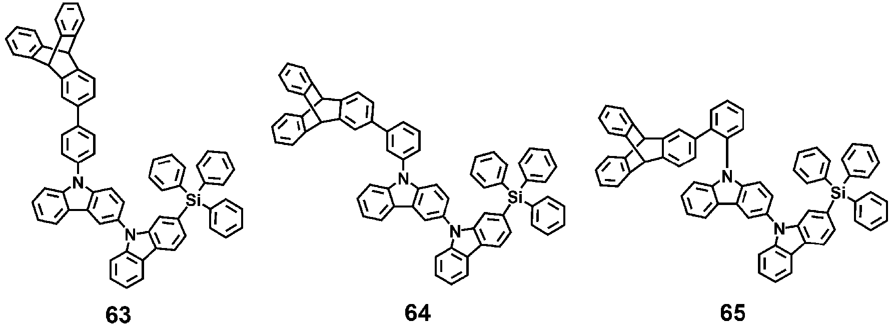

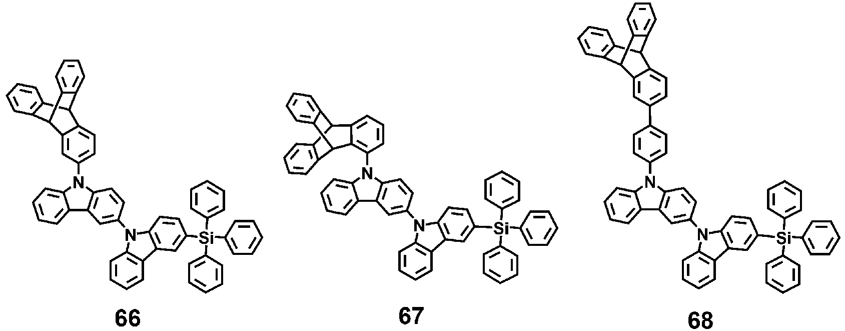

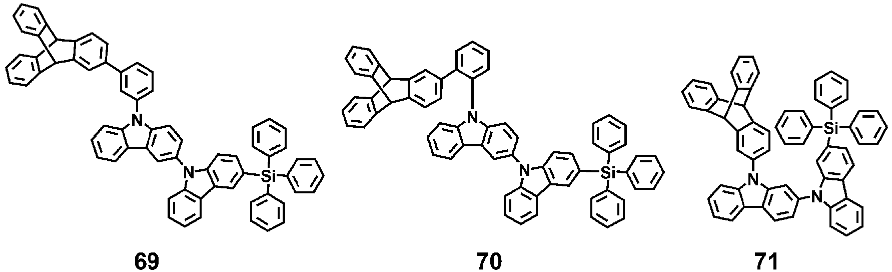

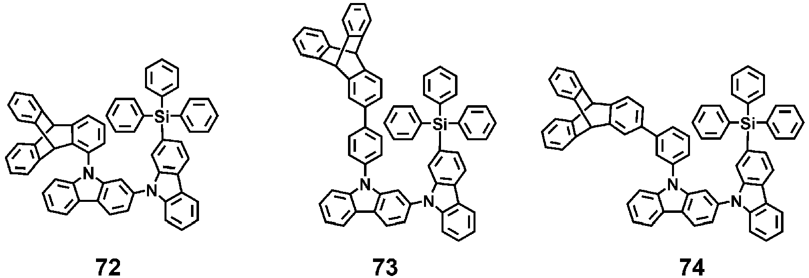

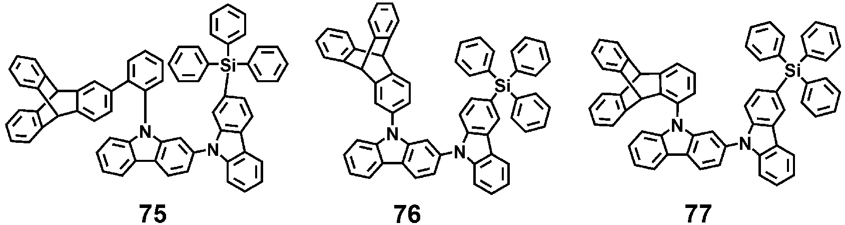

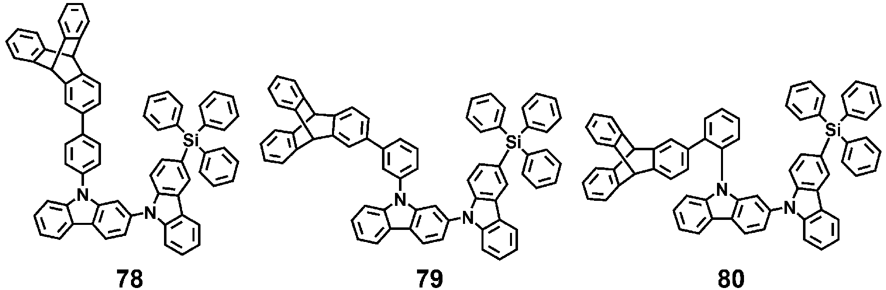

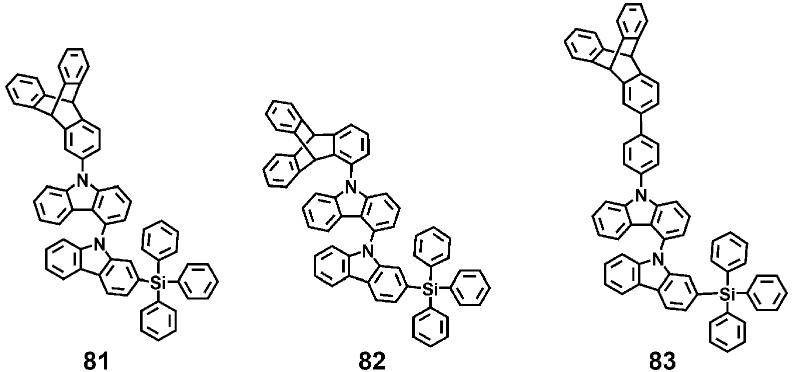

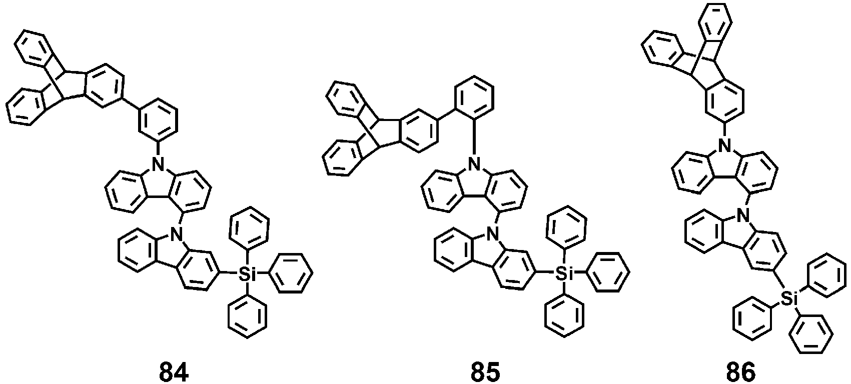

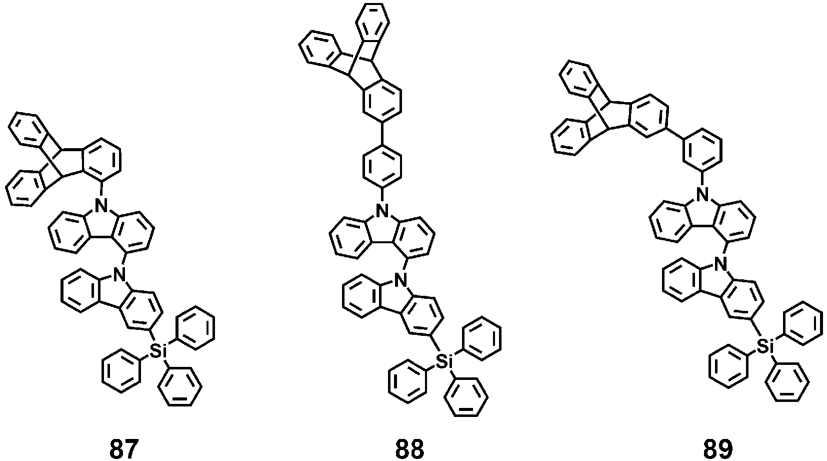

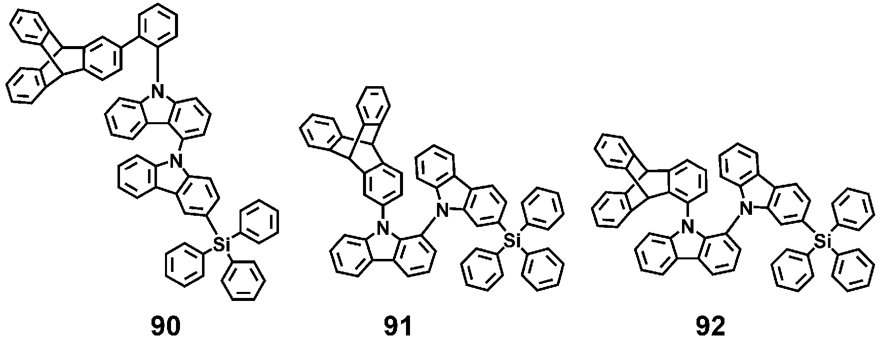

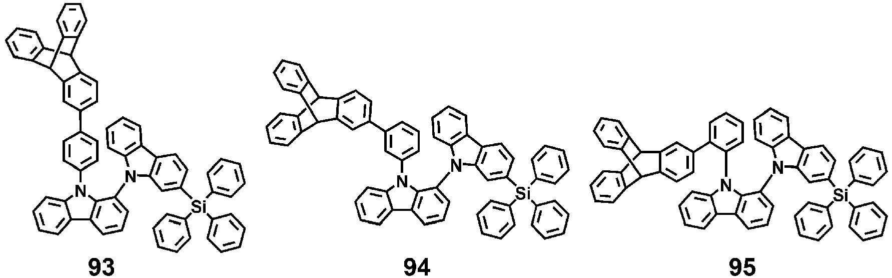

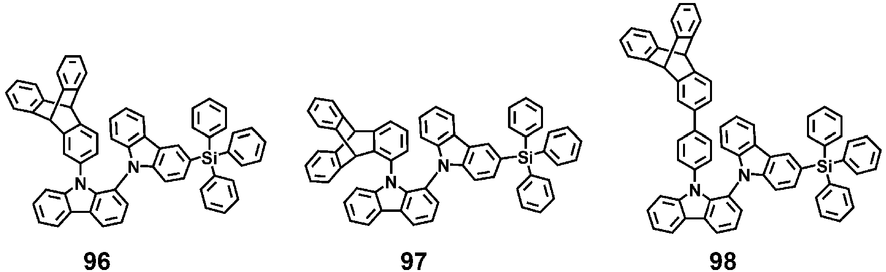

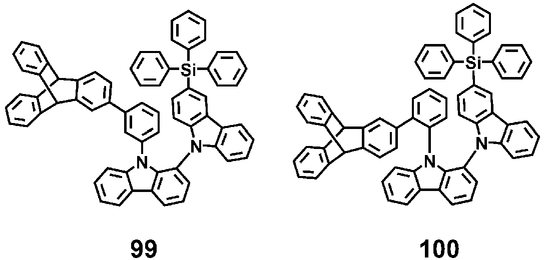

## Amended claims

### Amended claims in accordance with Rule 137(2) EPC.

1. An organic compound represented by Formula 1: 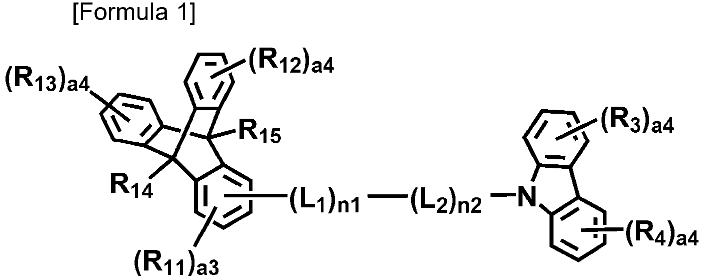 wherein in Formula 1,
L₁ is a C₃-C₆₀ carbocyclic group unsubstituted or substituted with at least one R₁ or a C₁-C₆₀ heterocyclic group unsubstituted or substituted with at least one R₁,
n1 is an integer from 0 to 3,
L₂ is a carbazole group unsubstituted or substituted with at least one R₂,
n2 is 1 or 2,
R₁ to R₄ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -Si(Q₁)(Q₂)(Q₃), -N(Q₁)(Q₂), -B(Q₁)(Q₂), -C(=O)(Q₁)**,** -S(=O)₂(Q₁), or -P(=O)(Q₁)(Q₂)**,**
R₁₁ to R₁₅ are each independently hydrogen, deuterium, -F, -Cl, -Br, -I, a hydroxyl group, a cyano group, a nitro group, a C₁-C₆₀ alkyl group unsubstituted or substituted with at least one R₁₀ₐ, a C₂-C₆₀ alkenyl group unsubstituted or substituted with at least one R₁₀ₐ, a C₂-C₆₀ alkynyl group unsubstituted or substituted with at least one R₁₀ₐ, a C₁-C₆₀ alkoxy group unsubstituted or substituted with at least one R₁₀ₐ, a C₃-C₆₀ carbocyclic group unsubstituted or substituted with at least one R₁₀ₐ, a C₁-C₆₀ heterocyclic group unsubstituted or substituted with at least one R₁₀ₐ, -N(Q₁)(Q₂), - B(Q₁)(Q₂), -C(=O)(Q₁), -S(=O)₂(Q₁), or -P(=O)(Q₁)(Q₂),
a3 is an integer from 0 to 3,
a4 is an integer from 0 to 4,
R₁₀ₐ is:
deuterium, -F, -Cl, -Br, -I, a hydroxyl group, a cyano group, or a nitro group;
a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₁₁)(Q₁₂)(Q₁₃), -N(Q₁₁)(Q₁₂), -B(Q₁₁)(Q₁₂), - C(=O)(Q₁₁), -S(=O)₂(Q₁₁), -P(=O)(Q₁₁)(Q₁₂), or a combination thereof;
a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, or a C₂-C₆₀ heteroarylalkyl group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₇-C₆₀ arylalkyl group, a C₂-C₆₀ heteroarylalkyl group, -Si(Q₂₁)(Q₂₂)(Q₂₃), -N(Q₂₁)(Q₂₂), -B(Q₂₁)(Q₂₂), -C(=O)(Q₂₁), - S(=O)₂(Q₂₁), -P(=O)(Q₂₁)(Q₂₂), or a combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁ to Q₃, Q₁₁ to Q₁₃, Q₂₁ to Q₂₃, and Q₃₁ to Q₃₃ are each independently:
hydrogen, deuterium, -F, -Cl, -Br, -I, a hydroxyl group, a cyano group, or a nitro group; or
a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or a combination thereof.

2. The organic compound of claim 1, wherein L₁ is a C₆-C₆₀ arylene group unsubstituted or substituted with at least one R₁.

3. The organic compound of claim 1, wherein L₁ is a group represented by one of Formulae B1 to B3: 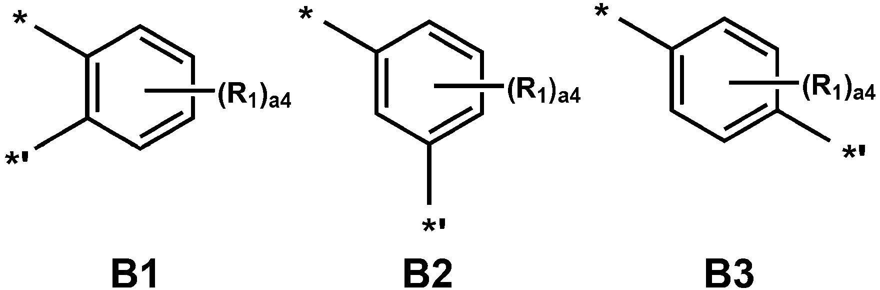 wherein in Formulae B1 to B3,
R₁ and a4 are each the same as defined in Formula 1,
* indicates a binding site to (L₂)ₙ₂ in Formula 1, and
*' indicates a binding site to a neighboring atom.

4. The organic compound of any one of claims 1 to 3, wherein in Formula 1, L₂ is a group represented by Formula C1: 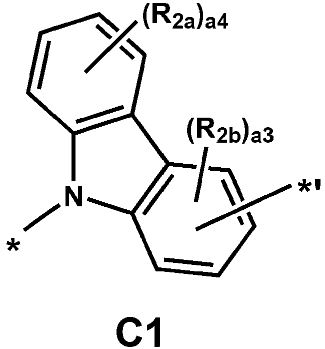 wherein in Formula C1,
a3 and a4 are each the same as defined in Formula 1,
R₂ₐ and R_{2b} are each independently the same as defined in connection with R₂ in Formula 1,
* indicates a binding site to (L₁)ₙ₁ in Formula 1, and
*' indicates a binding site to a neighboring atom.

5. The organic compound of any one of claims 1 to 4, wherein R₁ to R₄ and R₁₁ to R₁₅ each does not include a carbazole group.

6. The organic compound of any one of claims 1 to 5, wherein R₁₄ and R₁₅ are each independently hydrogen or deuterium.

7. The organic compound of any one of claims 1 to 6, wherein in Formula 1, a moiety represented by 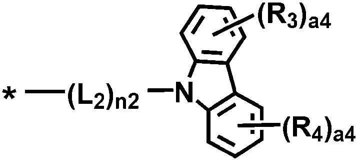 is a moiety represented by one of Formulae A1 to A8: 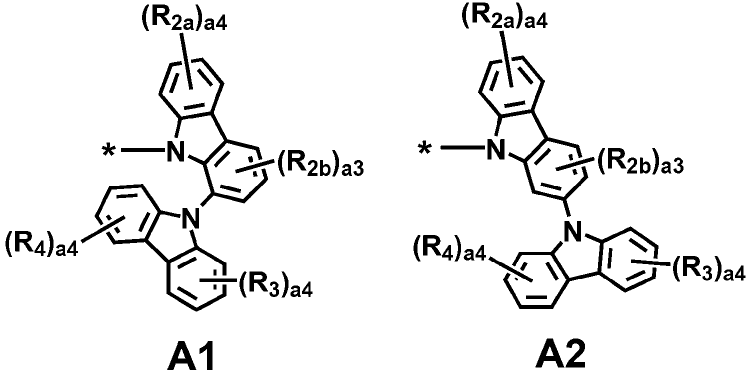 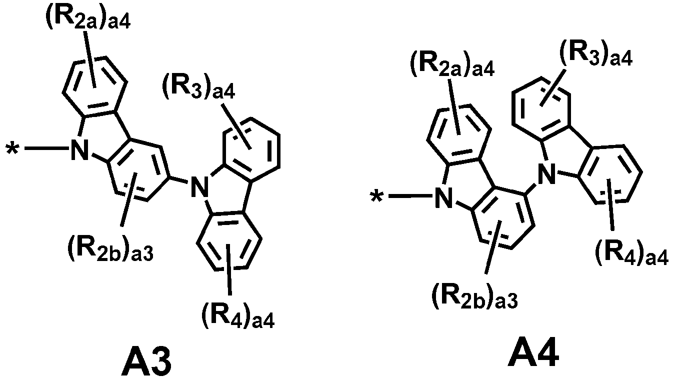 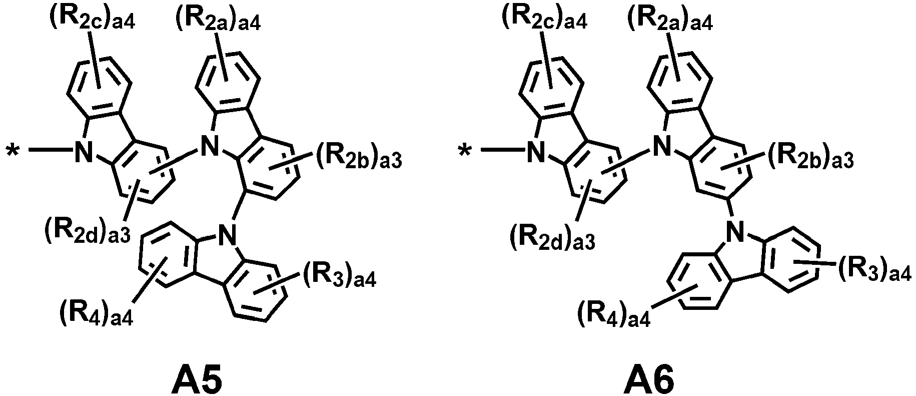 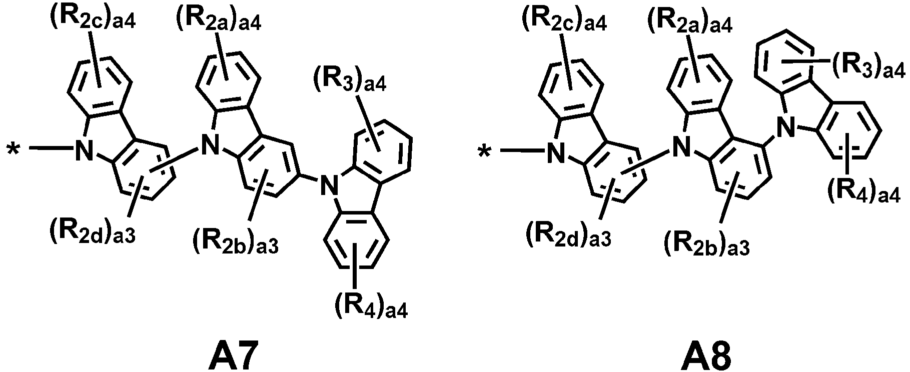 wherein in Formulae A1 to A8,
* indicates a binding site to (L₁)ₙ₁ in Formula 1,
R₂ₐ to R_{2d} are each independently the same as defined in connection with R₂ in Formula 1, and
R₃, R₄, a3, and a4 are each the same as defined in Formula 1.

8. The organic compound of any one of claims 1 to 7, wherein the organic compound is represented by Formula 1' or Formula 1": 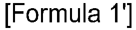 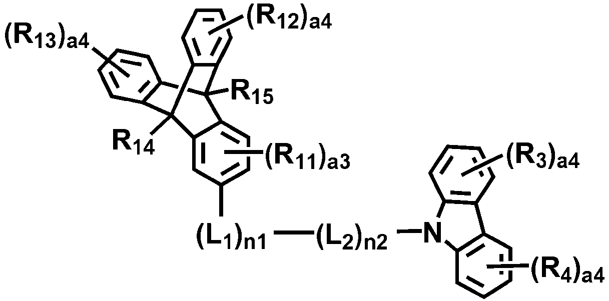 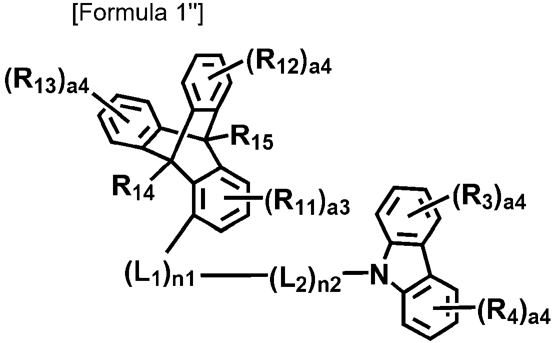 wherein in Formulae 1' and 1",
L₁, L₂, n1, n2, R₃, R₄, R₁₁ to R₁₅, a3, and a4 are each the same as defined in Formula 1.

9. The organic compound of claim 1, wherein the organic compound is represented by Formula 1-1: 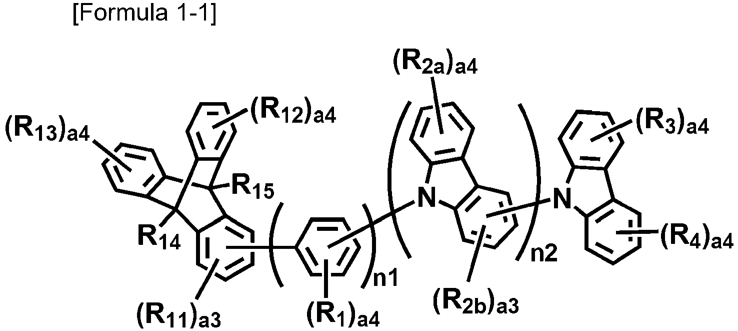 wherein in Formula 1-1,
R₁, R₃, R₄, R₁₁ to R₁₅, n1, n2, a3, and a4 are each the same as defined in Formula 1, and
R₂ₐ and R_{2b} are each independently the same as defined in connection with R₂ in Formula 1.

10. The organic compound of claim 1, wherein
the organic compound is one of Compounds 1 to 100, or
the organic compound is a compound in which at least one hydrogen atom in one of Compounds 1 to 100 is substituted with deuterium:

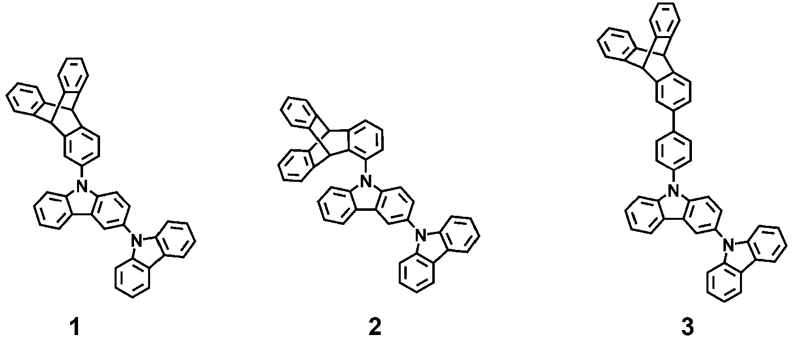

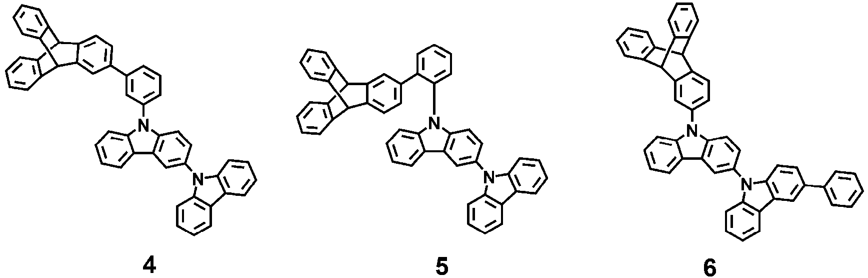

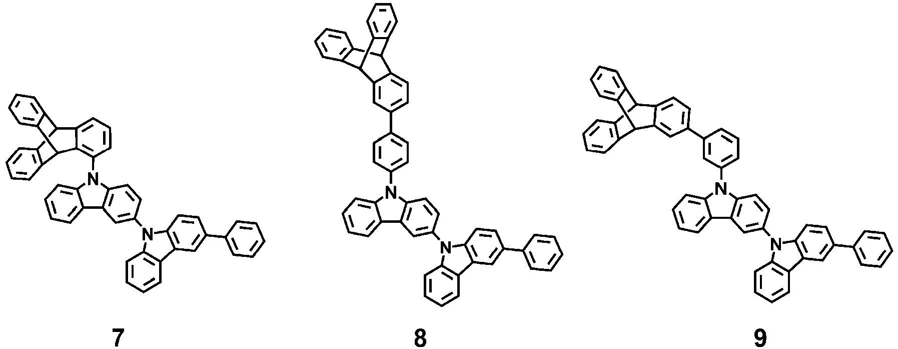

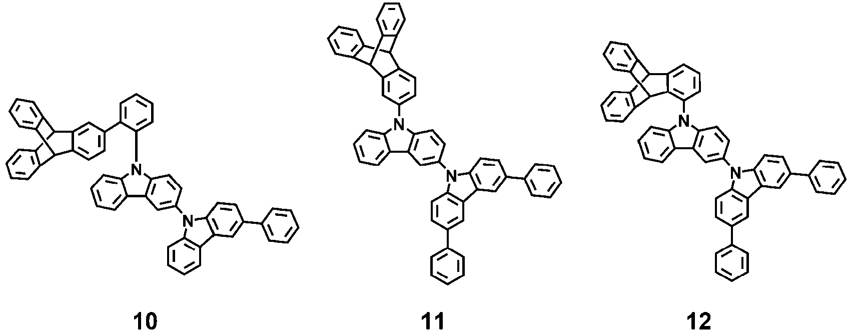

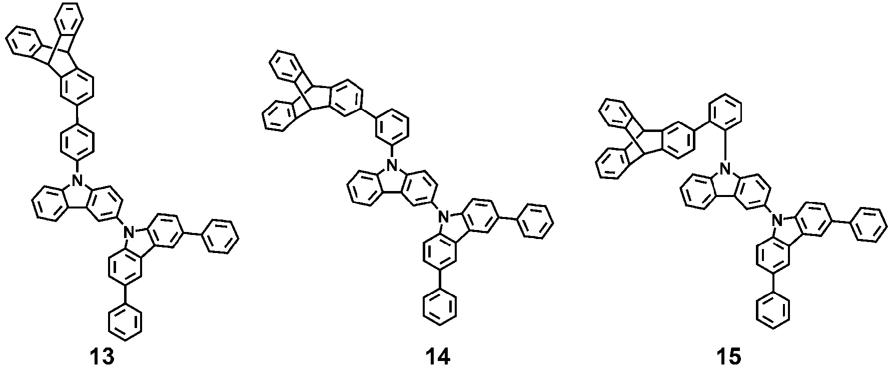

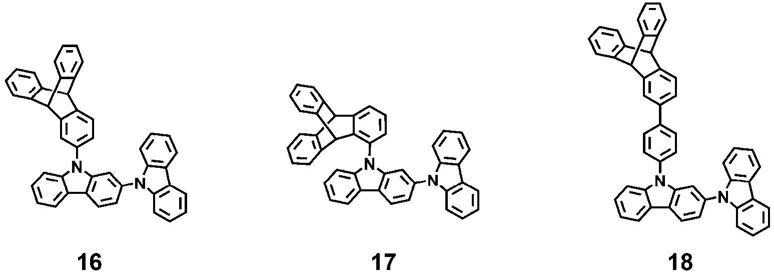

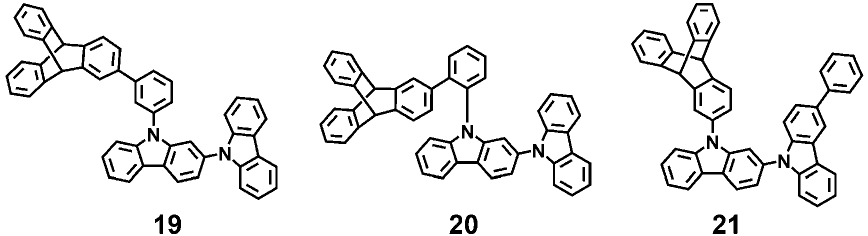

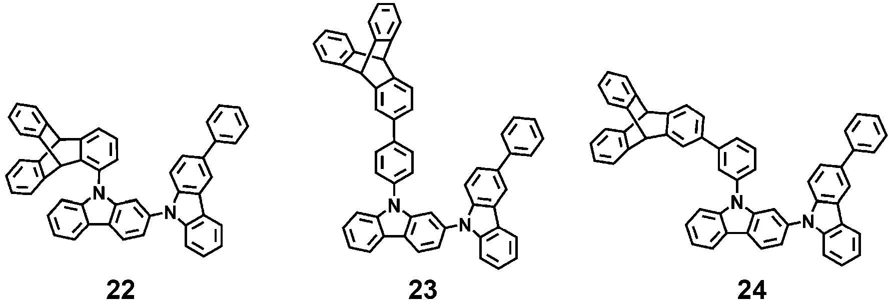

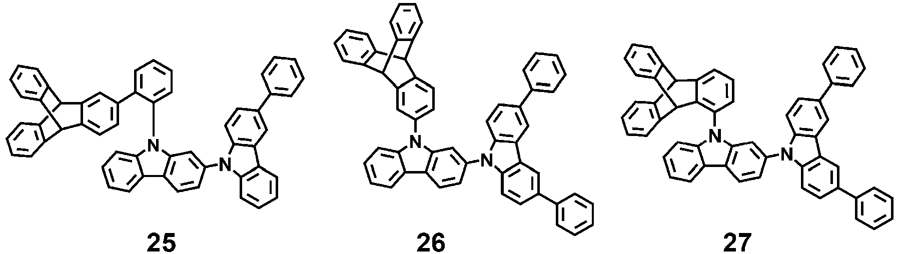

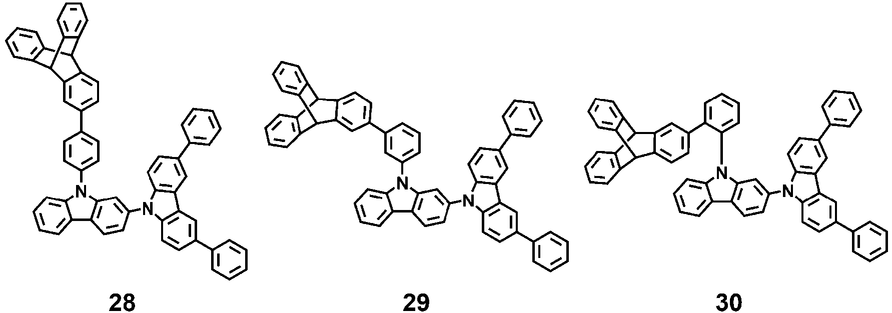

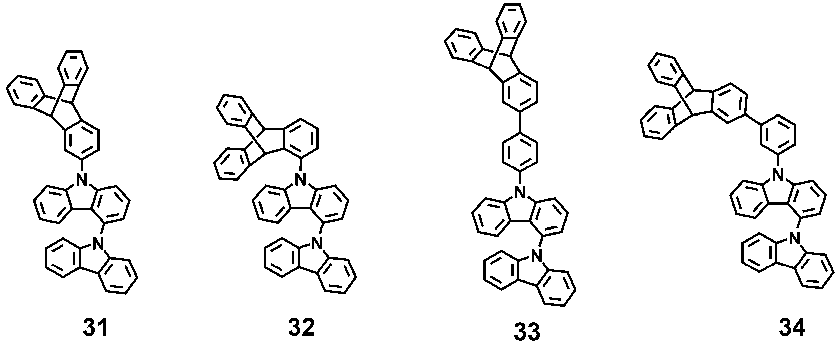

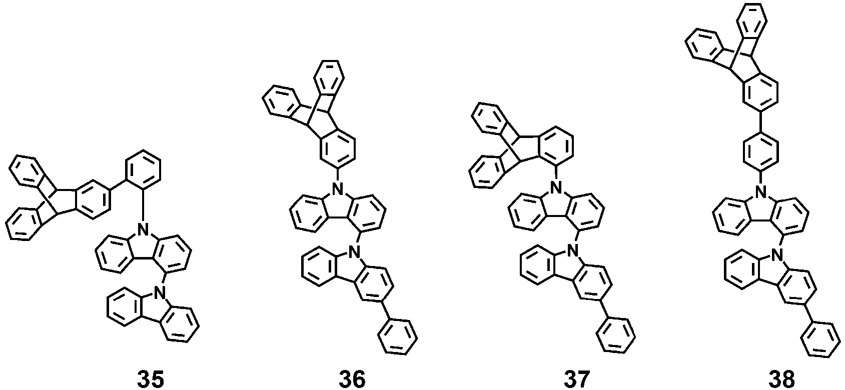

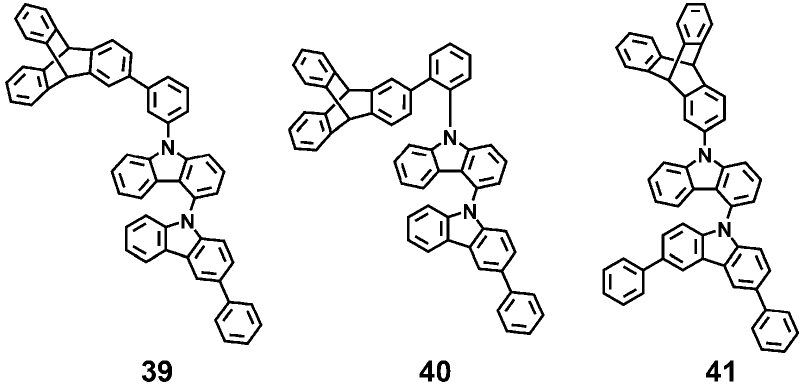

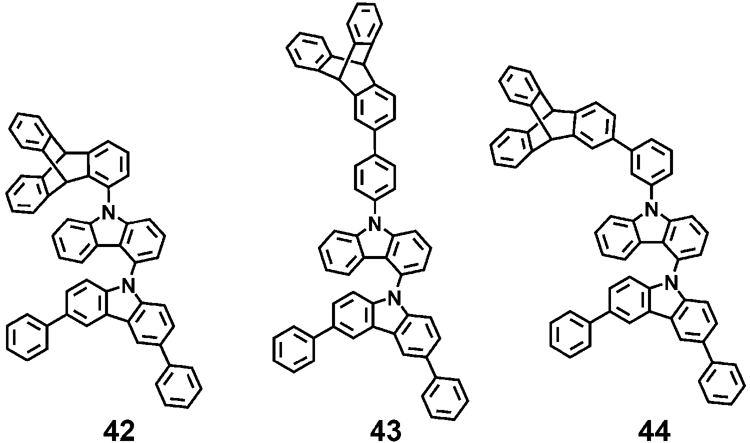

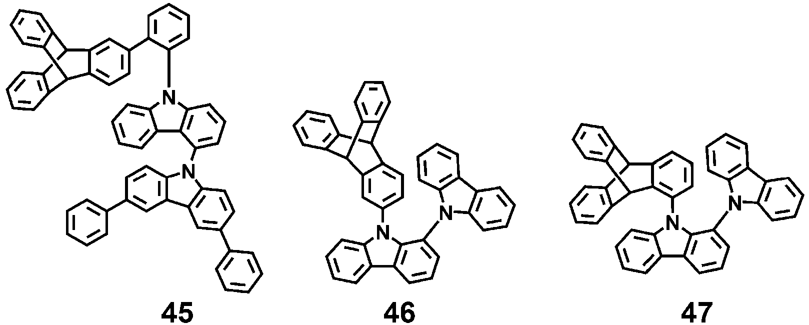

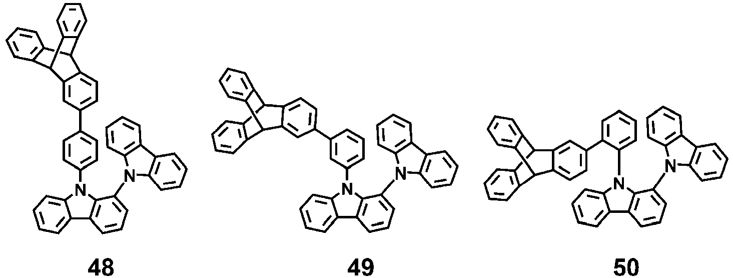

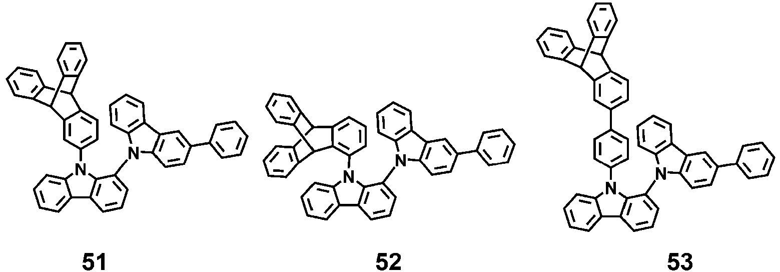

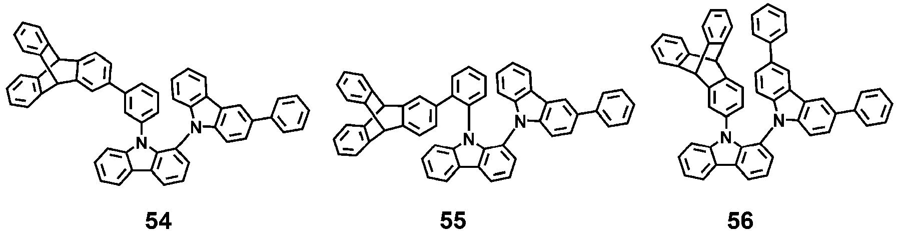

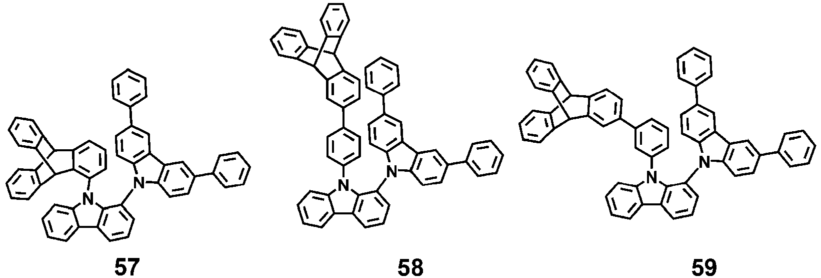

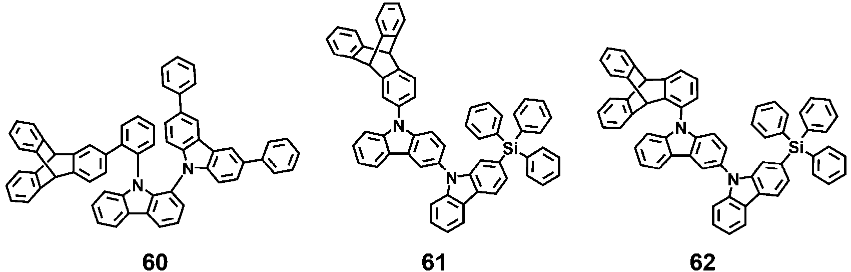

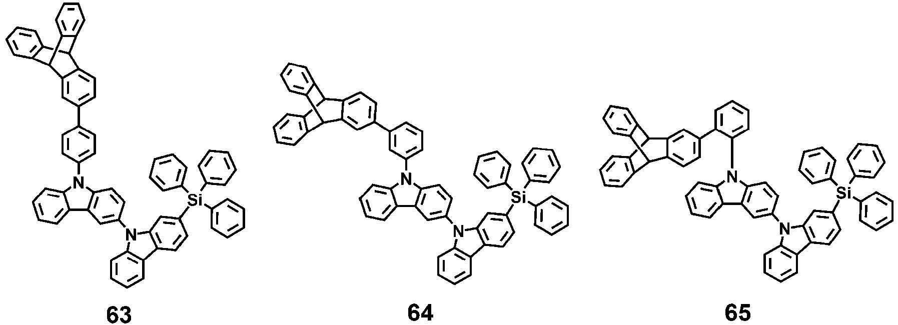

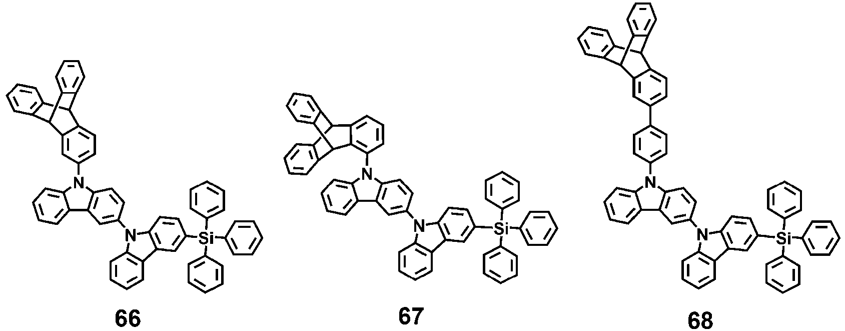

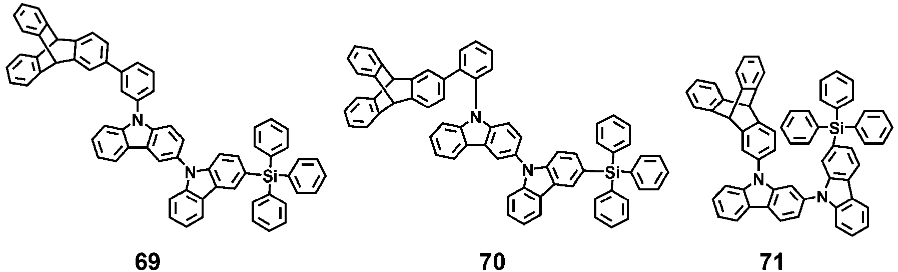

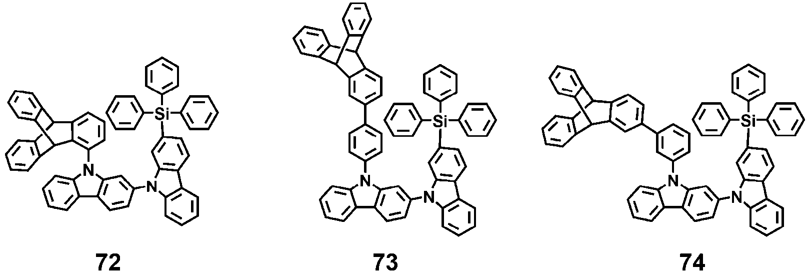

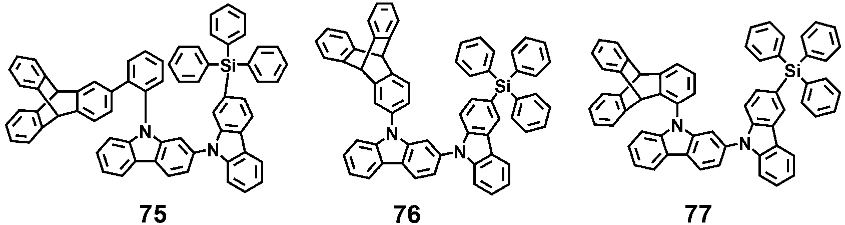

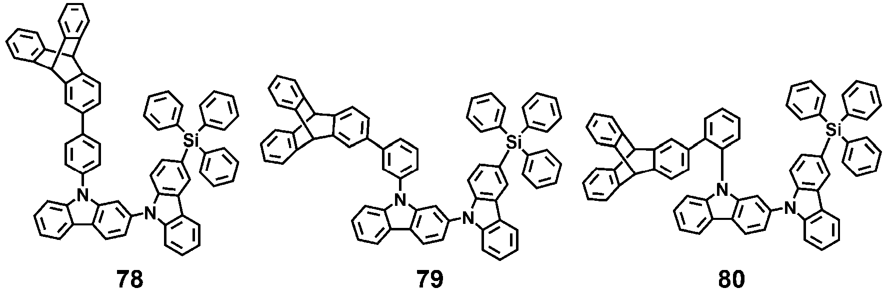

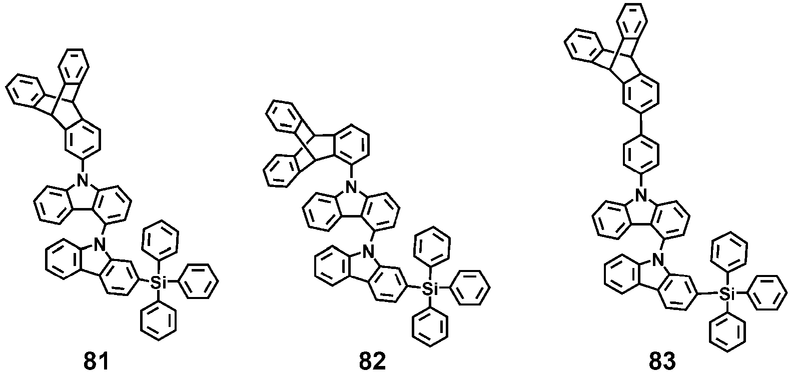

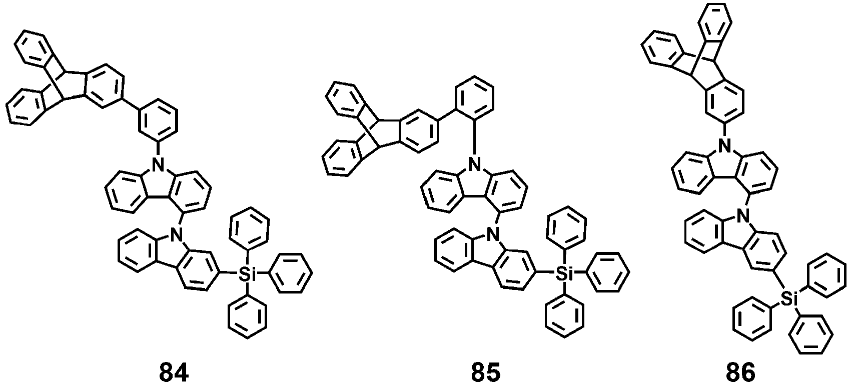

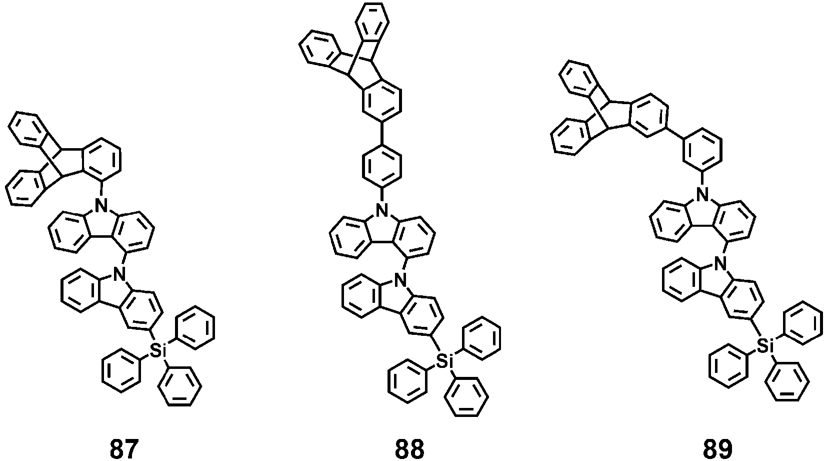

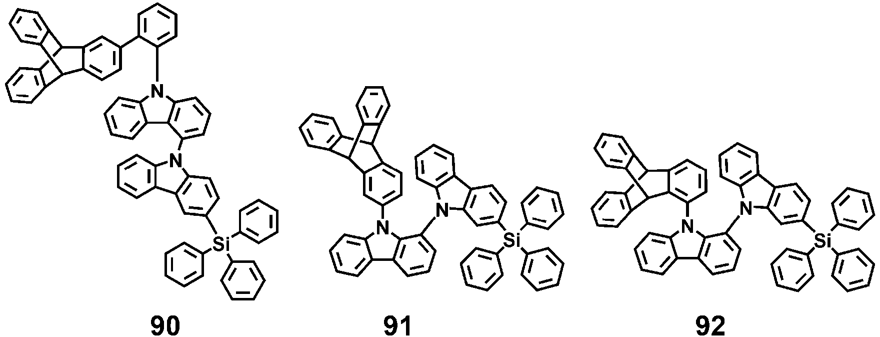

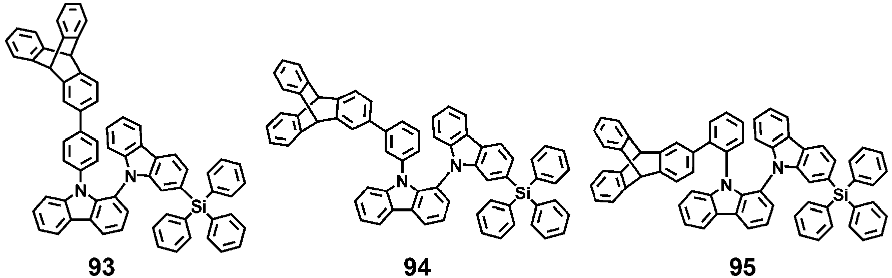

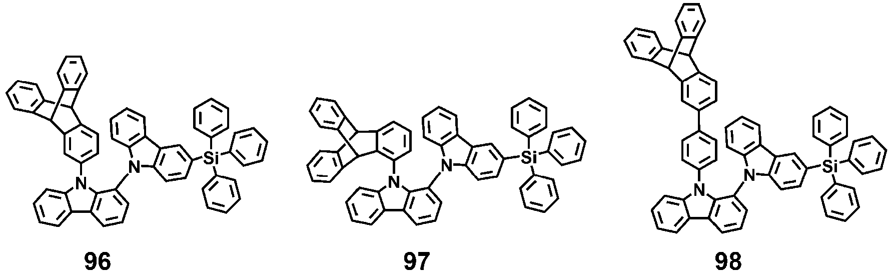

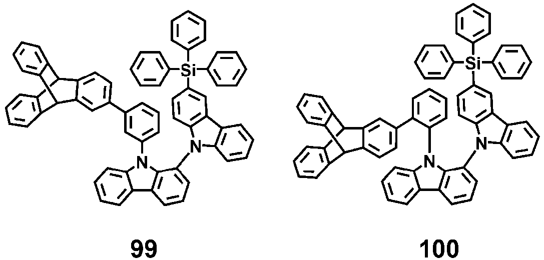

11. A light-emitting device comprising:
a first electrode;
a second electrode facing the first electrode;
an interlayer between the first electrode and the second electrode and comprising an emission layer; and
the organic compound of any one of claims 1 to 10.

12. The light-emitting device of claim 11, wherein the interlayer comprises the organic compound.

13. The light-emitting device of claim 11 or claim 12, wherein the emission layer is configured to emit blue light.

14. An electronic apparatus comprising:
the light-emitting device of any one of claims 11 to 13; and
a thin-film transistor electrically connected to the light-emitting device.

15. An electronic equipment comprising:
the electronic apparatus of claim 14, wherein
the electronic equipment is a flat panel display, a curved display, a computer monitor, a medical monitor, a television, a billboard, an indoor light, an outdoor light, a signal light, a head-up display, a fully transparent display, a partially transparent display, a flexible display, a rollable display, a foldable display, a stretchable display, a laser printer, a telephone, a mobile phone, a tablet computer, a phablet, a personal digital assistant (PDA), a wearable device, a laptop computer, a digital camera, a camcorder, a viewfinder, a micro display, a three-dimensional (3D) display, a virtual reality display, an augmented reality display, a vehicle, a video wall with multiple displays tiled together, a theater screen, a stadium screen, a phototherapy device, or a signboard.
